(19) Europäisches Patentamt
European Patent Office
Office européen des brevets

(11) EP 4 167 959 B1

(12) EUROPEAN PATENT SPECIFICATION

(45) Date of publication and mention of the grant of the patent:
**28.08.2024 Bulletin 2024/35**

(21) Application number: **21743229.3**

(22) Date of filing: **21.06.2021**

(51) International Patent Classification (IPC):
***A61K 8/34*** (2006.01) ***A61K 8/365*** (2006.01)
***A61K 8/891*** (2006.01) ***A61K 9/107*** (2006.01)
***A61K 31/05*** (2006.01) ***A61K 31/07*** (2006.01)
***A61K 31/17*** (2006.01) ***A61K 31/192*** (2006.01)
***A61K 31/203*** (2006.01) ***A61K 47/10*** (2017.01)
***A61K 47/12*** (2006.01) ***A61K 47/18*** (2017.01)
***A61K 47/24*** (2006.01) ***A61P 17/00*** (2006.01)
***A61Q 19/00*** (2006.01) ***A61K 8/49*** (2006.01)
***A61K 8/894*** (2006.01) ***A61K 8/898*** (2006.01)
***A61P 35/00*** (2006.01) ***A61K 8/67*** (2006.01)

(52) Cooperative Patent Classification (CPC):
(C-Sets available)
**A61K 9/0014; A61K 8/342; A61K 8/345; A61K 8/365; A61K 8/4913; A61K 8/675; A61K 8/891; A61K 8/894; A61K 8/898; A61K 9/107; A61K 31/047; A61K 31/05; A61K 31/07; A61K 31/17; A61K 31/192;** (Cont.)

(86) International application number:
**PCT/GB2021/051569**

(87) International publication number:
**WO 2021/255483 (23.12.2021 Gazette 2021/51)**

(54) **COMPOSITION FOR TOPICAL DERMATOLOGICAL DELIVERY**

ZUSAMMENSETZUNG ZUR TOPISCHEN DERMATOLOGISCHEN VERABREICHUNG

COMPOSITION POUR UNE ADMINISTRATION TOPIQUE DERMATOLOGIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.06.2020 GB 202009437**

(43) Date of publication of application:
**26.04.2023 Bulletin 2023/17**

(73) Proprietors:
- **Limeway Pharma Design Limited**
  **Dorking**
  **Surrey**
  **RH4 1HZ (GB)**
- **Davis, Adrian**
  **Surrey RH4 1HZ (GB)**

(72) Inventor: **DAVIS, Adrian**
**Dorking Surrey RH4 1HZ (GB)**

(74) Representative: **Barker Brettell LLP**
**100 Hagley Road**
**Edgbaston**
**Birmingham B16 8QQ (GB)**

(56) References cited:
**WO-A1-2016/139471 GB-A- 2 562 270**
**GB-A- 2 597 526**

- **CHERYL LEE EBERTING ET AL: "Repairing a Compromised Skin Barrier in Dermatitis: Leveraging the Skin's Ability to Heal Itself", JOURNAL OF ALLERGY & THERAPY, vol. 05, no. 05, 1 January 2014 (2014-01-01), XP055531732, DOI: 10.4172/2155-6121.1000187**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

• **YOSHINAO SOMA ET AL: "Moisturizing effects of topical nicotinamide on atopic dry skin", INTERNATIONAL JOURNAL OF DERMATOLOGY, vol. 44, no. 3, 1 March 2005 (2005-03-01), UK, pages 197 - 202, XP055629553, ISSN: 0011-9059, DOI: 10.1111/j.1365-4632.2004.02375.x**

(52) Cooperative Patent Classification (CPC): (Cont.)
**A61K 31/203; A61K 31/352; A61K 31/366; A61K 31/4174; A61K 31/436; A61K 31/4436; A61K 31/455; A61K 31/496; A61K 31/522; A61K 31/56; A61K 31/573; A61K 31/58; A61K 31/593; A61K 31/7032; A61K 31/728; A61K 47/10; A61K 47/12; A61K 47/18; A61K 47/24; A61P 17/00; A61P 35/00; A61Q 19/00**

C-Sets
**A61K 31/047, A61K 2300/00;**
**A61K 31/05, A61K 2300/00;**
**A61K 31/07, A61K 2300/00;**
**A61K 31/17, A61K 2300/00;**
**A61K 31/192, A61K 2300/00;**
**A61K 31/203, A61K 2300/00;**
**A61K 31/352, A61K 2300/00;**
**A61K 31/366, A61K 2300/00;**
**A61K 31/4174, A61K 2300/00;**
**A61K 31/436, A61K 2300/00;**
**A61K 31/4436, A61K 2300/00;**
**A61K 31/455, A61K 2300/00;**
**A61K 31/496, A61K 2300/00;**
**A61K 31/522, A61K 2300/00;**
**A61K 31/56, A61K 2300/00;**
**A61K 31/573, A61K 2300/00;**
**A61K 31/58, A61K 2300/00;**
**A61K 31/593, A61K 2300/00;**
**A61K 31/7032, A61K 2300/00;**
**A61K 31/728, A61K 2300/00**

## Description

The present invention relates to a composition suitable for use in topical dermatological delivery of an active agent.

### *Background of the Invention*

In their 2003 report on medication adherence, the World Health Organization (WHO) assert that increasing the effectiveness of adherence interventions may have a far greater impact on the health of the population than any improvement in specific medical treatments. In this report, WHO were mainly concerned with the major systemic diseases and subsequently, mainly oral, therapies. Several factors related to therapy, slow onset or low intensity of efficacy, actual or feared unwanted effects and treatment regimens inconsistent with lifestyle were found to be associated with poor adherence.

The topical application of medicines to the skin to treat conditions and diseases of the skin and the underlying tissues is an intuitive therapeutic concept; it makes sense. Topical products are widely and enthusiastically used by consumers as evidenced by the $145 billion 2020 world-wide annual sale of skin care products, the largest sector in the overall cosmetic beauty market.

Since the 2003 WHO Report, several studies have investigated the extent of adherence and its drivers for topical dermatological medicines. Adherence to recommended or prescribed topical dermatological medicinal products is disappointingly low. The American dermatologist Dr Steven Feldman concludes in the February 2018 edition of Practical Dermatology that adherence to topical medicinal products is "miserable".

Research into the reasons for these low rates of adherence identifies, much as the WHO Report, that slow and poor response to treatment, incidence of or fear of incidence of local and systemic drug adverse effects (especially the phobia of topical corticosteroids), treatment regimens inconsistent with lifestyle and, specific to topical products, the poor aesthetics and experience in use of topical dermatological products, are main causal factors (Zschocke et al. Venereol. 2014 May;28 Suppl 2:4-9; Tan et al. Expert Opin Drug Deliv. 2012 Oct;9(10):1263-71; Devaux et al. Acad Dermatol Venereol. 2012 May;26 Suppl 3:61-7).

The table below shows a summary of various dimensions effecting adherence. Especially relevant in this current context are the formulation biopharmaceutical and cosmeceutical design factors, briefly 1) efficacy, 2) local and systemic adverse effects potential, 3) time and effort in use (all as described by WHO) and 4) the consumer experience in use.

**Dimensions of Adherence**

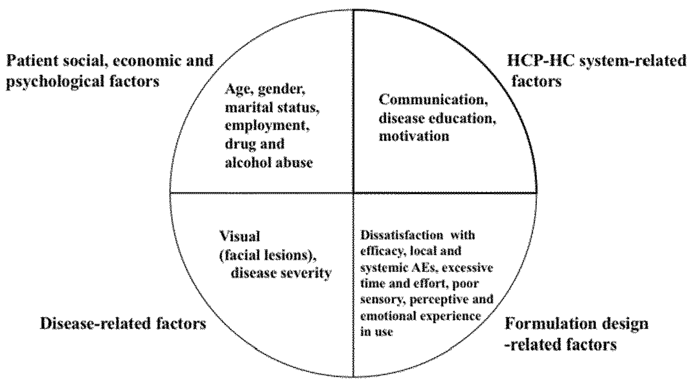


Passive topical drug delivery technology: Co-enhancer gel technology

Co-enhancer technology may be defined as incorporation of partition coefficient and diffusion coefficient enhancing functional ingredients into a topical formulation, thus to increase the skin permeation of an active agent.

More recent passive delivery co-enhancer technologies, addressing all of the principles embodied in Fick's First Law of Diffusion, represent the current the state-of-the art as they provide:

- an effective dose of one or more active agents at or near saturation in the non-volatile residual phase, (N-VRP); that phase remaining after evaporation of volatiles to equilibrium state
- inclusion of a partition coefficient enhancer in the N-VRP at an effective dose
- inclusion of a diffusion coefficient enhancer in the N-VRP at an effective dose, also at or near saturation in the non-volatile residual phase

US 8,541,470 describes such a co-enhancer gel composition for topical application of an NSAID, which comprises a solution of the NSAID in a carrier system comprising a polyhydric alcohol, a glycol ether and an ester of a higher fatty acid, the carrier system being present as a single phase at ambient temperatures. The NSAID may be diclofenac as diclofenac acid. The polyhydric alcohol may be a glycol such as propylene glycol and the glycol ether may be a diethylene glycol ether such as diethylene glycol monoethyl ether. The higher fatty acid ester is generally isopropyl myristate.

The formulations described in US 8,541,470 are designed from a non-volatile single-phase residual phase (N-VRP) comprising the active and functional partition coefficient, (for example, propylene glycol) and diffusion coefficient (for example, isopropyl myristate) co-enhancer excipients together with a cosolvent (diethylene glycol monoethyl ether). Formulation-dependent skin permeation of the active is primarily dependent upon:

- the dose of the partition coefficient enhancer
- the dose and degree of saturation of the active in the non-volatile single phase residual phase
- the dose and degree of saturation of the diffusion coefficient enhancer in the non-volatile single phase residual phase

To complete such co-enhancer-type gel formulations, highly volatile organic solvents, such as ethanol and isopropyl alcohol, optionally water, and a gelation polymer may be added to the non-volatile residual phase to ensure single-phase solubility, and appropriate macro-viscosity for ease of application in use. On application to the skin as a thin film, evaporation of the highly volatile solvents quickly reforms the essential physicochemistry of the non-volatile single-phase residual phase.

Although it was well known to consider the degree of saturation of the active, US 8,541,470 first describes the importance of the dose and degree of saturation of the diffusion coefficient enhancer, in this case isopropyl myristate, in the non-volatile residual phase. Figure 1 of US 8,541,470 is a ternary phase diagram which describes those compositions of the non-volatile single-phase residual phase in which the diffusion coefficient enhancer is at saturation. If the degree of saturation of the diffusion coefficient enhancer is low, a significant reduction in stratum corneum permeation of the diffusion coefficient enhancer, and thus of the active, is observed.

Despite their drug delivery benefits, co-enhancer formulations such as those described in US 8,541,470 are associated with poor rub-in and skin feel, being associated with tackyness and skin drying. It is widely acknowledged that aesthetic concerns, in particular poor skin feel, reduce the adherence to a method of medical treatment involving application of a topical formulation. Patients consistently report preference for creams over gels or ointments. Co-enhancer systems formulated as emollient creams would seem to address all of the formulation-related factors driving poor adherence. However, addition of the surfactant and emollient oil phase cream excipients tends to effect the performance of an included co-enhancer system, leading to poor permeation of the active through the skin barrier, and thus poor delivery of the active agent to the target site. Thus, a limitation of current topical co-enhancer gel technologies is their inability to incorporate emollients and yet maintain a high degree of saturation of critical functional ingredients and thus optimised skin permeation.

Without being bound by theory, we hypothesised that this was because the emollient oil phase of the cream has detrimental effects on the performance of the co-enhancer system. Particularly, emollient oil phase components comprising hydrocarbon structural elements might be expected to solubilise diffusion coefficient-enhancing excipients, such as those comprising alcohols, acids or ester derivatives of $C_8$-$C_{22}$ hydrocarbons. Ideally, the diffusion coefficient enhancer should be at or near saturated solubility in the non-volatile residual phase (N-VRP) of the formulation. Emollient excipients that lead to an increase in the solubility of the diffusion coefficient enhancer in the residual non-volatile emollient oil phase of the vehicle, such that the diffusion coefficient enhancer becomes grossly subsaturated, might be expected to adversely affect the partitioning of the diffusion coefficient enhancer into the stratum corneum barrier and to reduce skin permeation of an incorporated active by 5-10fold.

***Passive topical drug delivery technology: Co-enhancer silicone cream technology***

UK patent GB 2549418B "Topical formulations comprising dimethicone macromers" describes a topical formulation technology which aims to address all four of the formulation design-related adherence factors. The first three are biopharmaceutical factors concerning broadly, respective, the amount of dose absorbed, amount of dose applied and rate of dose absorbed. These requirements are met by use of a co-enhancer system comprising partition and diffusion coefficient enhancers.

The fourth factor is met by dispersing the glycol co-enhancer system within a silicone elastomer-silicone-fluid-silicone emollient continuous phase to form a cream. On application to the skin of glycol-co-enhancer silicone dispersions, the consumer experiences the soft and silky skin feel of the mixed silicone elastomer-silicone emollient fluid continuous phase. Loss of volatile silicones occurs quickly to give the perception of absorption into the skin.

In vitro skin permeation studies on human skin show that the partitioning of $C_{14}$ alcohol diffusion coefficient

enhancer into the stratum corneum barrier from the co-enhancer silicone cream technology is not impaired by inclusion of silicone emollients, such as caprylyl methicone. Figure 1, herein, shows the solubility of a range, from $C_8$ to $C_{22}$ of fatty alcohol, fatty acid and fatty ester diffusion coefficient enhancers in propylene glycol and the silicone emollient caprylyl methicone. From $C_8$ to $C_{10}$ the fatty acid and alcohol diffusion coefficient enhancers are highly soluble in propylene glycol and as such would show insignificant permeation into the skin barrier. Figure 2, herein, shows that only the $C_{12}$ and $C_{14}$ acid and $C_{14}$ alcohol have appropriate solubility in both propylene glycol and caprylyl methicone and, for this reason are preferred diffusion coefficient enhancers. Figure 3, herein, shows the solubility of a preferred diffusion coefficient enhancer $C_{14}$ alcohol in a range of common emollient esters and the silicone emollient, caprylyl methicone. The high solubility of the $C_{14}$ alcohol in the common emollient esters is highly consistent with the hypothesis that the partitioning of the $C_{14}$ alcohol into the stratum corneum barrier will be impaired such to reduced skin permeation of an incorporated active by 5-10fold.

The UK patent GB 2549418B describes topical cream formulations with enhanced skin permeation allowing efficient and predictable delivery of the active to the target site in the skin. Also, on application to the skin of the glycol-co-enhancer silicone emollient/elastomer cream, the consumer experiences the soft and silky skin feel of the mixed silicone elastomer-silicone fluid emollient continuous phase. Loss of volatile silicones occurs quickly to give the perception of absorption into the skin. This aesthetic technology is widely used in Premium Branded Cosmetic skin creams. However, in our consumer research, although consumers recognise and appreciate the experience of soft and silky skin and the perception of absorption into the skin as volatiles are lost, they still add *"but I don't know if it does any real good".*

Here we describe, in addition to enhanced skin permeation and Premium Class aesthetics, formulations comprising a primary active(s) together with an ancillary user adherence-improving skin barrier restoring system comprising nicotinamide and polyhydroxy acid, to restore the skin to its natural healthy state to give the user a reason to believe in the treatment and the motivation to continue to adhere to the primary treatment regimen.

Both nicotinamide, logP O/W -0.38, and polyhydroxy acids (lactobionic acid/gluconolactone logP O/W -4.8) are hydrophilic compounds and so have little or no potential to solubilize, for example, diffusion coefficient enhancers such as $C_{14}$ alcohol, logP O/W > +4.00. For the same reason, both partition into the glycol dispersed phase and have extremely low solubility in the continuous silicone phase. Both have a pKa of ~3.5 at which pH, by definition, they are 50% unionized, thus more skin permeable. However, as pH is decreased, percent unionized increases for the polyhydroxy acids but decreases for nicotinamide. As pH is increased the reverse happens. As we will demonstrate in our examples, this gives flexibility to optimize pH; for example, depending upon the chemistry of the primary active(s). Also, a continuation of our N-VRP formulation concept, we are able, optionally, to use volatile buffer technology to achieve different, individually optimized, in-pack and equilibrium phase pH as described in US 10,028,927. WO 2016/139471 A1 discusses the low functionality of skin co-enhancer technologies (combinations of partition (PC) and diffusion coefficient (DC) skin permeation enhancers) when formulated into a cream base. It discloses that the emollient hydrocarbon oil phase of the cream solubilised the DC enhancer to significantly reduce its partitioning into its target, the stratum corneum. Studies on the solubility of a wide range of fatty acid, fatty alcohol and fatty acid esters in silicone emollients, such as caprylyl methicone, and propylene glycol (PC enhancer) were conducted leading to the selection of $C_{12}$ and $C_{14}$ alcohols and $C_{12}$ acid (DC enhancers), preferably propylene glycol (PC enhancer) and alkyl methicones such as caprylyl methicone as a preferred combination to restore functionality.

In light of the problems discussed above, various proposals have been made, but there remains a need for an improved composition which address one or more of the problems presented by prior art arrangements. In this regard, the present invention seeks to provide a composition which preferably addresses one or more of the problems presented by prior art arrangements.

### *Summary of the Invention*

In accordance with a first aspect of the present invention there is provided a composition for topical application comprising:

a primary active agent for topical treatment of the skin, and
a user adherence-improving skin barrier restoring combination of:

1.0 to 5%w/w of nicotinamide
1.0 to 5%w/w of polyhydroxy acid
10 to 60%w/w of a partition coefficient enhancer (PC enhancer), having a structure of the general formula: CnH2n+2O2 where n represents an integer from 3 to 5 inclusive,
a diffusion coefficient enhancer (DC enhancer) selected from the group consisting of a C12 to C14 straight chain fatty acid and a C14 straight chain primary alcohol,
a first dimethicone macromer mixture including a dimethicone macromer and a hydrocarbyl methyl siloxane

emollient selected from the group consisting of an alkyl methyl siloxane, an aryl methyl siloxane and an alkyl aryl methyl siloxane, and
a second dimethicone macromer mixture including a methyl siloxane compound and a cross-linked dimethicone macromer;
wherein the composition comprises less than 15 % water by weight.

Preferably, the composition includes one or more active agents.
The composition of the present invention has aesthetics associated with that found using Premium cosmetic and cosmeceutical technologies yet contains a functional co-enhancer active delivery system. In addition, the user adherence-improving skin barrier restoring combination of nicotinamide and polyhydroxy acid restore the skin surface to its natural healthy state and is perceived by users with continued us and thus drives adherence. Accordingly, patient adherence to a method of treatment involving application of the composition is further increased resulting in a better medical outcome.
Also disclosed herein is a cosmetic composition comprising any of the compositions described herein and one or more pharmaceutically acceptable carriers or excipients.
In a further aspect, the invention provides a composition as disclosed herein for use in therapy.
Also disclosed herein is a method of preventing, reducing the likelihood of, alleviating or treating a medical condition in the human or animal body comprising the topical administration in a therapeutically effective amount of a composition described herein.
According to a further aspect of the present invention, there is provided a composition as described herein for use in the prevention, alleviation or treatment of a medical condition of the human or animal body.
The medical condition may be selected from the group consisting of conditions associated with or caused by one or more of pain and/or inflammation, pigmentation, pruritus, acne, eczema, psoriasis, rosacea, skin blistering diseases such as bullous pemphigoid, nappy rash, dry skin, microbial conditions including fungal and/or bacterial conditions such as skin infections including yeast infections and dermatophyte infections, viral infections of the skin or mucosa, warts, dry or ageing skin, hypoandrogenism, immunological conditions, sun spots, actinic keratosis, basal cell and squamous cell skin cancers and melanoma, alopecia and dermatitis due to radiation therapy. The medical condition is generally treated by topical application.
Also disclosed herein isa method of forming the composition as described herein. The composition is generally applied to the biological membrane, in particular the skin of the human or animal body, including the mucous membranes of the human or animal body.

***Brief Description of the Drawings***

The invention will now be further described with reference to the accompanying drawings in which:

Figure 1 shows the solubility of a range, from C8 to C22 of fatty alcohol, fatty acid and fatty ester diffusion coefficient enhancers in propylene glycol and the silicone emollient caprylyl methicone;

Figure 2 shows that only the C12 and C14 acid and C14 alcohol have appropriate solubility in both propylene glycol and caprylyl methicone and, for this reason are preferred diffusion coefficient enhancers;

Figure 3, shows the solubility of a preferred diffusion coefficient enhancer C14 alcohol in a range of common emollient esters and the silicone emollient, caprylyl methicone;

Figure 4 shows the dose response of ceramide synthesis to NAM added to human keratinocytes in vitro (Figure 4a) and the dose response of PARP-1 inhibition to NAM in vitro (Figure 4b);

Figure 5 shows data on the steady state flux achieved (n=6) from 8-24 hours following the lag time;

Figure 6 shows the results of a questionnaire which explored the experience of subjects in use of a cream; cosmetic properties, easy of application, efficacy and tolerance;

Figure 7 shows materials used; and

Figure 8 shows the chemical structure and stability of nicotinamide (Figure 8a) and the result of 40C storage for 6 months (Figure 8b).

***Detailed Description of the Invention***

It will be appreciated that aspects, embodiments and preferred features of the invention have been described herein in a way that allows the specification to be written in a clear and concise way. However, unless circumstances clearly dictate otherwise, aspects, embodiments and preferred features can be variously combined or separated in accordance with the invention. Thus, preferably, the invention provides a device having features of a combination of two or more, three or more, or four or more of the aspects described herein. In a preferred embodiment, a device in accordance with the invention comprises all aspects of the invention.

Within the context of this specification, the word "about" means plus or minus 20%, more preferably plus or minus 10%, even more preferably plus or minus 5%, most preferably plus or minus 2%.

Within the context of this specification, the word "substantially" means preferably at least 90%, more preferably 95%, even more preferably 98%, most preferably 99%.

Within the context of this specification, the word "comprises" means "includes, among other things" and should not be construed to mean "consists of only".

Within the context of this specification, the term "active agent" means a molecule having pharmaceutical activity. The term includes pharmaceutically active compounds.

By an "effective" amount or "therapeutically effective amount" is meant an amount of one or more active substances which, within the scope of sound medical judgment, is sufficient to provide a desired effect without excessive toxicity, irritation, allergic response, or other problem or complication, commensurate with a reasonable benefit/risk ratio.

All numerical values provided preferably incorporate 10% by weight less than and 10% by weight more than the numerical value provided.

A partition coefficient is the ratio of amounts of a substance in a mixture of two immiscible phases at equilibrium. The partition coefficient may be calculated according to the expression below:

$$K_{partition} = [X_{(phase\ 1)}]/[X_{(phase\ 2)}]$$

Where K is the partition coefficient, X is the substance, $X_{(phase\ 1)}$ is the amount of substance in the first phase and $X_{(phase\ 2)}$ is the amount of substance in the second phase.

As used herein, a partition coefficient (PC) enhancer increases the partition coefficient of an active agent between the non-volatile residual phase of the composition and the stratum corneum barrier layer of the skin, thus enhancing the penetration of the active agent to the target site in or below the skin.

As used herein, a diffusion coefficient (DC) enhancer increases the diffusion coefficient of an active agent within the stratum corneum barrier layer of the skin, thus enhancing the penetration of the active agent to the target site in or below the skin.

The partition coefficient (PC) enhancer and diffusion coefficient (DC) enhancer affect penetration of the active agent to the target site in or below the skin as defined by Fick's First Law of Diffusion

$$F = Cv * PC * DC/h$$

where F is the flux, the mass of agent penetrating the stratum corneum per unit time per unit area, Cv is the concentration of active in solution in the non-volatile residual phase, PC refers to the partition coefficient enhancer effect, DC refers to the diffusion coefficient enhancer effect and h is the thickness of the stratum corneum barrier.

Preferably, the modified form of Fick's First Law of Diffusion is used in composition design:

$$F =\sim DS_V * \text{sat sol SC} * DC/h$$

where F is the flux, the mass of agent penetrating the stratum corneum per unit time per unit area, DSv is the degree of saturation of the active in solution in the non-volatile residual phase, sat sol SC is the saturated solubility of the active in the stratum corneum (as affected by the partition coefficient enhancer), DC refers to the diffusion coefficient enhancer effect and h is the thickness of the stratum corneum barrier.

For the purpose of the current invention; by the term highly volatile are described liquids such as Dow Corning® Q7-9180 Silicone Fluid (0.65 cSt, hexamethyldisiloxane and 1.0 cSt, octamethyltrisiloxane), ethanol, isopropyl alcohol and water which have half-lives of evaporation at skin temperature of under 5 minutes, by the term volatile are described liquids such as cyclopentacyloxane (D5) which have half-lives of evaporation at skin temperature of approximately 1 hour and by the term non-volatile are described liquids such as caprylyl methicone and polydimethylsiloxanes (Dow

Corning Q7-9120 silicone fluids) which have half-lives of evaporation at skin temperature of approximately 6-24 hours. Compositions of the current invention are intended for application once or twice a day.

The term "non-volatile residual phase" describes the composition of the composition remaining after evaporation of volatile solvents such as Dow Corning® Q7-9180 Silicone Fluid (0.65 cSt and 1.0 cSt), ethanol, isopropyl alcohol and water and thus generally comprises the active agent, PC enhancer, DC enhancer and medium and high molecular weight elastomer materials. As used herein, the nominally volatile silicones ST cyclomethicone 5-NF and alkyl methyl siloxanes such as caprylyl methicone are considered relatively medium / non-volatile in the context of the time frame of topical application and absorption.

The term "carbinol" is used to refer to a hydroxyl functional group attached to a carbon atom. The carbon atom may be attached to a carbon atom (in particular a carbon atom forming part of a hydrocarbon group), a non-carbon atom including Si, N and O.

The term "small alkyl group" refers to an alkyl group having a carbon backbone of 1 to 6 carbon atoms, typically 1 to 4 carbon atoms.

In accordance with a first aspect of the present invention there is provided a composition for topical application comprising:

a primary active agent for topical treatment of the skin, and
a user adherence-improving skin barrier restoring combination of:

1.0 to 5%w/w of nicotinamide
1.0 to 5%w/w of polyhydroxy acid
10 to 60%w/w of a partition coefficient enhancer (PC enhancer), having a structure of the general formula: $C_nH_{2n+2}O_2$ where n represents an integer from 3 to 5 inclusive,
a diffusion coefficient enhancer (DC enhancer) selected from the group consisting of a $C_{12}$ to $C_{14}$ straight chain fatty acid and a $C_{14}$ straight chain primary alcohol,
a first dimethicone macromer mixture including a dimethicone macromer and a hydrocarbyl methyl siloxane emollient selected from the group consisting of an alkyl methyl siloxane, an aryl methyl siloxane and an alkyl aryl methyl siloxane, and
a second dimethicone macromer mixture including a methyl siloxane compound and a cross-linked dimethicone macromer;
wherein the composition comprises less than 15 % water by weight.

Generally, the composition is a polyol-in-silicone dispersion cream or serum.

According to one embodiment, there is provided a composition suitable for the topical application of the active agent, the composition comprising the active agent in a carrier, wherein the carrier comprises a partition coefficient enhancer comprising a secondary or primary alcohol group, a diffusion coefficient enhancer selected from the group consisting of a $C_{12}$ to $C_{14}$ fatty acid and a $C_{14}$ alcohol, a first dimethicone macromer mixture and a second cross-linked dimethicone macromer mixture.

Generally, the composition comprises a solution, suspension or dispersion of the active agent in the carrier. Preferably, the active is in solution.

The composition of the present invention achieves and improves efficacy of active delivery, itself promoting adherence. In particular, the composition of the present invention provides enhanced skin penetration compared to known compositions, allowing a high therapeutic free active agent concentration at the target site to be achieved and sustained. Generally, the free active agent concentration at the target site is significantly above the $EC_{50}$ (half maximal effective concentration). The efficacy of the delivery of the active agent to the target site is maximised, producing an efficacious, robust treatment regime. Severe conditions may thus be treated or a more robust clinical response may be achieved in the general patient population.

In addition, adherence to use of the composition is increased due to us silicone fluid and silicone emollients which enhance skin feel and perceived rub-in. With continued use the ancillary user adherence-improving skin barrier restoring system gives a visible improvement in skin heath, a reason to believe, to sustain adherence over long or intense treatment periods

The extent of absorption of the majority of dermatological actives is in the range 1-5% of the dose applied on normal skin. At permeable skin sites the dose absorbed increases markedly and is associated with local and systemic adverse effects. The composition of the present invention provides controlled dosing of the active agent, reducing the potential of adverse effects, this also promotes adherence. As the delivery of the active agent to the target site is optimized through the use of the composition of the present invention, the dose of active agent contained in the composition may be reduced, typically to 5-10% of current doses. This reduces the risk and fear of adverse effects, maximizing adherence to the method of medical treatment, and reduces the waste and associated cost of excessive dosing in less efficacious

drug delivery systems. Historically, this has been seen as an economic issue of wastage, to which might now be added the environmental issue of pollution caused by washing off and release into the environment of the excess drug. Primarily, this is an important therapeutic issue.

The composition of the present invention is sufficiently efficacious to allow reduced dosing, reducing the necessity of applying known compositions 2 to 6 times per day to 1 to 2 times per day, generally once daily. Single daily treatments or intermittent treatment regimens are universally appreciated by patients and consumers and are supported in science by drug depot formation within the stratum corneum. The composition of the present invention allows the possibility of such dosage regimes whilst retaining the efficacy of the treatment and this promotes adherence.

It has consistently been found that users prefer creams to gel and ointment compositions.

Emollients are the main functional aesthetic components within creams usually requiring surfactant and wax co-excipients for effective dispersion. Emollients bring cosmetic attributes of spreadability, slip and smoothness, which drive adherence. As described, most emollients contain hydrocarbon structural elements, which might be expected to adversely affect the performance of diffusion coefficient-enhancing excipients by solubilizing them within the residual non-volatile phase of the vehicle.

The composition generally comprises a cosmetic, cosmeceutical or pharmaceutical active.

Typically, the composition comprises a partition coefficient enhancer selected from the group consisting of the general formula $C_nH_{2n+2}O_2$ where n= 3-5 inclusive.

Typically, the composition comprises a hydrocarbyl methylsiloxane emollient compound selected from the group consisting of caprylyl methicone, lauryl methicone, stearyl methicone and caprylyl trimethicone; suitably the hydrocarbyl methylsiloxane emollient is caprylyl methicone.

The composition may also include a highly volatile solvent selected from the group consisting of hexamethyldisiloxane, octamethyltrisiloxane, ethanol, isopropyl alcohol and water.

*Hydrocarbyl Methyl Siloxane Emollient*

A limitation of current coenhancer technologies is their inability to incorporate emollients and yet maintain a high degree of saturation of the active and also a high degree of saturation of the diffusion coefficient enhancer, thus overall to ensure optimized skin penetration. It has surprisingly been found that polyol-in-silicone emulsions provide a structural matrix for incorporation of chemically diverse excipients. In particular, the inventors have identified the chemical class of hydrocarbyl methysiloxane emollient excipients as being of particular interest due to their compatibility with silicone oils.

The composition of the present invention includes an optionally substituted hydrocarbyl methyl siloxane emollient. The emollients for use in the composition of the present invention contain hydrocarbon and methyl siloxane backbone structural elements. The methyl siloxane backbone contributes an additional light, smooth, silky, powdery feel to further improve the aesthetics of the resultant composition. The methyl siloxane backbone may be in the form of a straight chain-, branched- or cyclo- siloxane compound. The hydrocarbyl portion of the emollient compound may be saturated or unsaturated and may include alkyl, alkenyl, alkynyl, haloalkyl, carbocyclyl, for example heterocyclyl, aryl and heteroaryl groups. The hydrocarbyl portion of the emollient compound may be straight chain or branched and may be substituted or unsubstituted.

According to one embodiment, one or more carbon or silicon atoms of the hydrocarbyl methyl siloxane group may be independently substituted with one or more of the group consisting of small hydrocarbyl group, typically small alkyl group (suitably 1 to 6 carbon atoms), cycloalkyl group, $C_{1\text{ to }6}$ alkoxy, halogen, trifluoromethyl, cyano, thio, amino, nitro, oxo and hydroxyl.

Typically, the hydrocarbyl methyl siloxane group is substituted with one or more small alkyl group, halogen group and/or hydroxyl group; generally, one or more small alkyl group.

Generally, the hydrocarbyl methyl siloxane group is unsubstituted.

The hydrocarbyl methyl siloxane generally has the structure shown below.

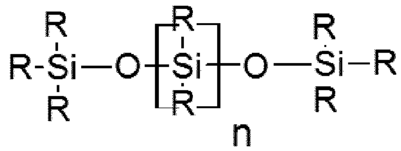

Wherein each R group independently represents a hydrocarbyl group or hydrogen, at least one R group represents methyl, and at least one R group represents a hydrocarbyl group.

Generally, 1 to 3 R groups independently represent a hydrocarbyl group comprising 2 or more carbon atoms, typically selected from the group consisting of an alkyl group having a carbon backbone of two or more, an aryl group

and an alkyl group attached to an aryl group. Typically, each alkyl group is a small alkyl group. Suitably 1 or 2 R groups independently represent a hydrocarbyl group comprising 2 or more carbon atoms.

Typically, each R group represents methyl or a hydrocarbyl group comprising 2 or more carbon atoms. Suitably each R group represents methyl, an alkyl group having a carbon backbone of two or more, an aryl group or an alkyl group attached to an aryl group. Typically, the, or each alkyl group is a small alkyl group.

According to one embodiment, the hydrocarbyl methyl siloxane refers to compounds having the structure as shown below

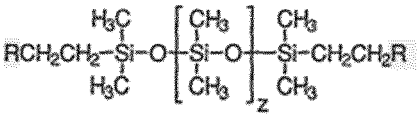

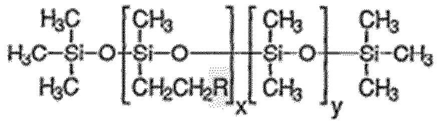

Wherein R represents a hydrocarbyl group, generally an alkyl or aryl group.

Particular mention may be made of alkyl methyl siloxanes, aryl methyl siloxanes and alkyl aryl methyl siloxanes. According to one embodiment the emollient is an alkyl methyl siloxane or an alkyl aryl methyl siloxane. Suitable emollients include cetyl dimethicone, stearyl dimethicone, phenyl dimethicone, caprylyl methicone (available, for example, from Dow Corning as TI-2021 AMS), myristyl methicone, stearyl methicone and lauryl methicone (available, for example, from Siltec), caprylyl trimethicone (available, for example from Clariant) and decamethylcyclopentasiloxane.

The dominant chemistry and state of matter of any hydrocarbyl methyl siloxane (including alkyl methyl siloxane and alkyl aryl methyl siloxane) may be estimated from the ratio of hydrocarbon to methyl siloxane and from the chain length of the hydrocarbon. Thus, the principles of selection of appropriate alkyl and alkyl aryl methyl siloxanes are understood by those skilled in the art. Alkyl methyl siloxane and alkyl aryl methyl siloxane which are liquids, by virtue of the lower percentage of hydrocarbon and lower hydrocarbon chain length, are generally preferred as emollients. Such liquid alkyl methyl siloxanes include caprylyl methicone, lauryl methicone, stearyl methicone and caprylyl trimethicone.

Typically, the hydrocarbyl methyl siloxane compound has a number average molecular weight of less than 1000, suitably less than 800, generally less than 500. According to one embodiment, the hydrocarbyl methyl siloxane compound has a number average molecular weight of 100 to 700, generally 200 to 400.

Studies on the solubility of actives and functional diffusion coefficient (DC) enhancers may be conducted to select those hydrocarbyl methylsiloxane emollients suitable for use in the current invention. The composition of the present invention comprises more than one methyl siloxane-containing compound, in particular more than one hydrocarbyl methyl siloxane, typically more than one alkyl-, aryl- or aryl alkyl-methyl siloxane compound (generally having a number average molecular weight of less than 1000).

Generally, the hydrocarbyl methyl siloxane consists of or comprises caprylyl methicone.

Suitably the composition includes one or more cyclomethicone compounds such as cyclopentasiloxane, in particular decamethylcyclopentasiloxane.

The saturated solubility of the typical corticosteroids fluticasone propionate (FP) and mometasone furoate (MF) in a range of liquid silicones suitable for use in the present invention, including the alkyl methylsiloxane emollient caprylyl methicone, is appropriately low. Thus, even at greatly reduced active doses, a high degree of saturation of the active may be achieved in the non-volatile residual phase in the presence of an emollient.

*Diffusion Coefficient-Enhancing Excipients*

As described previously, diffusion coefficient-enhancing excipients (DC enhancers) commonly comprise alcohol, acid or ester derivatives of $C_8$-$C_{22}$ hydrocarbons. Figures 1 to 3 show the solubility of straight chain saturated $C_8$-$C_{22}$ alcohol, acid and ester derivatives in caprylyl methicone, the partition coefficient enhancer propylene glycol, hydrocarbon-based emollient esters and 5-NF (decamethylcyclopentacycloxane, silicone fluid) at 23-25 °C.

In the acid and alcohol series, Figure #1, as carbon chain length increases beyond $C_{16}$, solubility in caprylyl methicone decreases to less than 0.1% w/w at $C_{18}$-$C_{22}$, such that these higher carbon chain lengths may be considered unsuitable for use as the DC enhancer in the composition of the present invention owing to low solubility-dissolution constraints. Conversely, lower carbon chain length acids and alcohols such as $C_8$ and $C_{10}$ alcohols and $C_8$ and $C_{10}$ acids may be considered to be far too soluble in hydrocarbyl methyl siloxanes including caprylyl methicone, such that their performance as diffusion coefficient-enhancing excipients would be adversely affected. Similarly, medium chain

(for instance $C_{14}$-$C_{16}$) isopropyl esters are far too soluble in hydrocarbyl methyl siloxanes such as caprylyl methicone to allow performance as DC enhancers. For both of these classes of DC enhancer, up to 50% DC enhancer would be required to be at or near saturation in the non-volatile residual phase. In contrast, the $C_{12}$-$C_{14}$ acids and $C_{14}$ alcohol have appropriate solubility in caprylyl methicone.

Figure #3 shows that $C_{14}$ alcohol has appropriate solubility in a range of hydrocarbyl methylsiloxanes and also in 5-NF (decamethylcyclopentacycloxane).

Figure #1 shows that the solubility of short chain $C_8$-$C_{10}$ fatty acids and short chain $C_8$-$C_{12}$ fatty alcohols in the partition coefficient (PC) enhancer propylene glycol may be considered too high, such that the performance of such alcohols and fatty acids as DC enhancers would be adversely affected. Conversely, from $C_{16}$-$C_{22}$ solubility is too low to allow adequate performance. Only the $C_{12}$-$C_{14}$ acids and $C_{14}$ alcohol have appropriate solubility in propylene glycol.

As shown in detail, Figure #2 shows that only the $C_{12}$-$C_{14}$ acids and $C_{14}$ alcohol have appropriate total solubility in propylene glycol and caprylyl methicone.

Figure 3 shows the solubility of a preferred diffusion coefficient enhancer $C_{14}$ alcohol in a range of common emollient esters and the silicone emollient, caprylyl methicone. The high solubility of the $C_{14}$ alcohol in the common emollient esters is highly consistent with the hypothesis that the partitioning of the $C_{14}$ alcohol into the stratum corneum barrier will be impaired such to reduced skin permeation of an incorporated active by 5-10fold.

As an example of the outline design of a simple co-enhancer system comprising propylene glycol, and, for example, a $C_{14}$ alcohol diffusion coefficient enhancer with caprylyl methicone, we might first consider that the concentration of propylene glycol and caprylyl methicone in the final composition (otherwise consisting of non-solvents) to be 25% of each. On this basis, the total amount of $C_{14}$ alcohol to saturate both of these phases would be 2.44%/4 +2.30%/4 = 1.185%, approximately 1.2%. Based on experience, concentrations of diffusion coefficient enhancer around the range 1-5% w/w are required, thus in the optimum range for the selected diffusion coefficient enhancers.

The DC enhancers for use in the composition disclosed herein are generally selected from the group consisting of $C_{12}$-$C_{16}$ acids and $C_{12}$-$C_{14}$alcohols, typically $C_{12}$-$C_{14}$ straight chain fatty acids and $C_{14}$ straight chain primary alcohols. This select group of compounds are suitably soluble in both the hydrocarbyl methyl siloxane and the partition coefficient enhancer, allowing strong diffusion coefficient enhancement and efficient epidermal delivery of the active agent

The fatty acids for use in the composition disclosed herein have a carbon backbone of 12 to 16 carbon atoms, preferably 12 to 14 carbon atoms. The alcohols for use in the composition disclosed herein have a carbon backbone of 12 to 14 carbon atoms, generally 14 carbon atoms. In some embodiments, the fatty acids/alcohols may comprise substituents from the carbon backbone which may include additional carbon atoms. In particular, the fatty acids/alcohols may comprise hydrocarbyl substituents including 1 to 3 carbon atoms.

Generally, the fatty acids/alcohols for use in the composition of the present invention are not substituted.

The DC enhancer is generally an optionally substituted $C_{12}$-$C_{16}$ fatty acid which may be saturated or unsaturated. Generally, the fatty acids/alcohols for use in the composition of the present invention are saturated.

Typically, the DC enhancer is a saturated fatty acid having a carbon backbone of 12 to 14 carbon atoms.

According to one embodiment, the fatty acid/alcohol is unsaturated and the two carbon atoms in the carbon backbone adjacent the/each double bond may be in a cis or trans configuration, generally in a trans configuration.

Alternatively, the DC enhancer may be an optionally substituted $C_{14}$ alcohol which may be saturated or unsaturated. Generally, the DC enhancer is a straight chain primary alcohol.

Generally, the DC enhancer is a saturated alcohol having a carbon backbone of 12 to 14 carbon atoms, generally 14 carbon atoms. According to one embodiment, the acid or alcohol DC enhancer may be substituted. One or more of the carbon atoms may independently be substituted with one or more $C_1$ to $C_6$ hydrocarbyl group, generally $C_1$ to $C_4$ alkyl group.

Suitable substituted $C_{12}$-$C_{16}$ acids and $C_{12}$-$C_{14}$ alcohols may be readily identified by their combined solubilities in the hydrocarbyl methyl siloxane and the partition coefficient enhancer.

According to one embodiment, the DC enhancer is selected from the group consisting of $C_{12}$-$C_{14}$ straight chain fatty acids and $C_{12}$-$C_{14}$ alcohols; in particular the group consisting of $C_{12}$-$C_{14}$ straight chain fatty acids and $C_{14}$ straight chain primary alcohols.

Generally, the DC enhancer is selected from the group consisting of lauric acid, myristic acid and myrystyl alcohol.

The amount of DC enhancer required depends on the other components of the composition, in particular, as described, the identity and amounts of the hydrocarbyl silicone emollient used and the PC enhancer used.

Generally, the composition of the present invention comprises less than about 10%w/w DC enhancer, typically less than about 5%w/w DC enhancer, suitably less than about 4%w/w DC enhancer. The composition suitably comprises at least about 0.5%w/w DC enhancer, typically at least about 0.7%w/w DC enhancer, generally at least about 1%w/w DC enhancer.

According to one embodiment, the composition comprises less than 10%w/w DC enhancer, generally 1 to 4%w/w, typically less than 2%w/w, suitably 0.5 to 2%w/w DC enhancer.

*Partition Coefficient-Enhancing Excipients*

The inclusion of a partition coefficient enhancer (PC enhancer) increases the solubility of the active agent in the stratum corneum barrier and thus increases skin penetration.

The composition disclosed herein comprises at least one PC enhancer, in particular at least one OH-terminated PC enhancer. Generally, the PC enhancer is a primary or secondary alcohol, in particular a diol or polyol compound. In particular the PC enhancer has a structure of the general formula: $C_nH_{2n+2}O_2$ where n represents an integer from 3 to 6 inclusive.

Typically, the PC enhancer has a number average molecular weight of 1500 or less, typically 750 or less, suitably 150 or less.

Typically, the PC enhancer is by common name and *IUPAC names*: propylene glycol, *propane-1,2-diol, n=3;* butylene glycol, *butane-1,3-diol, n=4; or* pentylene glycol, *pentane 1,5 diol, n=5.*

Generally, the partition coefficient enhancer is propylene glycol.

Generally, a second mutually miscible PC enhancer/cosolvent is present in the composition to modulate the degree of saturation of the active in the residual phase. This may take the form of a diol, triol, alcohol, ether-alcohol, or alkyl pyrrolidone. Suitable diols are of the general formula $C_nH_{2n+2}O_2$ where n= >6. Suitable alcohols are of the general formula $C_nH_{2n+2}O$, where n= 2-3 inclusive. Suitable INCI-listed ether-alcohols are of the general formula $C_nH_{2n+2}O_3$, for example: dipropylene glycol, $C_6H_{14}O_3$, Transcutol (diethylene glycol monoethyl ether), $C_6H_{14}O_3$, butoxydiglycol, $C_8H_{16}O_3$; diethylene glycol, $C_4H_{10}O_3$; dimethoxydiglycol, $C_6H_{14}O_3$ and methoxydiglycol, $C_5H_{12}O_3$. Further suitable INCI-listed ether-alcohols are of the general formula $C_nH_{2n+2}O_2$, for example: butoxyethanol, $C_6H_{14}O_2$; ethoxyethanol, $C_4H_{10}O_2$; ethyl hexanediol, $C_8H_{18}O_2$, methoxyethanol, $C_3H_8O_2$ and methoxyisopropanol, $C_4H_{10}O_2$. A suitable alkyl pyrrolidone is N-methyl pyrrolidone. A suitable triol is glycerol.

The amount of PC enhancer required depends upon the extent of skin penetration enhancement required and on the other components of the composition, in particular the identity and amounts of the hydrocarbyl silicone emollient used and the DC enhancer used.

Generally, the composition disclosed herein comprises less than about 70%w/w PC enhancer, suitably less than about 50%w/w PC enhancer, typically less than about 40%w/w PC enhancer, more suitably less than about 30%w/w PC enhancer. The composition suitably comprises at least about 10%w/w PC enhancer, typically at least about 20%w/w PC enhancer.

According to one embodiment, the composition includes 10 to 60%w/w PC enhancer.

*First Dimethicone Macromer Mixture*

The first dimethicone macromer mixture includes a dimethicone macromer and a hydrocarbyl methyl siloxane emollient selected from the group consisting of an alkyl methyl siloxane, an aryl methyl siloxane and an alkyl aryl methyl siloxane.

The first dimethicone macromer mixture generally includes a polyglycol dimethicone macromer surfactant.

The first dimethicone macromer mixture typically includes a polyglycol dimethicone macromer surfactant having a number average molecular weight of more than 1000 (typically more than 2000) and a hydrocarbyl methyl siloxane emollient (generally an alkyl methyl siloxane) having a number average molecular weight of less than 500.

The first dimethicone macromer mixture may include 5 to 30%w/w polyglycol dimethicone macromer surfactant; generally, 10 to 20%w/w; typically, 12 to 19%w/w.

Generally, the polyglycol dimethicone macromer surfactant comprises one or more polyalkylsiloxane portions (generally one or more dimethylsiloxane portions) and one or more oxypropylene or oxyethylene portions. Suitably the dimethicone macromer may comprise one or more copolymers of ethylene oxide and propylene oxide.

In particular, the first polyglycol dimethicone macromer surfactant mixture includes a polyglycol dimethicone macromer cross-linked with a polyalkylene oxide compound (generally a polyethylene glycol compound, a polypropylene glycol compound or a copolymer of ethylene oxide and propylene oxide).

The first dimethicone macromer mixture may include a polyglycol dimethicone macromer surfactant selected from the group consisting of PEG dimethicone PPG crosspolymer and PEG dimethicone bis-isoalkyl PPG crosspolymer.

Generally, the dimethicone macromer comprises one or more terminal carbinol groups.

The dimethicone macromer may have the structure of a dihydroxy terminated block copolymer oxyethylene - dimethylsiloxane - oxyethylene; oxypropylene - dimethylsiloxane - oxypropylene or caprolactone - dimethylsiloxane - caprolactone of different molecular weights containing different weight % of non-siloxane units. Optionally such block co-polymers may include pendant oxyalkylene groups. Typically, the block copolymer may be cross-linked.

The dimethicone macromer may have a non-siloxane content of 20 to 70 wt. %.

The number average molecular weight of the, or each dimethicone macromer is generally 800 or more, suitably 1000 or more, typically 1000 to 10000, suitably 2000 to 7000.

According to one embodiment, the greater the number average molecular weight of the dimethicone macromer, the greater the non-siloxane weight percentage content.

The dimethicone macromer may be in the form of a dimethicone-containing central linking group, linked to two to five polyoxyalkylene groups, generally two to five OH-terminated polyoxyalkylene groups. Generally, the dimethicone macromer comprises three, four or five polyoxyalkylene groups.

Generally, the polyoxyalkylene groups are polymers and/or copolymers of ethylene oxide and/or propylene oxide. The polyoxyalkylene groups may comprise primary or secondary hydroxyl groups or a mixture thereof.

According to one embodiment, the dimethicone macromer comprises a dimethicone backbone with one or more pendant polyoxyalkylene groups selected from the group consisting of oxyethylene groups, oxypropylene groups and copolymers of oxyethylene and oxypropylene. Generally the pendant polyoxyalkylene groups include 5 to 50 repeat units, suitably 10 to 30 repeat units, typically 15 to 20 repeat units.

Typically, the dimethicone macromer surfactant comprises a dimethicone backbone with one or more pendant oxyethylene groups and one or more pendant oxypropylene groups.

For example, the dimethicone macromer may have the general structure below of the pendant polymer PEG/PPG-18/18 Dimethicone where m and n independently represent an integer from 10 to 30, suitably 18.

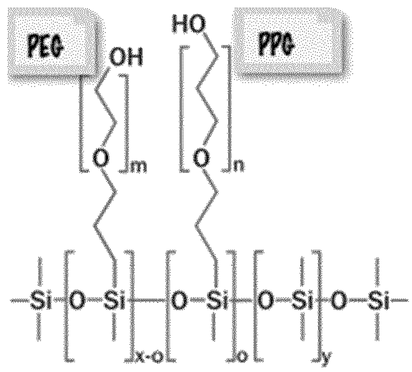

Alternatively or additionally, the dimethicone macromer may comprise a dimethicone backbone with one or more pendant block copolymers of oxyethylene and oxypropylene. For example, the dimethicone macromer may have the general structure below where m and n independently represent an integer from 10 to 30, suitably wherein m = 20 and n = 15. Such macromers are commercially available under the registered trade mark Silsoft ® SF1540.

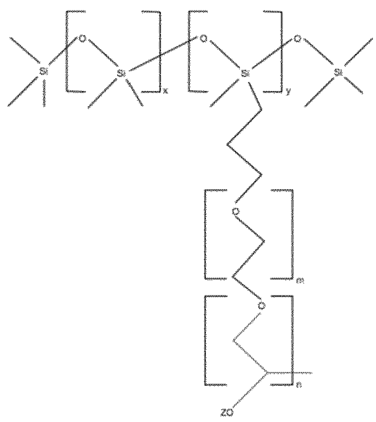

Alternatively, or additionally, the dimethicone macromer may comprise a dimethicone backbone with one or more oxyethylene end blocks, generally one or more PEG end blocks, typically two PEG end blocks. For example, the structure of an end-block copolymer, bis-PEG-10 Dimethicone is shown below.

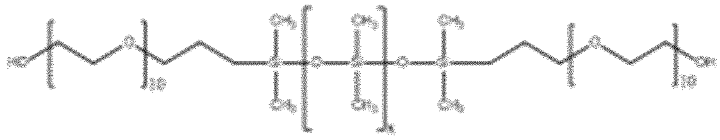

Advantageously, the dimethicone macromer is cross-linked. In particular, a first dimethicone backbone is cross-linked with a second dimethicone backbone through one or more cross linking groups, for example substituted or unsubstituted hydrocarbyl groups, in particular substituted or unsubstituted alkylene groups. In particular, the cross linking groups may be selected from the group consisting of unsubstituted alkylene groups and oxyalkylene groups, in particular one or more oxyethylene or one or more oxypropylene groups. Generally the oxyalkylene cross-linking group includes 5 to 50 repeat groups. The dimethicone macromer may also include one or more pendant oxyalkylene groups which do not cross-link the dimethicone backbones.

Dimethicone PEG-10 crosspolymer is an example of a polyethylene glycol cross-linked dimethicone macromer.

Dimethicone/ PEG-10 crosspolymer

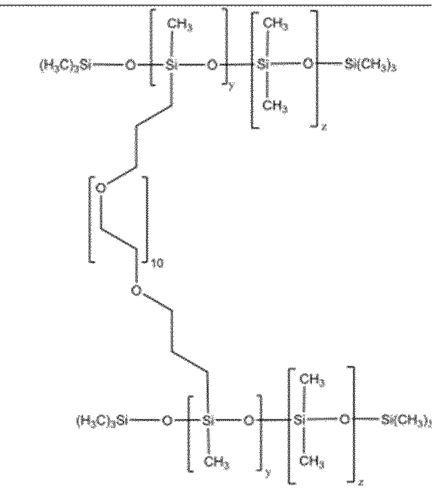

PEG-12 dimethicone crosspolymer is an example of a hydrocarbon diene cross-linked copolymer emulsifier with pendant polyethylene glycol groups.

PEG-12 dimethicone crosspolymer

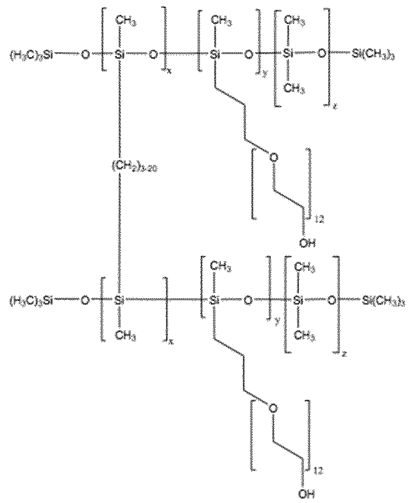

According to one embodiment, the dimethicone copolymer comprises a first dimethicone backbone including at least one pendant group comprising oxyalkylene (in particular oxypropylene or oxyethylene group), cross-linked with a second dimethicone backbone including at least one pendant group comprising oxyalkylene (in particular oxypropylene or oxyethylene group), wherein the cross-linking group comprises an oxyalkylene group (in particular oxypropylene or oxyethylene group). The repeating oxyalkylene group may be linked to the, or each dimethicone backbone through a substituted or unsubstituted hydrocarbyl group, in particular a substituted or unsubstituted alkyl group (generally $C_{1-4}$ alkyl group).

Generally, the pendant group(s) is an oxyethylene group, typically including 5 to 50 repeat groups, generally 10 to 15 repeat groups.

Typically, the cross-linking group is an oxypropylene group, typically including 5 to 50 repeat groups, generally 15 to 30 repeat groups.

PEG-12 dimethicone PPG 20 crosspolymer is an example of a suitable silicone polyether for use as the dimethicone macromer, with a polypropylene glycol crosslink and pendant polyethylene glycol.

PEG-12 dimethicone/PPG-20 crosspolymer

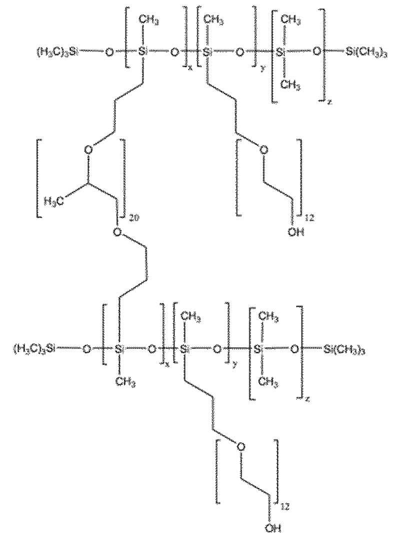

A further example of a suitable cross-linked silicone polyether is provided below:

PEG-12 dimethicone/bis-isobutyl PPG-20 crosspolymer

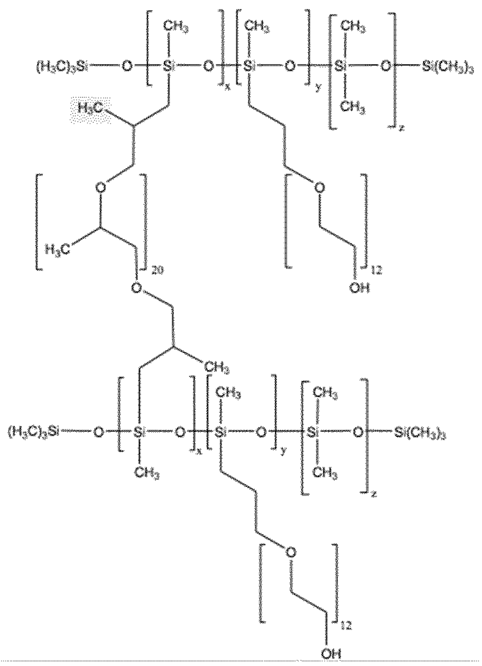

These PPG crosslinked PEG-12 pendant dimethicone crosspolymers are particularly useful in stabilizing propylene glycol in silicone oil non-aqueous emulsions containing dissolved $C_{12}$-$C_{14}$ acid and $C_{12}$ alcohol functional excipients.

Alternatively, or additionally, the dimethicone macromer may comprise a dimethicone backbone with an ionic pendant chain of the general structure as shown below where X may be a hydrophilic amine, quaternary amino or acid functional grouping.

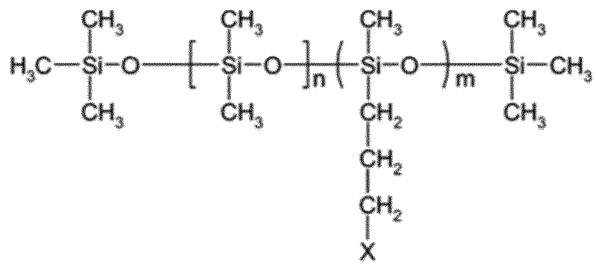

Especially preferred is the pyrrolidone carboxylic acid functionalized dimethicone macromer with INCI name PCA dimethicone, This ionic dimethicone macromer, especially in combination with PPG crosslinked PEG-12 pendant dimethicone crosspolymers is particularly useful in stabilizing propylene glycol in silicone oil non-aqueous emulsions containing dissolved $C_{12}$-$C_{14}$ acid and $C_{12}$ alcohol functional excipients.

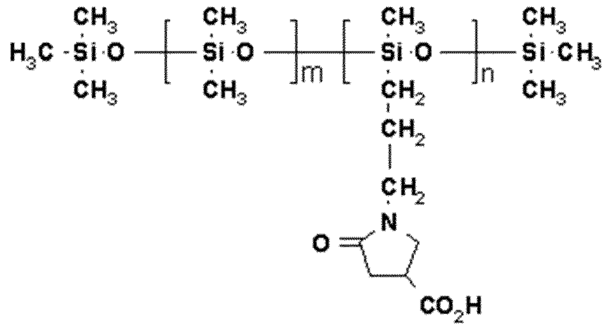

According to one embodiment the composition of the present invention comprises more than one dimethicone macromer, generally more than one polydialkylsiloxane diol compound or ionic dimethicone macromer surfactant.

Generally, the dimethicone macromer(s) is dispersed, dissolved or suspended in the hydrocarbyl methyl siloxane compound(s) or vice versa.

In particular, the, or one of, the dimethicone macromers may be dispersed, dissolved or suspended in an alkyl methyl siloxane such as caprylyl methicone, lauryl methicone, stearyl methicone or caprylyl trimethicone.

Alternatively, or additionally, the, or one of the dimethicone macromers may be dispersed, dissolved or suspended in a methyl siloxane compound, such as a cyclomethicone such as decamethylcyclopentasiloxane.

According to one embodiment, the composition of the present invention comprises more than one siloxane-containing compound, in particular one or more alkyl-, aryl- and/or alkyl arylmethyl siloxane compound(s) (generally having a number average molecular weight of less than 1000) and one or more dimethicone macromer(s) (typically having a number average molecular weight of more than 1000, suitably more than 2000), generally more than one polyalkylsiloxane diol compounds.

Generally, the composition comprises a mixture of one or more alkyl and/or alkyl aryl methyl siloxane compound(s) (generally having a number average molecular weight of less than 1000) and one or more dimethicone macromer(s) having a number average molecular weight of more than 1000, wherein the mixture comprises 50 to 95 %w/w alkyl and/or alkyl aryl methyl siloxane compound(s) having a number average molecular weight of less than 1000, and 5 to 50%w/w dimethicone macromer surfactant.

According to one embodiment, there is provided a composition comprising a hydrocarbyl methyl siloxane compound, an alkyl siloxane compound (generally a methyl siloxane compound, in particular a cyclomethicone compound), and two polyalkylsiloxane macromers.

The composition of the present invention may comprise one or more alkyl-, aryl- or alkyl arylmethyl siloxane compounds, typically selected from the group consisting of caprylyl methicone, lauryl methicone, stearyl methicone, caprylyl trimethicone and decamethylcyclopentasiloxane; and one or more dimethicone macromers, generally comprising one or more polyalkylsiloxane portions (generally one or more dimethylsiloxane portions) and one or more oxypropylene or oxyethylene portions. Typically, the dimethicone macromer comprises one or more copolymers of ethylene oxide and propylene oxide.

According to one embodiment, the composition of the present invention comprises an alkyl methyl siloxane and/or an aryl alkyl methyl siloxane having a number average molecular weight of 800 or less and a cross-linked dimethicone macromer having a number average molecular weight of more than 1000, said dimethicone macromer comprising one or more copolymers of ethylene oxide and propylene oxide.

Generally, the viscosity of the mixture of hydrocarbyl methyl siloxane compound and dimethicone macromer is greater than 200,000 cSt, typically 250,000, to 1,000,000 cSt.

The first dimethicone macromer mixture may comprise a mixture of caprylyl methicone and a polyethylene glycol dimethicone/polypropylene glycol cross-linked polymer. Suitable compositions are available from Dow Corning ® under the INCI name caprylyl methicone PEG-12 dimethicone/PPG-20 cross polymer (EL-7040 hydro elastomer blend).

The composition of the present invention may comprise a mixture of an alkyl methyl siloxane (generally having a number average molecular weight of less than 1000, typically less than 400) and a polyalkylsiloxane diol compound.

*Second Dimethicone Macromer Mixture*

The composition of the present invention comprises a second dimethicone macromer mixture including a methyl siloxane compound and a cross-linked dimethicone macromer.

The composition of the present invention may comprise a mixture of an alkyl siloxane compound generally having a number average molecular weight of less than 1000 (typically a cyclomethicone compound, in particular an alkyl cyclomethicone compound) and a dimethicone macromer having a number average molecular weight of more than 1000, typically a cross-linked polyalkylsiloxane diol. Suitable compositions are available from Dow Corning ® under the trade name ST Elastomer 10.

*First and Second Dimethicone Macromer Mixtures*

According to one embodiment, the composition of the present invention may comprise a first dimethicone macromer mixture including a hydrocarbyl methyl siloxane compound (generally an alkyl or alkyl aryl silicone) and a polyglycol dimethicone macromer, typically a cross-linked polyalkylsiloxane diol compound; and a second dimethicone macromer mixture including a methyl siloxane compound (in particular a methyl cyclomethicone compound) and a dimethicone macromer, typically a cross-linked polyalkylsiloxane diol compound.

According to one embodiment, the composition of the present invention comprises 50%w/w or less hydrocarbyl methyl siloxane, typically 10 to 40%w/w hydrocarbyl methyl siloxane, suitably 20 to 30%w/w hydrocarbyl methyl siloxane.

According to one embodiment, the composition comprises less than 40%w/w of any alkyl siloxane-containing compound, generally 20%w/w or less, suitably 20%w/w or less.

The composition of the present invention may comprise 30%w/w or less dimethicone macromer, in particular cross-linked polyalkylsiloxane diol compound.

According to one embodiment, the composition of the present invention comprises 30%w/w or less hydrocarbyl methyl siloxane having a number average molecular weight of 1000 or less, generally one or more alkyl or alkyl aryl methyl siloxane compound; typically 20%w/w or less; suitably 10%w/w or less.

According to one embodiment, the composition of the present invention comprises 30%w/w or less dimethicone macromer surfactant having a number average molecular weight of more than 1000, generally more than 2000; typically 20%w/w or less; suitably 10%w/w or less.

According to one embodiment, the second dimethicone macromer mixture includes a methyl siloxane compound having a number average molecular weight of less than 1000 and a cross-linked polyalkylsiloxane diol dimethicone macromer having a number average molecular weight of more than 1000, generally of more than 2000.

In particular, the second dimethicone macromer mixture may include 5 to 30%w/w cross-linked dimethicone macromer; generally, 10 to 20%w/w; typically, 12 to 19%w/w.

The composition may comprise 5 to 45%w/w second dimethicone macromer mixture, typically 10 to 40%w/w, generally 20 to 30%w/w.

Generally, the first and second dimethicone macromers are mixed in a ratio of from 3:1 to 0.6:1, ideally 1.5:1.

The first cross-linked polyalkylsiloxane diol compound may be in the form of a mixture comprising an alkyl or alkyl aryl methyl siloxane and a dimethicone macromer, the second cross-linked polyalkylsiloxane diol may be in the form of a mixture comprising an alkyl siloxane compound, such as an alkyl cyclomethicone compound and a dimethicone macromer.

According to one embodiment, the first cross-linked polyalkylsiloxane diol compound may be a PEG dimethicone/ PPG crosspolymer such as that sold under the trade name Dow Corning EL-7040 Hydro Elastomer Blend comprising 17.5-19.50% of PEG-12 dimethicone/ PPG-20 crosspolymer.

Typically, the second dimethicone macromer mixture comprises a dimethicone cross-linked polymer swelled in a silicone fluid such as, for instance, cyclopentasiloxane such dimethicone macromer mixtures tend to impart a dry smoothness and a non-greasy feel to the skin. In thickening the continuous phase of water-in-silicone or polyol in silicone emulsions they improve physical stability and help reduce creaming and phase separation. The second cross-linked polyalkylsiloxane diol compound may be in the form of a mixture of cylcopentacyloxane and dimethicone cross polymer such as that sold under the trade name Dow Corning ST Elastomer 10 comprises 12.5% high molecular weight silicone

elastomer in decamethylcyclopentasiloxane.

**Ancillary user adherence-improving skin barrier restoring system comprising nicotinamide and polyhydroxy acid**

*Niacinamide and the Skin, Cosmetic uses.*

The use of Nicotinamide (NAM) in cosmetic and medicinal dermatology, via topical or oral administration, extends back almost 50 years and has been well documented in several major reviews.

Mostly, NAM is used topically and for cosmetic purposes. NAM, in vitro in cultured human keratinocytes at concentrations as low as 1-10 μM (75%-100% of maximum response, 1-10 μmol L-1; (μM)), and in vivo topically in man, very significantly increases the biosynthesis of ceramides and other stratum corneum lipids by up to 5-fold to increase skin barrier function. Figure 4a herein, from Tanno O et al. (British Journal of Dermatology 2000; 143: 524-531) shows the dose response of ceramide synthesis to NAM added to human keratinocytes in vitro.

Consistent with this, Soma Y et al. (Int J Dermatol. 2005 Mar;44(3): 197-202) found that 2% NAM cosmetic cream was a more effective moisturiser than standard petrolatum in a group of 28 patients with atopic dermatitis. Similarly, Draelos ZD et al. (J Cosmet Laser Ther. 2006 Jun;8(2):96-101) reported that a 2% NAM-containing facial moisturizer improved skin barrier in subjects with rosacea in a non-treatment-controlled study. Also, Kawada A et al. (J Dermatol. 2008 Oct;35(10):637-42) demonstrated facial anti-wrinkle effects of a cosmetic containing 4% NAM compared with a placebo control. Finally, in their extensive review, Wohlrab and Kreft (Skin Pharmacol Physiol. 2014;27(6):311-5) conclude that the antipruritic effects of NAM are mainly based on barrier-protective/restorative effects.

*Polyhydroxy acids and the skin, Cosmetic uses*

Polyhydroxy acids, (PHAs) as typified by lactobionic acid and gluconolactone, are very effective skin moisturisers and antiaging compounds (Grimes et al., Cutis. 2004 Feb;73(2 Suppl):3-13) and achieve these effects without significant skin irritation (Tasic-Kostov et al., J Cosmet Dermatol. 2019;18(6): 1705-1710).

The fundamental mechanism is one of acidification deep into the stratum corneum (SC), for example, to inhibit serine protease activity. Hachem JP et al. (Acute acidification of stratum corneum membrane domains using polyhydroxyl acids improves lipid processing and inhibits degradation of corneodesmosomes. J Invest Dermatol. 2010 Feb; 130(2):500-10) applied 10% PHA (lactobionic acid) to murine flank skin and was able to decrease pH at all SC depths by 0.5-1.0 pH units. In GB 2562270A we were able to demonstrate that 5% PHA (lactobionic acid) in a propylene glycol/myrystyl alcohol ($C_{14}$ alcohol) co-enhancer non-silicone cream was able to decrease human SC pH in vivo by up to 1.5 pH units. We conclude that 5% PHA is optimum for inhibition of serine protease activity, which occurs in the pH range 3-4 in human stratum corneum.

Increased stratum corneum serine protease activity leads to *degradation* of critical lipid processing enzymes, thus inhibition of synthesis of essential stratum corneum lipids and also to reduced stratum corneum integrity/cohesion due to effects on corneodesmosome linking between cells.

These effects are entirely complementary with those of NAM, which works to *increase* ceramide/essential lipid synthesis. Nature teaches us that positive and negative feedback loops are needed to keep any system in control and that, in crisis; for example, skin flare, the normal state may be restored by increasing positive and decreasing negative feedback loops.

*Therapeutic Potential of (High Dose Delivery) Topical Niacinamide (NAM) in Dermatology*

In early 2017, Limeway Pharma Design (LPD) undertook a literature search on the dermatological therapeutic potential of NAM incorporated into LPDs platform glycol-in-silicone cream co-enhancer technology. The string (niacinamide OR nicotinamide) AND (skin OR dermatology OR whitening OR melasma OR ultraviolet OR actinic OR melanoma OR eczema OR psoriasis) was input into PubMed and gave 1,787 hits of which 101 were considered relevant and were then input into the database "nicotinamide".

*Biological effects of (High Dose, 7.5-10%) Topical Niacinamide (NAM) in Dermatology*

NAM is the sole substrate for the DNA repair enzyme poly (ADP) ribose polymerase (PARP-1) and at a concentration of 50μM inhibits PARP-1 by 66% (Park J, Photochem Photobiol. 2010; 86(4):942-948). Figure 4b, herein, from Park J, 2010, shows the dose response of PARP-1 inhibition to NAM in vitro. Concentrations of NAM of from 50-100 μM are predicted to be required for significant PARP-1 inhibition, thus from at least 5-10 times the concentration range of 1-10 μmol L-1 (μM) associated with increase in the biosynthesis of ceramides and other stratum corneum lipids to increase skin barrier function and restore healthy skin.

NAM inhibition of PARP-1 controls NFκB-mediated transcription and is therefore important for the expression of adhesion molecules and proinflammatory mediators such as IL-12, TNF-o, IL-1 and nitric oxide (Wohlrab and Kreft, Skin Pharmacol Physiol. 2014;27(6): 311-5). Ting L et al. (Signal Transduct Target Therap. 2017; 2:17023) in their review of signaling in inflammation, list IL-1, IL-2, 1L-6, 1L-8, 1L-12 and TNF alpha as being important NFκB-controlled proinflammatory cytokines. Ungerstedt JS. et al. (Clin Exp Immunol. 2003;131(1):48-52) report NAM to be a potent inhibitor of proinflammatory cytokines including IL-1 beta, IL-6, IL-8 and TNF alpha although in their endotoxin-induced model concentrations of NAM of 2,000 μM NAM were required for significant effects.

Hwang and Song (Cell Mol Life Sci. 2017;74(18):3347-3362) report the $IC_{50}$ concentration for NAM inhibition of SIRT1 to be in the range of 50-180 μM. SIRT1 is a member of the sirtuin family, inhibition of which leads to the inhibition of oxidative stress signaling pathways MAPK, NF-κB, and STAT3, down-regulation of inflammatory factors, suppression of inflammation and keratinocyte hyperproliferation, and inhibition of angiogenesis.

Hakosaki et al. (Br J Dermatol. 2002; 147(1): 20-3; Exp Dermatol. 2005; 14(7): 498-508) reported the effect of NAM on reducing cutaneous pigmentation and suppression of melanosome transfer from melanocytes to keratinocytes in vitro in co-cultures. 1.0mmol$L^{-1}$ (1mM) NAM inhibited inhibition of melanosome transfer by 35-68% in the coculture model (Hakosaki et al. Br J Dermatol. 2002). Hakosaki et al. (Exp Dermatol. 2005), using a similar co-culture model, found that NAM at 10μM inhibited melanosome transfer from the melanocytes to the keratinocytes but by only 14% after 3 days of treatment. There is a reasonable expectation that increasing NAM skin concentrations to >50μM would result in enhanced efficacy to reduce skin pigmentation.

Thus, NAM might be expected to have different pharmacological/therapeutic effects upon the skin depending upon the dose delivered to the skin.

As has been described by Tanno et al. (British Journal of Dermatology. 2000; 143: 524-531) NAM in vitro and at concentrations as low as 1-10 μM (75%-100% of maximum response, 1-10 μmol L-1; (μM)) very significantly increases the biosynthesis of ceramides and other stratum corneum lipids by up to 5-fold to increase skin barrier function. Consistent with this, Draelos ZD et al. (J Cosmet Laser Ther. 2006 Jun;8(2):96-101) reported that a 2% NAM-containing facial moisturizer significantly improved skin barrier in subjects with rosacea in a non-treatment-controlled study.

However, at higher doses of NAM delivered, PARP-1 and SIRT1 pathways and melanosome transfer are inhibited leading to the general down-regulation of related biology and thus with potential for previously undescribed pharmacological potential. This dose-dependency of elicited pharmacology is not a new idea; Paracelus (1493-1541) 'all things are poison and not without poison; only the dose makes a thing not a poison".

As a real-world example, aspirin (acetyl salicylic acid) is used at oral doses of <100mg /day in the prevention of heart attack and stroke. In temporary relief of minor pain due to headache, arthritis, muscle pain, or toothache daily doses of 1,200 to 2,600mg of aspirin are used. Many chemicals have different potential therapeutic activity, thus are different drugs, depending upon the dose delivered.

Because pharmacokinetic modelling predicted that NAM free drug skin concentrations of 50μM could be achievable following topical application of 7.5%-10% NAM in a glycol co-enhancer-in silicone cream (Zhang Y et al. Pharmaceutics. 2019;11:668-681) we further reviewed the topical dermatological therapeutic potential of high dose NAM.

*Therapeutic Potential of High Dose Topical Niacinamide in Dermatology; Indications*

*Topical Nicotinamide (NAM) cream for use in UV-induced skin cancer*

Of particular scientific interest are publications from the research group led by Professor Diona Damian, Department of Dermatology, at the University of Sydney, NSW on the potential of oral and topical NAM to be chemoprotective for skin cancers.

*Chemoprevention of skin cancers using NAM*

In 2008 Damian et al. (J Invest Dermatol. 2008;128(2):447-54) showed that topical 5% NAM prevented UV radiation-induced immune suppression in healthy volunteers and proposed that NAM be added to sunscreens and after-sun lotions to improve protection from immunosuppression and skin cancers.

Moloney-Damian et al. (Br J Dermatol 2010;162(5): 1138-9) conducted a vehicle-controlled study on the use of 1% NAM gel to prevent actinic keratoses (AK) in AK-prone patients. AKs were significantly reduced at 3 months but not at 6 months and the authors conclude that a higher NAM concentration or use of a different (more efficient NAM delivery) topical vehicle may have led to an increase in efficacy. Consistent with this conclusion, Surjana-Damian et al. reported that high oral dose NAM significantly reduced actinic keratoses in a phase II double-blinded randomized placebo-controlled trial. (J. Invest. Dermatol. 2012;132: 1497-1500).

In 2015 Chen-Damian et al. published "A Phase 3 Randomised Trial of Nicotinamide for Skin-Cancer Chemoprevention" (ONTRAC) (N. Engl J Med 2015; 373(17): 161-26), reporting that oral NAM was safe and effective in

reducing the rates of new non-melanoma human skin cancers (basal cell (BCC) and squamous cell (SCC) cancers) and actinic keratoses (AK).

In a further analysis of ONTRAC tissue specimens, Malesu A et al. (Photochem Photobiol Sci Feb 2020; 19(2): 171-179), reported on melanomas arising in both the NAM and placebo group. Peritumoral and tumour infiltrating CD4+ and CD8+ lymphocytes were statistically significantly increased in melanomas arising on NAM treatment compared to those arising on placebo. The authors conclude that, due to the chemopreventive activity of NAM against keratinocyte cancers, its DNA repair enhancing effects in melanocytes and now its potential enhancement of tumour-infiltrating lymphocytes, clinical trials on use of nicotinamide for melanoma chemoprevention are indicated. It can be concluded from these studies that nicotinamide has the potential to help prevent AK, BCC, SCC and melanoma, particularly when used with broad spectrum sunscreens.

Importantly, Malesu et al. (Photochem Photobiol Sci Feb 2020;19(2):171-179) confirm our 2017 pharmacokinetic analysis that free drug plasma NAM (and thus at steady state also in viable skin tissues) of approximately 50-80μM were achieved in the ONTRAC oral dosing study. These predictions support a PARP-1 / SIRT1 mechanism of action for NAM in prevention of skin cancers.

A further Canadian pilot clinical trial (NCT03769285); "Nicotinamide Chemoprevention for Keratinocyte Carcinoma in Solid Organ Transplant Recipients: A Pilot, Placebo-controlled, Randomized Trial" was due to complete December 2020.

*Treatment of skin cancers using high dose NAM*

Drago et al. (Dept. Dermatology, Genoa, Italy, Eur J Dermatol 2017; 27(4): 382-385) studied the efficacy of high dose oral nicotinamide as treatment for AKs in transplant recipients. After six months nicotinamide treatment, AKs had significantly decreased in size in 18/19 patients (88%). Among these 18 patients, seven patients (42%) had shown complete clinical regression and no patient developed new AKs. Conversely, among the controls, 91% showed an increase in AK size and/or developed new AKs. Seven pre-existing AKs progressed to squamous-cell carcinoma. Dragos et al. concluded that nicotinamide appears to be effective in both preventing and treating AKs.

A recent review "The Role of Nicotinamide in Cancer Chemoprevention and Therapy" (Biomolecules. Nikas, IP et al. 2020 March;10(3):477-497) focuses on skin cancers, including all the studies on chemoprotection described above. The authors conclude that in contrast to its role in chemoprevention, there is little evidence concerning the clinical efficacy of NAM as a chemotherapeutic, treatment, regimen. Only a single Phase 1 clinical trial on lymphoma is cited. However, NAM inhibits SIRT1 and PARP1 and oncogenic KRAS/AKT pathways in skin. Because of the expected consequental effects on vasculogenic mimicry, metastasis, invasion and proliferation, Nikas, IP et al. included treatment of existing melanoma with NAM in future directions deserving of clinical investigation. Chen A (Chin J Cancer. 2011 Jul;30(7):463-471) reports on the clinical development of PARP-1 inhibitors to treat existing melanoma.

Because of the link between the presence of AK and potential for further development into NMSC, especially BCC, and the effect of NAM to treat existing AK (Drago et al. (Dept Dermatology, Genoa, Italy, Eur J Dermatol 2017;27(4):382-385) it is tempting to speculate that NAM may be effective to treat existing BCC and SCC. Nikas, IP et al. caution that SIRT1 inhibition by NAM could suppress metabolism, a deregulation of which is potentially oncogenic, while suppression of PARP1 could result in accumulated genetic damage in the long term. This speculation thus seems not only ill-advised but potentially dangerous; at least currently.

Clinical data on use of NAM in treatment of NMSC does not currently exists and a search of the US database ClinicalTrials.gov indicates that no such studies to provide this evidence are currently planned. We believe that there is currently insufficient data to conclude that NAM will be effective to treat existing BCC and SCC.

*Topical Nicotinamide (NAM) cream for use in treatment of proliferative/inflammatory skin diseases*

*Topical Nicotinamide (NAM) cream for use in treatment of psoriasis*

The influential review of Namazi MR (Nicotinamide: a potential addition to the anti-psoriatic weaponry. FASEB J. 2003;17(11):1377-9) first describes the (oral) anti-psoriatic potential of nicotinamide as an inhibitor of poly (ADP-ribose) polymerase-1 (PARP-1) that, through enhancement of nuclear kappa B-mediated transcription, plays a pivotal role in the expression of inflammatory cytokines, chemokines, adhesion molecules, and inflammatory mediators. Understandably there is no discussion on the skin concentrations of NAM needed to inhibit PARP-1. We conclude that product concentration of 7.5-10.00% w/w in functional topical co-enhancer delivery systems are needed to significantly inhibit PARP-1 (>50μM), respectively, thus for use in topical treatment of psoriasis.

Levine D et al. (J Am Acad Dermatol. 2010;63(5):775-81) conducted a 12-week study in patients with psoriasis and were unable to show any significant difference between topical calcipotriol 0.05% and calcipotriol 0.05% with 1.4% NAM. Siadat, AH et al. (Adv Biomed R. 2013;2:90) conducted a 12-week study in patients with mild-moderate psoriasis

on topical calcipotriol 0.005% or calcipotriol 0.005% with 4.0% NAM. At the end of 12 weeks, PASI score was numerically more reduced with calcipotriol plus NAM compared to calcipotriol alone (83.6 ± 7.9% vs. 77.8 ± 9.7%).

Topical Calcipitriol plus Corticosteroid creams are a mainstay of psoriasis treatment and speak to the fidelity of this antiproliferative + anti (NF-κB-related) inflammatory therapeutic concept. A calcipotriol plus high dose functional topical co-enhancer delivery 7.5-10.0% NAM cream, thus without corticosteroid, should advance this therapeutic concept.

*Topical Nicotinamide (NAM) cream for use in treatment of dermatitis, inflammatory skin conditions*

Various reviews attest to the therapeutic expectation of topical NAM to be effective to treat inflammatory skin diseases. Oral high dose of 2g/day (~100μM skin target concentration) NAM (with -cycline antibiotics) (Kolbach DN. et al. BJD. 1995;13(1): 88-90) was effective in achieving total remission of the potentially life-threatening blistering skin condition bullous pemphigoid within 6-8 weeks.

However, we hypothesis that the relative ineffectiveness of topical NAM in inflammatory skin disease is due to failure to delivery sufficient NAM concentrations to the target site in the skin. For example, the study of Grange et al. "Nicotinamide inhibits Propionibacterium acnes-induced IL-8 production in keratinocytes through the NF-kB and MAPK pathways" (Grange et al. Journal of Dermatological Science. 2009;56:106-112) reports that 5.0ug/ml (~40μM) of NAM is required to significantly inhibit P. acnes-induced IL-8 mRNA production in keratinocytes. We conclude that product concentrations of 7.5-10.00% w/w of NAM in functional topical co-enhancer delivery systems are needed to achieve significantly anti-inflammatory effects in the treatment of inflammatory skin conditions such as acne, psoriasis, eczema, rosacea and blistering skin conditions such as bullous pemphigoid.

*Topical Nicotinamide (NAM) cream for use in treatment of radiation dermatitis*

Bowstrom A. et al. (Radiotherapy and Oncology. 2001; 59:257-265) reported that mometasone furoate, topical corticosteroid cream, was significantly more effective than emollient cream alone in reducing acute radiation dermatitis in breast cancer patient. A key component of the mode of action was postulated to be inhibition of IL-6. Yanez et al. *(2019;9.-102-19* https://doi.org/10.1038/s41598-019-46678-8) used an LPS-induced inflammatory macrophage model in vitro to produced high levels of TNF-o, IL-6, and VEGF. NAM, at 500μm, the only dose studied, produced an 88% reduction in IL-6 compared to untreated control. We conclude that product concentrations of 7.5-10.00% w/w NAM in functional topical co-enhancer delivery systems are needed to achieve significantly anti-inflammatory effects in the treatment of dermatitis, including radiation-induced dermatitis.

*Topical Nicotinamide (NAM) cream for use in treatment of pigmentation*

Hakosaki et al. (Exp Dermatol. 2005; 14(7): 498-508) reported that NAM at 10μM inhibited melanosome transfer from the melanocytes to the keratinocytes but by only 14% after 3 days of treatment. A clinical study was also conducted on 79 women with symmetrical pigmentation. In a split-face design, Group 1 (n=1/4 39) utilized 5% NAM in a moisturizer to one side of the face vs. the vehicle moisturizer (without NAM) to the other side of the face and Group 2 (n=1/4 40) utilized 2% NAM moisturizer vs. the vehicle moisturizer. The authors reported a dose-dependent reduction in hyperpigmented lesions. However, at 8 weeks the visual difference between the 5% NAM and its split-face control, although statistically significant, was only 6%. We conclude that product concentrations of 7.5-10.00% w/w NAM in functional topical co-enhancer delivery systems are needed to achieve significantly anti-pigmentation effects.

*Permeation of NAM from topical co-enhancer delivery systems across human skin in vitro*

**In vitro human skin permeation to steady state and prediction of free NAM epidermal concentrations in vivo: Barrier Repair.**

The cream composition described in Table 9 example #46 containing 5.0% NAM and 5% PHA at a product pH ~4.60 (equilibrium pH ~3.86) was subject to a 24-hour in vitro human skin permeation study. Receptor fluid samples were assayed for NAM at 0, 2, 4, 8, 10 and 24 hours. Figure 5, herein, shows data on the steady state flux achieved (n=6) from 8-24 hours following the lag time. A mean steady state flux value, J, (ug/cm$^2$/hour) for input rate into the skin of 2.28 ug/cm$^2$/hour was calculated. From this, an estimation of the NAM free drug concentration achieved at the basal epidermal site was made. In this study, steady state flux, and thus by definition, steady state skin tissue concentrations of NAM were achieved and sustained over at least 16 hours. Following oral dosage of NAM, peak concentrations in both plasma and skin are anticipated to decrease with a half-life of approximately 90 minutes (Bongiovanni T et al. Review Proc. Med. Def. Bioscience. 1993;1: Conference paper).

*Estimation of basal cell target site tissue concentrations from in vitro skin permeation data*

C*, the concentration of free NAM at the basal epidermal target site was calculated from equation (1).

$$C^* = J/P_D \quad (1)$$

The permeability coefficient of NAM in the dermis was estimated using equation (2) where $D_D$, the dermal diffusion coefficient for free NAM was estimated from equation (2).

$$D_{DNAM} = -4.15 - (0.655 \times \log MW_{NAM}) \quad (2)$$

Inputting the molecular weight of NAM of 122.12 into equation (2) gives a value for $D_{DNAM}$ of $3.04 \times 10^{-6}$ $cm^2$ $s^{-1}$. A value of 100 μm (0.01cm) was used for $h_D$, the thickness of the unperfused upper dermis (17) and $P_D$ calculated to be approximately 1.08 cm $h^{-1}$. For clarity of communication $(3.04 \times 10^{-6} \times 60 \times 60)/0.01 = 1.08$ cm $h^{-1}$.

Finally, from equation (1):

C*, the concentration of free NAM at the basal epidermal target site is = $J/P_D$ = 2.28/1.08 = 2.11ug/$cm^3$, which at a molecular weight of NAM of 122.12 is ~17.50uM concentration. As we have described earlier, Tanno O et al. (British Journal of Dermatology 2000; 143: 524±531) show the in vitro dose response of ceramide synthesis is at a maximum at ~10uM nicotinamide. Creams, for example Table #10, Examples #49 and #50 containing 2.5% nicotinamide and 5.0% PHA and using lactic acid non-volatile and liquid ammonia volatile buffers to control in-product and on-skin pH are predicted to achieve free NAM at the basal epidermal target site of ~9uM. Examples #49 and #50 are predicted to have maximal NAM and PHA coordinated skin barrier repair potential.

**In vitro human skin permeation to steady state and prediction of free NAM epidermal concentrations in vivo: PARP-1/ SIRT1 NF-kB mediated therapeutic potential.**

The cream composition described in Table 9 example #44 and two variants both containing 10.0% NAM with an equilibrium pH ~3.80-4.00 were subject to a 24-hour in vitro human skin permeation studies using abdominal skin from three difference donors. Receptor fluid samples were assayed for NAM at 0, 2, 4, 8, 10 and 24 hours. A mean steady state flux (n=18) J, (ug/$cm^2$/hour) for input rate into the skin of 6.64 ug/$cm^2$/hour was determined. From this, the mean NAM free drug concentration achieved at the basal epidermal site were estimated using equation (1) to be 50.40uM. The cream composition described in Table 9 example #47 using a non-volatile pH adjuster to achieve an equilibrium pH ~5.10, at which pH NAM is almost totally unionised, is predicted to achieve an epidermal skin concentration of NAM of ~70.00uM.

*In vitro human skin permeation from glycol in silicone creams*

As we hypothesised in GB 2549418B, the inclusion of a silicone emollient such as caprylyl methicone, into a functional glycol-co-enhancer system to form a cream does not adversely affect co-enhancer function. For example, sponsored studies on a glycol-co-enhancer cream containing 0.1% retinoic acid metabolism blocking agent achieved peak skin concentration of 10-fold greater than conventional creams formulated by a specialist dermatology formulation group.

Ng SP. et al (J Cosmet Dermatol. 2020;19(10):2656-2662) compared the skin concentrations of the plasmin inhibitor tranexamic acid for the treatment of melasma from a commercial 2% cream and a 2% glycol-$C_{14}$ alcohol-co-enhancer cream also containing 5% PHA and 4% NAM. Skin concentrations of tranexamic following application of the co-enhancer cream were robustly within the concentration range estimated to be required for efficacy at both 6 and 24 hours, whereas those from the commercial cream were within the lower range at 24 hours only and approximately 10-fold reduced.

*2% plasmin inhibitor glycol-$C_{14}$ alcohol-co-enhancer cream + (5% PHA 4% NAM): Effect on Melasma (MASI) and Consumer Experience*

An unpublished dermatologist-investigator-controlled study on 22 subjects, aged between 42-60 years was conducted over 12 weeks using the composition studied by Ng SP. et al. applied to facial skin in conjunction with a

sunscreen.

After 12 weeks (84 days) MASI scores (Melasma Area and Severity Index) were assessed by the dermatologist clinical investigator, as shown below. All 22 subjects completed the study.

| | Day 0 | Day 84 | Δ (0-84) | Statistical analysis | |
|---|---|---|---|---|---|
| | Mean +/-SEM | Mean +/-SEM | Mean +/-SEM | p | Significance |
| MASI Score | 8.6 +/- 0.8 | 5.4 +/- 0.8 | -3.2 +/- 0.4 | <0.001 | Y |

The 2018 review of Bala et al. (Dermatol Surg. 2018;44(6):814-825) focusing on the use of oral tranexamic acid in the treatment of melasma, concludes that it is a safe and efficacious treatment for refractory melasma. This conclusion is strongly supported by two very recent double-blind placebo-controlled studies on oral tranexamic acid in the treatment of melasma ) in which an approximately 50% reduction in melasma (mMASI, MASI) scores, significantly different from placebo, were seen over 12 weeks following bid dosing of 250mg per day. In contrast, the clinical results obtained from topical administration are currently disappointing and our finding of efficacy at 12 weeks following topical application of tranexamic acid glycol-$C_{14}$ alcohol-co-enhancer cream is reassuring to support this topical therapeutic concept.

In addition, subjects were asked to complete a questionnaire (Figure 6) which explored their experience in use of the cream; cosmetic properties, easy of application, efficacy and tolerance were all rated very highly. All subjects (100%) answered yes to two rating:

- Would you like to buy the product again (regardless of cost)?

- Would you recommend this product to a friend?

These scores, especially, strongly suggest that the subjects have "a reason to believe in the product". This, even though the equilibrium pH of the cream is 5.00, such that pH is near optimum for NAM but PHAs will be at around 90% ionised, thus much less skin permeable.

Creams with equilibrium pH values in the range 3.0-4.0, such as examples #45, #49 and #50 provide optimum ionisation states for skin permeation of both NAM and PHAs, thus to drive adherence to treatment.

**Active agents**

Suitably, an active agent for inclusion in a composition described herein is selected from retinoids, retinoic acid metabolic blocking agents (RAMBAs), cannabinoids including tetrahydrocannabinol and cannabidiol, alpha and beta hydroxy acids and polymers, lactobionic acid, gluconolactone, immune response modifier compounds, tranexamic acid, calcidiol, calcitriol, calcipotriene, paricalcitol, 22-oxacolcitriol, dihydrotachysterol, calciferol, calcipotriol (aka calcipotriene), nicotinamide, corticosteroids, anti-rosacea agents, antihistamines, antibacterial agents, antiacne agents, antifungal agents, antiviral agents, cytotoxic agents for use in actinic keratoses, basal cell and squamous cell cancers and melanoma, psoralens, anti-alopecia agents, anti-androgens, anti-pruritic agents, keratolytic agents, skin lightening and depigmenting agents, dithranol, antiseptics, anaesthetics, analgesics, neuropathics, non-steroidal anti-inflammatory agents, vasoactive agents and agents to combat dry and ageing skin. In one embodiment, the composition may comprise more than one primary pharmaceutically active agent, or salt. Suitable concentration ranges for the active agent ranges from about 0.001% to about 10% by weight of the composition depending on the nature of the active agent or combination of active agents.

Preferred active agents for inclusion in the composition described herein are selected from retinoids, tranexamic acid, calcidiol, calcitriol, calcipotriene, paricalcitol, 22-oxacolcitriol, dihydrotachysterol, calciferol, calcipotriol (aka calcipotriene), nicotinamide, corticosteroids, immunomodulators, antiacne agents, antifungal agents, antiviral agents, cytotoxic agents for use in actinic keratoses, basal cell and squamous cell cancers and melanoma, anti-pruritic agents, keratolytic agents, skin lightening and depigmenting agents, analgesics, non-steroidal anti-inflammatory agents, cannabinoids including tetrahydrocannabinol and cannabidiol and agents to combat dry and ageing skin.

In one embodiment, the pharmaceutically active agent is a retinoid. Examples of suitable retinoids include, but are not limited to, tazarotene, tretinoin, isotretinoin, acitretin, etretinate, adapalene, bexarotene, alitretinoin, retinol, retinal, retinyl esters including retinyl palmitate, retinyl acetate, retinyl propionate, retinyl linoleate, ethyl 5-(2-(4,4-dimethylthiochroman-6-yl)ethynyl)thiophene-2-carboxylate, 6-(2-(4,4-dimethylthiochroman-6-yl)-ethynyl)-3-pyridylmethanol and 6-(2-(4,4-dimethylthiochroman-6-yl)-ethynyl) pyridine-3-carbaldehyde, salts thereof, and mixtures thereof. In one embodiment, the retinoid is tazarotene. In an alternative embodiment, the retinoid is tretinoin. In an alternative embodiment, the retinoid is retinol. In another embodiment, the composition comprises a retinoid in combination with a second pharmaceutically active agent. In one embodiment the combination is tazarotene and a second pharmaceutically active agent. In another embodiment the combination is tretinoin and a second pharmaceutically active agent.

Suitably, one combination of the retinoid is with a corticosteroid, such as clobetasol propionate; or in combination withone of calcidiol, calcitriol, calcipotriene, paricalcitol, 22-oxacolcitriol, dihydrotachysterol, calciferol, or calcipotriol; or in combination with an antibacterial such as clindamycin or a pharmaceutically acceptable salt thereof (e.g. clindamycin phosphate). Alternatively, in an embodiment, the present compositions comprise tretinoin in combination with an antibacterial agent, such as clindamycin or a pharmaceutically acceptable salt thereof (e.g. clindamycin phosphate).

Suitable concentration ranges for the retinoid in the composition include, for example, about 0.001% to about 1% by weight of the composition, In one embodiment the retinoid is present in an amount from about 0.01% to about 1% by weight. In another embodiment the retinoid is present in an amount from about 0.025% to about 0.5% by weight. In another embodiment the retinoid is present in an amount from about 0.005% to about 0.025% by weight. In one embodiment when the retinoid is tazarotene, it is present in an amount from about 0.05% or 0.1% by weight. In another embodiment when the retinoid is tretinoin, it is present in an amount from about 0.005%, 0.025%, 0.05% or 0.1% by weight. In another embodiment when the retinoid is retinol, it is present in an amount from about 0.05% or 0.1% or 1.0% by weight.

A suitable retinoic acid metabolic blocking agent (RAMBA) for use herein as a pharmaceutically acceptable active agent is Talarozole.

Suitable cannabinoids, including tetrahydrocannabinol and cannabidiol.

Suitable terpenes include myrcene, limonene, caryophyllene, pinene, ocimene, geraniol and terpinolene or combinations of such.

In another embodiment alpha and beta hydroxy acids and polymers and derivatives thereof are selected from: alkyl hydroxycarboxylic acids, aralky1 and aryl 2-hydroxycarboxylic acids, polyhydroxy-carboxylic acids, hydroxy-polycarboxylic acids. 2-hydroxycarboxylic acids present in forms other than the acid, such as, for example, salts or lactones; typical lactone forms include, for example, gluconolactone, galactonolactone, glucuronolactone, galacturonolactone, gulonolactone, ribonolactone, saccharic acid lactone, pantoyllactone, glucoheptonolactone, mannonolactone, and galactoheptonolactone; 2-ketoacids present as a free acid or an ester form, or in a salt form with an organic base or an inorganic alkali; representative 2-ketocarboxylic acids and their esters: ascorbic acid, quinic acid, isocitric acid, tropic acid (2-phenyl 3-hydroxypropanoic acid), trethocanic acid, 3-chlorolactic acid, citramalic acid, agaricic acid, aleuritic acid, pantoic acid, lactobionic acid and hexulosonic acid.

Suitable immune response modifier compounds, immunosuppressant agents, immunoregulating agents and immunomodulators for use herein include chemically or biologically-derived agents that modify the immune response or the functioning of the immune system (by the stimulation of antibody formation or the inhibition of white blood cell activity). Exemplary agents or compounds include, but are not limited to, cyclic peptides (such as cyclosporine), tacrolimus, tresperimus, pimecrolimus, sirolimius (rapamycin), verolimus, laflunimus, laquinimod, mycophenolic acid, and imidazoquinoline amines such as imiquimod, salts thereof, and mixtures thereof.

Suitable vitamin D analogues include, but are not limited to, calcidiol, calcitriol, calcipotriene, paricalcitol, 22-oxacolcitriol, dihydrotachysterol, calciferol, salts thereof, and mixtures thereof.

Suitable vitamin B3 compounds include nicotinamide (aka niacinamide).

Suitable corticosteroids include, but are not limited to, alclometasone dipropionate, amcinonide, beclomethasone dipropionate, betamethasone benzoate, betamethasone dipropionate, betamethasone valerate, budesonide, clobetasol propionate, clobetasone butyrate, cortisone acetate, desonide, desoximetasone, diflorasone diacetate, diflucortolone valerate, fluclorolone acetonide, flumethasone pivalate, fluocinolone acetonide, fluocinonide, fluocortin butyl, fluocortolone, fluprednidene acetate, flurandrenolide, flurandrenolone, fluticasone propionate, halcinonide, halobetasol propionate, hydrocortisone, hydrocortisone acetate, hydrocortisone butyrate, hydrocortisone propionate, hydrocortisone valerate, methylprednisolone acetate, mometasone furoate, pramoxine hydrochloride, prednisone acetate, prednisone valerate, triamcinolone acetonide, prednicarbate, salts thereof, and mixtures thereof.

Suitable hormone replacement agents include testosterone and estradiol.

Combinations of vitamin D analogues calcidiol, calcitriol, calcipotriene, paricalcitol, 22-oxacolcitriol, dihydrotachysterol, calciferol, or calcipotriol and corticosteroids, for example fluticasone propionate or mometasone furoate in combination with calcipotriene (aka calcipotriol), are suitable.

Suitable anti-rosacea compound include, but are not limited to, clindamycin, erythromycin, metronidazole, nicotinamide and azelaic acid.

Suitable antihistamines include, but are not limited to, cetirizine, vapitadine, diphenhydramine, triprolidine, pyrilamine, chlorcyclizine, promethazine, carbinoxamine, tripelennamine, brompheniramine, hydroxyzine, terfenadine, chlorpheniramine, salts thereof, and mixtures thereof.

Suitable antibacterial agents include, but are not limited to, gentamycin, neomycin, streptomycin, cefpodoxime proxetil, clindamycin, lincomycin, erythromycin, bacitracin, gramicidin(s), vancomycin, doxycycline, minocycline, oxytetracycline, tetracycline, fosfomycin, fusidic acid, mupirocin, sulfacetamide, metronidazole and dapsone, salts thereof, and mixtures thereof.

Suitable antifungal agents include, but are not limited to, those selected from the group consisting of echinocandins such as anidulafunin, caspofungin and micafungin; polyenes such as amphotericin B, candicidin, filipin, fungichromin, hachimycin, hamycin, lucensomycin, mepartricin, natamycin, nystatin, pecilocin, perimycin; allylamines such as butenafine, naftifine and terbinafine; imidazoles such as bifonazole, butoconazole, chlormidazole, cloconazole, clotrimazole, econazole, enilconazole, fenticonazole, flutrimazole, isoconazole, ketoconazole, lanoconazole, miconazole, neticonazole, omoconazole, oxiconazole nitrate, sertaconazole, sulconazole and tioconazole; thiocarbamates such as liranaftate, tolciclate, tolindate and tolnafate; triazoles such as albaconazole, fluconazole, itraconazole, posaconazole, ravuconazole, saperconazole, terconazole and voriconazole; and other antifungal agents such as acrisorcin, amorolfine, biphenamine, bromosalicylchloranilide, buclosamide, calcium propionate, chlorphenesin, ciclopirox, cloxyquin, coparaffinate, exalamide, flucytosine, haloprogin, hexetidine, loflucarban, nifuratel, potassium iodide, propionic acid, pyrithione, salicylanilide, sodium propionate, sulbentine, tenonitrozole, triacetin, undecylenic acid, zinc propionate, griseofulvin, oligomycins, pyrrolnitrin, siccanin, viridian, salts thereof, and mixtures thereof.

Suitable antivirals include, but are not limited to, acyclovir, desciclovir, carbovir, famciclovir, foscarnet sodium, ganciclovir, interferons, penciclovir, valaciclovir, salts thereof, and mixtures thereof.

Suitable cytotoxic agents include, but are not limited to, azathioprine, cyclophosphamide, cyclosporine, methotrexate, hydroxyurea, thalidomide, bleomycin, diclofenac, fluorouracil, salts thereof, and mixtures thereof.

An exemplary psoralen is methoxsalen. An exemplary anti-alopecia agent is minoxidil.

Suitable anti-androgens include, but are not limited to, spironolactone, cyproterone, flutamide, finasteride, salts thereof, and mixtures thereof.

Suitable anti-pruritics include, but are not limited to, calamine, camphor and menthol, and mixtures thereof. Other suitable anti-pruritics include kappa opioid agonists, protease inhibitors and the PAR-2 inhibitors.

Suitable keratolytic agents, for example for use in the treatment of acne include, but are not limited to, benzoyl peroxide, salicylic acid, urea, resorcinol, sulphur, salts thereof, and mixtures thereof.

Suitable antiseptics include, but are not limited to chlorhexidine, cetrimide, povidone iodine, triclosan, salts thereof, and mixtures thereof. Suitable anaesthetics and analgesics include, but are not limited to, benzocaine, lidocaine, prilocaine and choline salicylate, salts thereof, and mixtures thereof.

Suitable nonsteroidal anti-inflammatory agents include, but are not limited to, diclofenac, ibuprofen, ketorolac and ketoprofen and their optical isomers, salicylate esters including methyl salicylate and associated agents such as menthol, camphor, capsaicin and the like.

Suitable nonsteroidal anti-inflammatory agents with specificity for COX-2 over COX-1 include but are not limited to, celecoxib and refecoxib.

Suitable other anti-inflammatory agents include nicotinamide, resveratrol, cumin, gallates, for use in treating inflammatory skin diseases such as eczema, psoriasis and rosacea.

Suitable vasoactive agents include, but are not limited to glyceryl trinitrate, Alprostadil and the like.

Suitable agents for treatment of dry and ageing skin include lactic acid, glycolic acid and lactobionic acid, palmitoyl tri- and tetra- peptides, hyaluronic acid and salts, natural NF-kB inhibitors such as resveratrol, curcumin and gallates.

Suitable UV blocking agents include nano and micro particles of titanium, zinc or silicone or other inorganic oxides.

The composition of the present invention is generally in the form of a cream or serum.

According to one embodiment, the composition of the present invention is in the form of a serum or gel-serum.

According to one embodiment, the composition of the present invention is in the form of a cream. Polymers, particles and co-emulsifiers may be included to improve physical stability.

The active agents and other ingredients may form suspensions, solutions, or emulsions in suitable silicone oil or aqueous vehicles, and may contain formulatory agents such as suspending, stabilizing and/or dispersing agents. It is possible to add, if necessary, an adjuvant chosen from antioxidants, surfactants, other preservatives, film-forming, keratolytic or comedolytic agents, perfumes, flavorings and colourings.

The composition disclosed herein generally comprises less than about 50 % water by weight, preferably less than about 15% water by weight, more preferably less than about 10 % water by weight, most preferably less than 0.05% water by weight. According to one embodiment, the composition of the present invention comprises substantially no water.

The composition of the present invention is generally self-preserving, and may be typically sterile. The sterile media employed in the preparation of suitable compositions are all readily obtainable by standard techniques well-known to those skilled in the art.

Forms chiefly conditioned for application to biological membranes may take the form, for example, of creams, milks, gels, dispersions or lotions thickened to a greater or lesser extent, aerosol compositions (e.g. sprays or foams) or lotions. Other conventional forms for this purpose include serums, creams, lotions, and sprays.

The composition of the present invention may comprise an aqueous or non-aqueous base also comprising one or more dispersing agents, solubilising agents or suspending agents. Liquid sprays can be pumped, or are conveniently delivered from pressurized packs. Drops can be delivered via a simple dropper-capped bottle, via a plastic bottle adapted to deliver liquid contents drop-wise, or via a specially shaped closure. Airless pump packs are particularly preferred for cosmetic use

The percentage by weight of a therapeutic agent of the invention present in the topical composition will depend on various factors, but generally will be from 0.005% to 10% of the total weight of the composition, and typically 0.1-5% by weight.

The active agents mentioned in this specification can exist in different forms, such as free acids, free bases, esters and other prodrugs, salts and tautomers, for example, and the invention includes all variant forms of the agents.

There is also provided the product described above for therapeutic use.

**Method of Treatment**

Disclosed herein is a method of preventing, reducing the likelihood of, alleviating or treating a medical condition in the human or animal body comprising the topical administration in a therapeutically effective amount of the composition described herein.

According to a further aspect of the present invention, there is provided the composition as described herein for use in the prevention, alleviation or treatment of a medical condition of the human or animal body.

Generally, the methods/uses of the present invention provide topical relief of the medical condition for at least 4 hours after administration of the composition, typically at least 6 hours, suitably at least 12 hours after administration.

As the composition of the present invention is administered topically, the effects are generally associated with a rapid onset. Generally, the composition provides topical relief from the medical condition within 30 minutes or less of application, typically within 15 minutes or less, suitably within 5 minutes or less of application.

Generally, the topical relief effects have a duration of 6-8 hours, typically at least 12 hours, preferably 24 hours.

The method involves the topical application of the composition to the area affected by the condition or disorder, and to surrounding tissue. Topical application of the composition improves the bioavailability of the pharmaceutical actives contained therein, making dosing more predictable and reducing the likelihood of any adverse reaction.

The methods and uses of the present invention are topical and the method of the present invention typically provides a focused method of exploiting the beneficial effects of the active agent or combination thereof whilst limiting the associated dosage required and the risk of adverse effects.

The methods/uses of the present invention generally involve the application of the composition to the skin or mucous membrane(s) of the patient. According to one embodiment, the methods/uses may involve the application of the composition to the genitals of the patient.

The medical condition may be selected from the group consisting of conditions associated with or caused by one or more of pain and/or inflammation, pigmentation, pruritus, acne, eczema, psoriasis, rosacea, skin blistering diseases such as bullous pemphigoid, nappy rash, dry skin, microbial conditions including fungal and/or bacterial conditions such as skin infections including yeast infections and dermatophyte infections, viral infections of the skin or mucosa, warts, dry or ageing skin, hypoandrogenism, immunological conditions, sun spots, actinic keratosis, basal cell and squamous cell skin cancers and melanoma, alopecia and dermatitis due to radiation therapy.

Suitable skin conditions for treatment with the composition of the present invention include vitiligo, eczema, psoriasis and skin problems related to certain lymphomas.

The method disclosed herein may include the application of UV light to the affected area.

The medical condition may be a viral, fungal and/or bacterial condition.

The medical condition may be partly or wholly caused by or associated with a local immune response, in particular involving a histamine, wherein the composition comprises one or more antihistamines, immunosuppressant agents, immunoregulating agents and/or immunomodulators.

According to one embodiment, the method promotes the growth and repair of body tissues and includes the application of the composition comprising one or more steroid compounds, in particular one or more corticosteroid compounds.

The medical condition may be partly or wholly caused by or associated with a vitamin D deficiency.

According to one embodiment, the method treats, mitigates or prevents the formation of wrinkles, acne or psoriasis and includes the application of the composition comprising one or more of the group consisting of retinoid compounds, retinoic acid metabolic blocking agents (RAMBAs), alpha and beta hydroxy acids and polymers.

According to one embodiment, the composition comprises one or more analgesic or anti-inflammatory compounds and the condition is associated with topical and/or inflammation. The medical condition is generally treated via topical application. The active agents of the present invention are administered topically, typically to the skin or mucous membrane of the patient. The composition of the present invention is generally in the form of a pharmaceutical composition

adapted to such a route, and in a dose effective for the treatment, prevention or mitigation intended.

The amount of therapeutically active agent that is administered and the dosage regimen for treating a condition with the compounds and/or compositions of this invention depends on a variety of factors, including the age, weight, sex and medical condition of the subject, the severity of the condition, the route and frequency of administration, and the particular compound employed, as well as the pharmacokinetic properties of the individual treated, and thus may vary widely. The dosage will generally be lower if the compounds are administered for prevention rather than for treatment. Such treatments may be administered as often as necessary and for the period of time judged necessary by the treating physician. One of skill in the art will appreciate that the dosage regime or therapeutically effective amount of the inhibitor to be administrated may need to be optimized for each individual.

The methods/uses of the present invention generally involve application of the composition 1 to 5 times per day, typically 1 to 2 times per day, suitably one time per day.

The method disclosed herein may be undertaken whenever required. Generally courses of treatment last 1 to 6 months depending on the nature and severity of the condition to be treated.

The composition of the present invention is generally applied to a human.

In particular, there is provided a prophylactic method of preventing or minimizing the onset of the symptoms of a medical condition to a patient who has previously suffered from these symptoms.

Where the method disclosed herein is used preventatively, a course of treatment may last up to six months. However, where the method is used to treat severe symptoms, the method/use may involve an initial strong dose of the active agent or combination thereof. This may be followed by a relatively lower dose for a second prolonged period of from, for instance 1 to 6 months.

The total amount of the composition applied per dose is generally around 2.5-10.00mg of composition per $cm^2$ of skin treated.

The active agents may be administered simultaneously, sequentially or separately. The active agents may be provided as a combination package. The combination package may contain the product of the invention together with instructions for simultaneous, separate or sequential administration of each of the active agents. For sequential administration, the active agents can be administered in any order.

The active agents of the methods/uses of the invention may be provided as pharmaceutical compositions additionally containing one or more pharmaceutically acceptable diluents, excipients and/or carriers. This applies to both fixed and free combinations.

The phrase "pharmaceutically acceptable" is employed herein to refer to those compounds, materials, and/or compositions which are, within the scope of sound medical judgment, suitable for use in contact with the tissues of human beings or, as the case may be, an animal without excessive toxicity, irritation, allergic response, or other problem or complication, commensurate with a reasonable benefit/risk ratio. Such carriers are well known in the art and include buffers, fillers, extenders, binding agents, moisturizing agents, disintegrating agents, resorption accelerators, surface active agents, adsorptive carriers, lubricants and preservatives.

The patient is generally a human although in some embodiments, an animal may be treated.

**Composition examples: Specific Compositions**

The present invention will now be described by way of example only with reference to the accompanying tables.

According to an embodiment of the present invention, there is provided a composition for topical application comprising:

a. an active agent(s);
b. a user adherence-improving skin barrier restoring combination of:
c. 1.0 to 5%w/w of nicotinamide
d. 1.0 to 5%w/w of polyhydroxy acid;
e. a partition coefficient enhancer, in particular propylene glycol, butylene glycol, pentylene glycol, or hexylene glycol (generally propylene glycol);
f. a diffusion coefficient enhancer selected from the group consisting of a $C_{12}$ to $C_{14}$ saturated fatty acid and a $C_{14}$ saturated primary alcohol;
g. a hydrocarbyl methyl siloxane emollient selected from the group consisting of alkyl methyl siloxane compound or an alkyl aryl methyl siloxane compound and having a number average molecular weight of less than 500;
h. a dimethicone macromer surfactant, generally having a number average molecular weight of more than 1000, typically comprising a polyalkylsiloxane portion and one or more copolymers of ethylene oxide and propylene oxide or ionic pendant groups.
i. a methyl siloxane, in particular a cyclomethicone compound having a number average molecular weight of less than 500;

j. a cross-linked dimethicone macromer generally having a number average molecular weight of more than 1000, and generally comprising a polyalkylsiloxane portion.

Examples of the current invention are from the classes cannabinoids (examples #1-6), antivirals and antifungals (examples #7-13), retinoids (examples #14-17), vitamin B3 (examples #18-24), immunomodulators (examples #25-26), humectants (examples #29-34) and corticosteroids (examples #35-37).

Process-related examples (Process control of Nicotinamide-PHA compositions)

Tables #8-11 (examples #38-#55) are described after the next section.

**Cream Composition Technology and Manufacturing Process (0.5-3.0kg laboratory scale)**

**Glycol-in-silicone cream technology**

Glycol-in-silicone emulsion cosmetic technology stems from work in the 1960's on non-aqueous emulsions; for example, glycerin dispersions in olive oil (Perterson RV and Hamill RD. J Soc. Cos. Chem. 1968;19:627-640). Florence AT et al. reported on "Novel anhydrous emulsions" using the silicone surfactant systems cyclomethicone/PEG/PPG-18/18 dimethicone and cyclopentasiloxane/PEG/PPG-18/18 dimethicone to stabilise anhydrous castor oil-in-silicone fluid dispersions. The important breakthrough into cosmetic science was the publication in 1996 by Zombec and Dahms (Dow Corning Corporation) of "Novel compositions based on non-aqueous emulsions of polyols (propylene glycol) in Silicones". Stable anhydrous propylene glycol-in-silicone emulsions were prepared using dimethicone copolyol in cyclomethicone and sodium chloride to salt out the surfactant from the glycol phase.

Co-enhancer in silicone cream technology adds a diffusion coefficient enhancer and optionally further glycol/water solvents into the propylene glycol partition coefficient enhancer and an hydrocarbyl methyl siloxane emollient, such as caprylyl methicone and silicone elastomers/fluids into the silicone continuous phase.

**Overview of manufacturing process**

The process uses two premix stages, firstly the N-VRP dispersed phase premix, then the silicone continuous phase premix. At small scale (0.50-3.0kg), the highly volatile trisiloxane, although a part of the silicone continuous phase, is added as a final stage after dispersion of the N-VRP premix into the silicone premix to avoid loss of trisiloxane at small scale. This may not be required in a closed manufacturing system

At the small scale (0.50-3.0kg), a Kenwood Chef Professional stand mixer (KVC7300S. 1.5kW, 4.6l capacity bowl) is used to form the glycol-in-silicone cream dispersions. These dispersions are white, rather than the grey colour often associated with silicone serums. Conventional Silverson-type homogenisers cavitate and are not useful to form these dispersions. However, in-line homogenisation, used at larger scale, may further improve the whiteness of these creams.

The table below show a typical listing of the Materials, but with primary actives omitted. Each material has its function and several multi-task, such that typically only 12-13 ingredients are required. The importance of In-Process-Control (IPC) is described in Tables #8-11.

| Premix | Materials | Order of addition | In process control |
|---|---|---|---|
| Tare weight | | | IPC |
| N-VRP dispersed premix | Sodium chloride | 1 | |
| | DI water | 2 | |
| | Propylene glycol | 3 | |
| | Nicotinamide (NAM) | 4 | |
| Check weight check | | | IPC |
| pH after NAM addition | | | IPC |
| | Gluconolactone | 5 | |
| | Lactobionic Acid | 6 | |

(continued)

| Premix | Materials | Order of addition | In process control |
|---|---|---|---|
| Check weight check | | | IPC |
| pH after PHA addition | | | IPC |
| pH adjusters/buffers | 25% LA NH3/ 25% NaOH/80% Lactic acid | 7 | |
| pH after addition | | | IPC |
| Post N-VRP addition to N-VRP | Phenoxyethanol | 8 | |
| | Myristyl alcohol | 9 | |
| Silicone continuous phase premix | EL-7040 hydro elastomer | 10 | |
| | DC-9040 elastomer | 11 | |
| | Carbomer | 12 | |
| | Trisiloxane | 13 | |
| Cream pH (4.4-4.8) | | | IPC |

As a general principle, non-volatile residual phase (N-VRP) materials are added in order of decreasing polarity and with the intent not to induce any sharp changes in the polarity nor to create supersaturation in-process.

*N-VRP Premix*

*Step/Material #1 Sodium chloride*

Sodium chloride is weighed out into a tared container, at 0.5kg scale, a 500ml Dewar flask.

*Step/Material #2 Deionised water*

Deionised water is weighed out into the 500ml Dewar flask and the flask shaken for 1 minute to complete solution.

*Step/Material #3 Propylene glycol*

Propylene glycol is weighed out into the 500ml Dewar flask.

*Step/Material #4 Niacinamide (Figure 7.1a and 7.1b)*

Niacinamide (sieved) is weighed out into the 500ml Dewar flask and the flask mixed on a rotating bed until a clear solution is obtained.

*Step/Material #5/#6 PHAs (Figures 7.2a, 7.26 and 7.2c)*

Gluconolactone #5 and then Lactobionic acid #6 are weighed out into the 500ml Dewar flask and the flask mixed on a rotating bed until a clear solution is obtained.

*Step/Material #7 addition of pH adjuster*

Add pH adjuster dropwise to appropriate target pH.

*Post N-VRP addition to N-VRP*

*Step/Material #8 addition of microbiological preservative*

Add phenoxyethanol dropwise to target weight. It is totally soluble in the N-VRP.

*Step/Material #9 addition of diffusion coefficient enhancer, myrystyl alcohol ($C_{14}$ alcohol) (Figures 7.3a, 7.36 and 7.3c)*

Myrystyl alcohol is weighed out into the 500ml Dewar flask. It is only soluble to 50% of its weight in the N-VRP and requires heating to ~30°C to form a crude dispersion which facilitates partitioning into the silicone continuous phase. At ambient temperature and slightly above the myrystyl alcohol is at saturated solubility in both phases and at approximately equal concentrations.

*Silicone continuous phase Premix*

*Step/Material #10/11/12 addition of silicone elastomer blends and Carbomer (Figures 7. 4a, 7.4b, 7.4c, 7.4d and 7.4e)*

A Kenwood Chef Professional stand mixer (KVC7300S. 1.5kW, 4.6l capacity bowl) is used to form the glycol-in-silicone cream dispersions. The highest speed setting was used but built up slowly so as not to cause splashing. The elastomer-silicone fluid blends (EL-7040 hydro elastomer and DC-9040 elastomer) are weighed into the bowl and then mixed for 1 minute (figure 7.4a). Material on sides of bowl scrapped to middle with a silicone spatula (figure 7.4b) and then mixed for 1 minute, sides of bowl scrapped to middle with a silicone spatula and the Carbomer added: this is then mixed for a further minute, sides of bowl scrapped to middle and this repeated (figures 7.4c, 7.4d and 7.4e).

*Addition of N-VRP premix to silicone elastomer blend-carbomer premix*

The mixer speed setting is increased with 1 sec intervals to maximum speed and the crude dispersion of myrystyl alcohol in the N-VRP is added into the silicone elastomer blend-carbomer premix as a "breaking stream" such that, at the 0.5kg scale, it takes ~12 minutes to complete the addition (~20g/minute). The initial glycol-in-silicone emulsion has sides of bowl scrapped to middle with a silicone spatula and then remixed at full speed for two minutes

*Step/Material #13 addition of volatile silicone fluid Trisiloxane (Figures 7.5a and 7.5b)*

Trisiloxane is weighed into the Kenwood bowl, added to the initial glycol-in-silicone mix. The mixer speed setting is increased with 1 sec intervals to maximum speed and mixed for 2 minutes. Then, sides of bowl scrapped to middle and this repeated. The resulting cream was packed into 500g vapour tight plastic buckets:

*Examples #39-54 (tables #8-#11)*

Table #8 shows a series of compositions (#39-#43) in which the ratio of PHAs (Lactobionic acid:Gluconolatone) is varied over the range 1:0, 3:1 and 1:1. Also in compositions (#41-#43) the ratio of nicotinamide:total PHA is varied over the range 2:1, 1.5:1 and 1:1. In all of these compositions, only the volatile buffer liquid (aqueous) ammonia is used to bring the glycol phase (materials 1-7) to a pH of ~4.6. Because the continuous silicone phase of the cream is anhydrous, this does not influence the pH of the glycol-silicone cream dispersion, which is thus also at pH ~4.6. On application of the cream to the skin as a thin film the ammonia is lost by evaporation and the pH decreases to an equilibrium primarily depending upon the ratio of nicotinamide:total PHA. In some embodiments of the invention a target equilibrium pH of 3.5 is appropriate, this, co-incidentally, being the pKa of both nicotinamide (a base) and PHAs (acids) and thus works to co-optimise the skin permeation of these ancillary actives. Example #40 containing 5% NAM at pH ~ 4.4 shows no reduction in NAM potency over 6 months at 40°C. US patent number 10,028,927 (Limeway invention for Futura Medical, UK) describes the use of the volatile buffer liquid ammonia to achieve a product pH of ~7.00 at which diclofenac is chemically stable, but reducing to the pKa, pH of ~4.00, on application to the skin, thus to optimise skin permeation.

Table #9 shows a series of compositions (#44-#47) in which the ratio of nicotinamide:total PHA is varied over the range 2:1, 1.50:1 and 1:1. Equilibrium pH is dependent on the ratio of nicotinamide:total PHA and at a 2:1 ratio is ~pH 4.00. This equilibrium pH may be optimum if the primary objective is to maximise the skin permeation of the nicotinamide component of the ancillary actives. Compositions (#47) uses a non-volatile basic buffer, 25% NaOH to take the equilibrium (and product) pH to ~5.00, where nicotinamide is almost totally in the unionised state. In compositions (#44-#46) the volatile buffer liquid (aqueous) ammonia is used to bring the glycol phase (materials 1-7) to a pH of ~4.6, which reduce to =< pH 4.00 on application to the skin.

Table #10 shows a series of compositions (#48-#51) in which the ratio of nicotinamide:total PHA is varied over the range 1:1and 0.5:1 (both as duplicate batches). In all of these compositions the non-volatile buffer lactic acid is used to decrease equilibrium pH. Then, the volatile buffer liquid (aqueous) ammonia is used to bring the glycol phase (materials 1-7) to a pH of ~4.6. Because the continuous silicone phase of the cream is anhydrous, this does not influence the pH of the glycol-silicone cream dispersion, which is thus also at pH ~4.6. On application of the cream to the skin as a thin film the ammonia is lost by evaporation and the pH decreases to an equilibrium pH in the range ~3.6-3.8 primarily depending upon the ratio of nicotinamide:total PHA and the amount of the non-volatile buffer lactic acid added. Table #10 shows how in process control/tracking of pH is most useful in the design and manufacture of these creams. pH reproducibility, by in process control of the weights, particularly of nicotinamide and total PHA, is excellent

Table #11. The texture of creams is an important part of the experience in use. The texture of the glycol-silicone cream dispersion described herein is dominated by the texture of the continuous silicone phase of the cream, which in turn is related to the ratio of elastomeric polymers to the low viscosity silicone fluids, especially trisiloxane. Examples #52-#54 show that, for a fixed amount of elastomeric polymers, viscosity is proportional to the amount of trisiloxane added.

In the discussions on tables #8, #9 and #10 above, we described how the equilibrium pH, that pH realised in the N-VRP after evaporation after loss by evaporation of volatiles (such as water and volatile buffer, if present) is dependent upon the ratio of nicotinamide:total PHA. Of course, addition of acidic and/or basic none-volatile buffers may also be used to adjust equilibrium pH. In addition, it may be advantageous to use know-how derived from US patent number 10,028,927. For example, by using the volatile basis buffer liquid (aqueous) ammonia it is possible to increase product pH to a value of 4.5-6.00, that associated with NAM chemical stability. However, on application to the skin as a thin film the volatiles, including the liquid ammonia volatile buffer evaporate to form an optimum pH for skin permeation, for example in the range pH 3.0-4.0.

Various modifications and variations of the described aspects of the invention will be apparent to those skilled in the art. Although the invention has been described in connection with specific preferred embodiments, it should be understood that the invention as claimed should not be unduly limited to such specific embodiments. Indeed, various modifications of the described modes of carrying out the invention which are obvious to those skilled in the relevant fields are intended to be within the claims.

**Chemical Structure and Stability of Nicotinamide creams**

Nicotinamide (aka niacinamide) (NAM) is the amide of nicotinic acid with chemical structure as shown in Figure 8a.

Amides, as a class, are much more stable to hydrolysis to the acid than are esters, but this is still the main route of degradation of NAM. Finholt and Higuchi (Rate Studies on the Hydrolysis of Niacinamide. J. Pharm Sci, 1962,51(7);p655-661) reported that the minimum first-order rate of NAM hydrolytic degradation was found in the pH range 4.5-6.0 (see figure 8a, adapted rate plot). The cream composition described in Table 8, #40, containing 5% nicotinamide and 5% PHA at pH 4.402 was stored at 5°C and 40°C for 6 months in sealed glass jars; no significant degradation of NAM was observed. Physical stability was as DOM at 5°C and 40°C for 6 months. Figure 8b shows that at 40C storage for 6 months, there was a slight greying, see middle jar compared with left at 5°C, believed to be due to the PHA. The right-hand jar is a fresh control sample.

NAM content in compositions 5 % NAM+ 5% PHA stored at 5°C and 40°C (mean ± SD, n = 5); pH 4.402 (target 4.40-4.80).

| Compositions | Assay (%, w/w) | |
|---|---|---|
| | Samples stored at 5°C | Samples stored at 40°C |
| NAM + PHA 1 | 5.42±0.36 | 5.43±0.47 |

NAM content in compositions NAM + PHA 2-6, stored at 5°C and 40°C for 6 months (mean ± SD, n = 5), pH 4.490, 4.480, 4.514, 4.495 and 4.440 respectively (target 4.40-4.60); see table below.

| Compositions | Assay (%, w/w) | |
|---|---|---|
| | Samples stored at 5°C | Samples stored at 40°C |
| NAM + PHA 2 | 6.55±0.28 | 6.43±0.33 |
| NAM + PHA 3 | 8.34±0.20 | 8.62±0.86 |
| NAM + PHA 4 | 10.23±0.39 | 10.71±0.39 |

(continued)

| Compositions | Assay (%, w/w) | |
|---|---|---|
| | Samples stored at 5°C | Samples stored at 40°C |
| NAM + PHA 5 | 10.33±0.45 | 10.73±0.40 |
| NAM + PHA 6 | 10.51±0.64 | 10.62±0.61 |

NAM + PHA compositions 2-6, were stored at 5°C and 40°C for 6 months in sealed glass jars; no significant degradation of NAM was observed.

In contrast, samples at pH ~3.8-3.9, retained at ambient UK temperature, showed a progressive decrease in NAM potency of approximately 20% over 24 months; see table below.

| Samples | pH DOM | DOM 21/01/19 | % w/w 07/08/19 | % w/w 25/01/21 | Comments |
|---|---|---|---|---|---|
| Test A 6% NAM 6% PHA | 3.827 | 6.13 | 5.50 | - | Date of manufacture 21/01/19, ~pH 3.8-3.9 |
| Test B 8% NAM 8% PHA | 3.828 | 8.03 | 7.20 | - | |
| Test C 10% NAM 10% PHA | 3.931 | 10.17 | 9.20 | 8.08±0.41 | |

NAM is exceptionally stable to degradation provided the pH is adjusted into the range pH 4.5-6.00. There are many options, using organic or inorganic bases, to adjust pH to this target range. As we have described, a further option is to use volatile buffer technology to achieve different, individually optimized, in-pack and equilibrium phase pH as described in US 10,028,927.

**Examples: Table 1 Cannabinoid actives**

| Materials | Example #1 g/500g batch | Example #2 g/500g batch | Example #3 g/500g batch | Example #4 g/500g batch | Example #5 g/500g batch | Example #6 g/500g batch |
|---|---|---|---|---|---|---|
| Active: | Cannabidiol 5.00 | Tetrahydrocannabinol 5.00 | Cannabidiol 5.00 | Tetrahydrocannabinol 5.00 | Cannabidiol 5.00 | Tetrahydrocannabinol 5.00 |
| Ancillary Barrier Agents | | | | | | |
| Lactobionic Acid | 20.00 | 20.00 | 10.00 | 10.00 | 4.00 | 4.00 |
| Gluconolactone | 5.00 | 5.00 | 2.50 | 2.50 | 1.00 | 1.00 |
| Total ABA's (5-25g/500g) | 25.00 | 25.00 | 12.50 | 12.50 | 5.00 | 5.00 |
| Nicotinamide (5-25g/500g) | 25.00 | 25.00 | 12.50 | 12.50 | 5.00 | 5.00 |
| Other ingredients | | | | | | |
| Sodium chloride | 2.00 | 2.00 | 2.00 | 2.00 | 2.00 | 2.00 |
| Glycerol | 17.50 | 17.50 | 17.50 | 17.50 | 17.50 | 17.50 |
| Propylene glycol | 150.00 | 150.00 | 150.00 | 150.00 | 170.00 | 170.00 |
| DI water | 28.50 | 28.50 | 28.50 | 28.50 | 28.50 | 28.50 |
| Myristyl alcohol | 7.50 | 7.50 | 7.50 | 7.50 | 7.50 | 7.50 |
| Phenoxyethanol | 2.50 | 2.50 | 2.50 | 2.50 | 2.50 | 2.50 |
| Trisiloxane | 55.00 | 55.00 | 80.00 | 80.00 | 75.00 | 75.00 |
| Carbomer | 2.00 | 2.00 | 2.00 | 2.00 | 2.00 | 2.00 |
| EL-7040 hydro elastomer[1] | 120.00 | 120.00 | 120.00 | 120.00 | 120.00 | 120.00 |
| DC-9040 elastomer[2] | 60.00 | 60.00 | 60.00 | 60.00 | 60.00 | 60.00 |
| Total | 500.00 | 500.00 | 500.00 | 500.00 | 500.00 | 500.00 |

1 Caprylyl Methicone (and) PEG-12 Dimethicone/ PPG-20 Crosspolymer
2 Cyclopentasiloxane (and) Dimethicone Crosspolymer

**Examples: Table 2 Antivirals and Antifungal actives**

| Materials | Example #7 g/500g batch | Example #8 g/500g batch | Example #9 g/500g batch | Example #10 g/500g batch | Example #11 g/500g batch | Example #12 g/500g batch | Example #13 g/500g batch |
|---|---|---|---|---|---|---|---|
| Active: | Acyclovir 5.00 | Penciclovir 5.00 | Acyclovir 2.50 | Penciclovir 2.50 | Econazole 2.50 | Ketoconazole 5.00 | Miconazole 5.00 |
| Ancillary Barrier Agents | | | | | | | |
| Lactobionic Acid | 20.00 | 20.00 | 18.75 | 18.75 | 18.75 | 18.75 | 18.75 |
| Gluconolactone | 5.00 | 5.00 | 6.25 | 6.25 | 6.25 | 6.25 | 6.25 |
| Total ABA's (5-25g/500g) | 25.00 | 25.00 | 25.00 | 25.00 | 25.00 | 25.00 | 25.00 |
| Nicotinamide (5-25g/500g) | 25.00 | 25.00 | 25.00 | 25.00 | 25.00 | 25.00 | 25.00 |
| Other ingredients | | | | | | | |
| Sodium chloride | 2.00 | 2.00 | 2.00 | 2.00 | 2.00 | 2.00 | 2.00 |
| Glycerol | 17.50 | 17.50 | - | - | - | - | - |
| Propylene glycol | 150.00 | 150.00 | 200.00 | 200.00 | 200.00 | 200.00 | 200.00 |
| DI water | 28.50 | 28.50 | 28.50 | 28.50 | 28.50 | 28.50 | 28.50 |
| Myristyl alcohol | 7.50 | 7.50 | 7.50 | 7.50 | 7.50 | 7.50 | 7.50 |
| Phenoxyethanol | 2.50 | 2.50 | 2.50 | 2.50 | 2.50 | 2.50 | 2.50 |
| Trisiloxane | 55.00 | 55.00 | 50.00 | 50.00 | 50.00 | 47.50 | 47.50 |
| Carbomer | 2.00 | 2.00 | 2.00 | 2.00 | 2.00 | 2.00 | 2.00 |
| EL-7040 hydro elastomer[1] | 120.00 | 120.00 | 103.00 | 103.00 | 103.00 | 103.00 | 103.00 |
| DC-9040 elastomer[2] | 60.00 | 60.00 | 52.00 | 52.00 | 52.00 | 52.00 | 52.00 |
| Total | 500.00 | 500.00 | 500.00 | 500.00 | 500.00 | 500.00 | 500.00 |

1 Caprylyl Methicone (and) PEG-12 Dimethicone/ PPG-20 Crosspolymer
2 Cyclopentasiloxane (and) Dimethicone Crosspolymer

**Examples: Table 3 Retinoid actives**

| Materials | Example #14 g/500g batch | Example #15 g/500g batch | Example #16 g/500g batch | Example #17 g/500g batch |
|---|---|---|---|---|
| Active: | Adapalene 0.50 | Tazarotene 0.50 | Retinoic acid 0.05 | Retinol 0.50 |
| Ancillary Barrier Agents | | | | |
| Lactobionic Acid | 20.00 | 20.00 | 10.00 | 10.00 |
| Gluconolactone | 5.00 | 5.00 | 2.50 | 2.50 |
| Total ABA's (5-25g/500g) | 25.00 | 25.00 | 12.50 | 12.50 |
| Nicotinamide (5-25g/500g) | 25.00 | 25.00 | 12.50 | 12.50 |
| Other ingredients | | | | |
| Sodium chloride | 2.00 | 2.00 | 2.00 | 2.00 |
| Glycerol | 17.50 | 17.50 | 17.50 | 17.50 |
| Propylene glycol | 150.00 | 150.00 | 150.00 | 150.00 |
| DI water | 28.50 | 28.50 | 28.50 | 28.50 |
| Myristyl alcohol | 7.50 | 7.50 | 7.50 | 7.50 |
| Phenoxyethanol | 2.50 | 2.50 | 2.50 | 2.50 |
| Trisiloxane | 59.5 | 59.5 | 84.95 | 84.5 |
| Carbomer | 2.00 | 2.00 | 2.00 | 2.00 |
| EL-7040 hydro elastomer[1] | 120.00 | 120.00 | 120.00 | 120.00 |
| DC-9040 elastomer[2] | 60.00 | 60.00 | 60.00 | 60.00 |
| Total | 500.00 | 500.00 | 500.00 | 500.00 |
| 1 Caprylyl Methicone (and) PEG-12 Dimethicone/ PPG-20 Crosspolymer<br>2 Cyclopentasiloxane (and) Dimethicone Crosspolymer | | | | |

**Examples: Table 4 Vitamin B3 Nicotinamide active**

| Materials | Example #18 g/ 500g batch | Example #19 g/ 500g batch | Example #20 g/ 500g batch | Example #21 g/ 500g batch | Example #22 g/ 500g batch | Example #23 g/ 500g batch | Example #24 g/ 500g batch |
|---|---|---|---|---|---|---|---|
| Active: Nicotinamide | 25.00 | 30.00 | 30.00 | 35.00 | 35.00 | 25.00 | 50.00 |
| Ancillary Barrier Agents | | | | | | | |
| Lactobionic Acid | 18.75 | 18.75 | 18.75 | 18.75 | 18.75 | 18.75 | 18.75 |
| Gluconolactone | 6.25 | 6.25 | 6.25 | 6.25 | 6.25 | 6.25 | 6.25 |
| Total ABA's (5-25g/500g) | (25.00) | (25.00) | (25.00) | (25.00) | (25.00) | (25.00) | (25.00) |
| Nicotinamide (5-25g/500g) | 5.00 | 5.00 | 15.00 | 25.00 | 15.00 | 5.00 | 10.00 |
| % Nicotinamide total | 6.00 (30g/500) | 7.00 (40g/500) | 9.00 (50g/500) | 12.00 (60g/500) | 10.00 (50g/500) | 6.00 (30g/500) | 12.00 (60g/500) |
| Other ingredients | | | | | | | |
| Sodium chloride | 2.00 | 2.00 | 2.00 | 2.00 | 2.00 | 2.00 | 2.00 |
| Glycerol | 17.50 | 2.50 | - | - | - | 17.50 | - |
| Propylene glycol | 150.00 | 175.00 | 200.00 | 200.00 | - | 150.00 | 200.00 |
| Butylene glycol | - | - | - | - | 200.00 | - | - |
| DI water | 28.50 | 28.50 | 8.50 | 8.50 | 8.50 | 28.50 | 8.50 |
| Myristyl alcohol | 7.50 | 7.50 | 7.50 | 7.50 | 8.00 | 8.00 | 7.50 |
| Phenoxyethanol | 2.50 | 2.50 | 2.50 | 2.50 | 2.00 | 2.00 | 2.50 |
| Trisiloxane | 55.00 | 65.00 | 65.00 | 50.00 | 60.00 | 55.00 | 50.00 |
| Carbomer | 2.00 | 2.00 | 2.00 | 2.00 | 2.00 | 2.00 | 2.00 |
| EL-7040 hydro elastomer[1] | 120.00 | 100.00 | 90.00 | 90.00 | 90.00 | 120.00 | 90.00 |
| DC-9040 elastomer[2] | 60.00 | 55.00 | 52.50 | 52.50 | 52.50 | 60.00 | 52.50 |
| Total | 500.00 | 500.00 | 500.00 | 500.00 | 500.00 | 500.00 | 500.00 |

1 Caprylyl Methicone (and) PEG-12 Dimethicone/ PPG-20 Crosspolymer
2 Cyclopentasiloxane (and) Dimethicone Crosspolymer

**Examples: Table 5 Immunomodulator actives**

| Materials | Example #25 g/ 500g batch | Example #26 g/ 500g batch | Example #27 g/ 500g batch | Example #28 g/ 500g batch |
|---|---|---|---|---|
| Active: | Pimecrolimus 0.50 | Pimecrolimus 0.50 | Tacrolimus 0.05 | Tacrolimus 0.05 |
| Ancillary Barrier Agents | | | | |
| Lactobionic Acid | 20.00 | 5.00 | 10.00 | 5.00 |
| Gluconolactone | 5.00 | 1.25 | 2.50 | 1.25 |
| Total ABA's (5-25g/500g) | 25.00 | 6.25 | 12.50 | 6.25 |
| Nicotinamide (5-25g/500g) | 20.00 | 20.00 | 20.00 | 20.00 |
| Other ingredients | | | | |
| Sodium chloride | 2.00 | 2.00 | 2.00 | 2.00 |
| Glycerol | 17.50 | 17.50 | 17.50 | 17.50 |
| Propylene glycol | 150.00 | 167.00 | 150.00 | 165.00 |
| DI water | 28.50 | 28.50 | 28.50 | 28.50 |
| Myristyl alcohol | 7.50 | 7.50 | 7.500 | 7.50 |
| Phenoxyethanol | 2.50 | 2.50 | 2.50 | 2.50 |
| Trisiloxane | 39.50 | 60.00 | 64.95 | 62.45 |
| Carbomer | 2.00 | 2.00 | 2.00 | 2.00 |
| EL-7040 hydro elastomer[1] | 120.00 | 120.00 | 120.00 | 120.00 |
| DC-9040 elastomer[2] | 60.00 | 60.00 | 60.00 | 60.00 |
| Total | 500.00 | 500.00 | 500.00 | 500.00 |
| 1 Caprylyl Methicone (and) PEG-12 Dimethicone/ PPG-20 Crosspolymer<br>2 Cyclopentasiloxane (and) Dimethicone Crosspolymer | | | | |

**Examples: Table 6 Humectant actives**

| Materials | Example #29 g/500g batch | Example #30 g/500g batch | Example #31 g/ 500g batch | Example #32 g/ 500g batch | Example #33 g/ 500g batch | Example #34 g/500g batch |
|---|---|---|---|---|---|---|
| Active: | Resveratrol 5.00 | Hyaluronic acid 7.50 | Glycerin 17.50 | Sorbitol 17.50 | Urea 17.50 | Lactobionic acid 25.00 |
| Ancillary barrier agents | | | | | | |
| Lactobionic Acid | 18.75 | 18.75 | 18.75 | 20.00 | 20.00 | 20.00 |
| Gluconolactone | 6.25 | 6.25 | 6.25 | 5.00 | 5.00 | 5.00 |
| Total ABA's (5-25g/500g) | 25.000 | 25.000 | 25.000 | 25.00 | 25.00 | 25.00 |
| Nicotinamide (5-25g/500g) | 25.00 | 25.00 | 25.00 | 25.00 | 25.00 | 25.00 |

(continued)

| Other ingredients | | | | | | |
|---|---|---|---|---|---|---|
| Sodium chloride | 2.00 | 2.00 | 2.00 | 2.00 | 2.00 | 2.00 |
| Propylene glycol | 150.00 | 150.00 | 150.00 | 150.00 | 150.00 | 150.00 |
| DI water | 28.50 | 28.50 | 28.50 | 28.50 | 28.50 | 28.50 |
| Myristyl alcohol | 7.50 | 7.50 | 7.50 | 7.50 | 7.50 | 7.50 |
| Phenoxyethanol | 2.50 | 2.50 | 2.50 | 2.50 | 2.50 | 2.50 |
| 25% LA NH3 | 2.00 | 2.00 | 2.00 | 2.00 | 2.00 | 2.00 |
| Trisiloxane | 70.50 | 68.00 | 58.00 | 58.00 | 58.00 | 33.00 |
| Carbomer | 2.00 | 2.00 | 2.00 | 2.00 | 2.00 | 2.00 |
| EL-7040 hydro elastomer[1] | 120.00 | 120.00 | 120.00 | 120.00 | 120.00 | 120.00 |
| DC-9040 elastomer[2] | 60.00 | 60.00 | 60.00 | 60.00 | 60.00 | 60.00 |
| Total | 500.00 | 500.00 | 500.00 | 500.00 | 500.00 | 500.00 |
| 1 Caprylyl Methicone (and) PEG-12 Dimethicone/ PPG-20 Crosspolymer 2 *Cyclopentasiloxane (and) Dimethicone Crosspolymer* | | | | | | |

**Examples: Table 7 Corticosteroids actives, antiproliferative**

| Materials | Example #35 g/500g batch | Example #36 g/500g batch | Example #37 g/500g batch | Example #38 g/500g batch |
|---|---|---|---|---|
| Active: | Betamethasone propionate 0.25 | Mometasone furoate 0.05 | Fluticasone propionate 0.05 | Calcipotriol 0.05 |
| Ancillary barrier agents | | | | |
| Lactobionic Acid | 20.00 | 5.00 | 5.00 | 12.50 |
| Gluconolactone | 5.00 | 1.25 | 1.25 | 12.50 |
| Total ABA's (5-25g/500g) | 25.00 | 6.25 | 6.25 | 25.00 |
| Nicotinamide (5-25g/500g) | 25.00 | 25.00 | 25.00 | 37.50 |
| Other ingredients | | | | |
| Sodium chloride | 2.00 | 2.00 | 2.00 | 2.00 |
| Glycerol | 17.50 | 17.50 | 17.50 | - |
| Propylene glycol | 150.00 | 165.00 | 165.00 | 150.00 |
| DI water | 28.50 | 28.50 | 28.50 | 25.00 |
| Myristyl alcohol | 7.50 | 7.50 | 7.50 | 7.50 |
| Phenoxyethanol | 2.50 | 2.50 | 2.50 | 2.50 |
| Trisiloxane | 59.75 | 63.7 | 63.70 | 68.45 |
| Carbomer | 2.00 | 2.00 | 2.00 | 2.00 |

(continued)

| Other ingredients | | | | |
|---|---|---|---|---|
| EL-7040 hydro elastomer[1] | 120.00 | 120.00 | 120.00 | 120.00 |
| DC-9040 elastomer[2] | 60.00 | 60.00 | 60.00 | 60.00 |
| Total | 500.00 | 500.00 | 500.00 | 500.00 |
| 1 Caprylyl Methicone (and) PEG-12 Dimethicone/ PPG-20 Crosspolymer<br>2 Cyclopentasiloxane (and) Dimethicone Crosspolymer | | | | |

**Examples: Table 8** Process control of Nicotinamide-PHA compositions (target wt, actual wt)

| Materials | **Example #39** | **Example #40** | **Example #41** | **Example #42** | **Example #43** | Order of addition In Process Control |
|---|---|---|---|---|---|---|
| Tare weight | 360.51 (Σ v act) | 360.48 (Σ v act) | 359.38 (Σ v act) | 359.91 (Σ v act) | 379.36 (Σ v act) | |
| Sodium chloride | 2.00, 2.00 | 2.00, 2.00 | 2.00, 2.01 | 2.00, 2.00 | 2.00, 2.01 | 1 |
| DI water | 15.00, 15.01 | 28.50, 28.50 | 25.00, 25.00 | 25.00, 25.00 | 28.50, 28.51 | 2 |
| Propylene glycol | 150.00, 150.00 | 150.00, 150.00 | 200.00, 200.00 | 150.00, 150.01 | 150.00, 150.01 | 3 |
| Glycerol | 20.00, 20.02 | 17.50, 17.50 | - | - | 17.50, 17.53 | 4 |
| Nicotinamide | 25.00, 25.01 | 25.00, 25.00 | 50.00, 50.00 | 37.50, 37.50 | 25.00, 25.01 | 5 |
| Weight check (1-5) | (572.51 v 572.54) | (583.39 v 583.48) | (636.36 v 636.39) | (574.40 v 574.42) | (602.36 v 602.43) | IPC |
| pH (1-5) | | - | 5.977 | 5.928 | 5.972 | IPC |
| Lactobionic acid (PHA) | 25.00, 25.02 | 18.75, 18.75 | 12.50, 12.49 | 12.50, 12.49 | 12.50, 12.50 | 6a |
| Gluconolactone (PHA) | - | 6.25, 6.27 | 12.50, 12.51 | 12.50,12.50 | 12.50, 12.55 | 6b |
| Weight check (1-6) | (597.46) | - | - | - | - | IPC |
| pH (1-6) | 3.910 | - | 3.888 | 3.778 | 3.611 | IPC |
| 80% Lactic acid | - | - | - | - | - | 7a |
| 25% liquid NH3 | 0.73, 0.73 | 2.00, 2.00 | 1.10, 1.10 | 1.40, 1.40 | 1.60, 1.60 | 7b |
| 25% NaOH | - | - | - | - | - | 7c |
| pH (1-7) | 4.538 | 4.452 | 4.468 | 4.472 | 4.500 | IPC |
| Phenoxyethanol | 2.50, 2.51 | 2.50, 2.49 | 2.50, 2.50 | 2.50, 2.50 | 2.50, 2.50 | 8 |
| Myristyl alcohol | 7.50, 7.51 | 7.50, 7.50 | 7.50, 7.51 | 7.50, 7.50 | 7.50, 7.50 | 9 |
| EL-7040 hydro elastomer | 120.00, 120.13 | 120.00, 120.29 | 94.50, 94.80 | 120.00, 120.04 | 120.00, 120.28 | 10 |

(continued)

| Materials | **Example #39** | **Example #40** | **Example #41** | **Example #42** | **Example #43** | Order of addition In Process Control |
|---|---|---|---|---|---|---|
| DC-9040 elastomer | 60.00, 60.00 | 60.00, 60.12 | 48.00, 48.20 | 60.00, 60.08 | 60.00, 60.07 | 11 |
| Carbomer | 2.00, 2.01 | 2.00, 2.00 | 2.00, 2.00 | 2.00, 2.00 | 2.00, 2.00 | 12 (+1-9) |
| Trisiloxane | ~70.27 (to 100%) | ~57.58 (to 100%) | ~42.40 (to 100%) | ~67.10 (to 100%) | ~58.40(to 100%) | 13 |
| pH cream (4.4-4.8) | 4.550 | 4.402 | 4.466 | 4.502 | 4.431 | IPC |
| Total | 500.00g | 500.00g | 500.00g | 500.00g | 500.00g | |

**Examples: Table 9** Process control of Nicotinamide-PHA compositions (target wt, actual wt)

| Materials | **Example #44** | **Example #45** | **Example #46** | **Example #47** | Order of addition In Process Control |
|---|---|---|---|---|---|
| Tare weight | **358.69** (Σ v act) | **360.63** (Σ v act) | **379.36** (Σ v act) | **358.45** (Σ v act) | |
| Sodium chloride | 2.00, 2.01 | 2.00, 2.00 | 2.00, 2.00 | 2.00, 2.01 | 1 |
| DI water | 25.00, 25.00 | 25.00, 25.00 | 28.50, 28.55 | 15.00, 15.03 | 2 |
| Propylene glycol | 200.00, 200.00 | 150.00, 150.02 | 150.00, 150.01 | 200.00, 200.00 | 3 |
| Glycerol | - | - | 17.50, 17.57 | - | 4 |
| Nicotinamide | 50.00, 50.01 | 37.50, 37.51 | 25.00, 25.01 | 50.00, 50.00 | 5 |
| Weight check (1-5) | (635.71 v 635.71) | (575.10 v 575.16) | (602.49 v 602.50) | 625.56 v 625.49 | IPC |
| pH (1-5) | 5.785 | 5.859 | 5.8768 | - | IPC |
| Lactobionic acid (PHA) | 25.00, 25.02 | 25.00, 25.00 | 25.00, 25.00 | 25.00, 25.02 | 6a |
| Gluconolactone (PHA) | - | - | - | - | 6b |
| Weight check (1-6) | - | - | - | - | IPC |
| pH (1-6) | 4.040 | 3.969 | 3.856 | 4.064 | IPC |
| 80% Lactic acid | - | - | - | - | 7a |
| 25% liquid NH3 | 1.20, 1.21 | 1.10, 1.10 | 1.20, 1.19 | - | 7b |
| 25% NaOH | | | | 3.01, 3.01 | 7c |
| pH (1-7) | 4.800 | 4.663 | 4.648 | 5.089 | IPC |
| Phenoxyethanol | 2.50, 2.51 | 2.50, 2.51 | 2.50, 2.50 | 2.50, 2.52 | 8 |
| Myristyl alcohol | 7.50, 7.51 | 7.50, 7.50 | 7.50, | 7.50, 7.52 | 9 |
| EL-7040 hydro elastomer | 94.50, 94.67 | 120.00, 120.30 | 120.00, 120.20 | 94.50, 94.86 | 10 |
| DC-9040 elastomer | 48.00, 48.26 | 60.00, 60.36 | 60.00, 60.16 | 48.00, 48.32 | 11 |
| Carbomer | 2.00, 2.01 | 2.00, 2.00 | 2.00, 2.00 | 2.00, 2.01 | 12 |

(continued)

| Materials | **Example #44** | **Example #45** | **Example #46** | **Example #47** | Order of addition In Process Control |
|---|---|---|---|---|---|
| Trisiloxane | ~42.29 (to 100%) | ~67.23 (to 100%) | ~58.81 (to 100%) | ~51.45 (to 100) | 13 |
| pH cream (4.4-4.8) | 4.795 | 4.610 | 4.605 | 5.010 | IPC |
| Total | 500.00g | 500.00g | 500.00g | 500.00g | |

**Examples: Table 10** Process control of Nicotinamide-PHA compositions (target wt, actual wt)

| Materials | **Example #48** | **Example #49** | **Example #50** | **Example #51** | Order of addition In Process Control |
|---|---|---|---|---|---|
| Tare weight | **358.57** (Σ v act) | **359.46** (Σ v act) | **379.36** (Σ v act) | (Σ v act) | |
| Sodium chloride | 2.00, 2.01 | 2.00, 2.00 | 2.00, 2.00 | 2.00, 2.00 | 1 |
| DI water | 15.00, 15.01 | 15.00, 15.02 | 15.00, 15.01 | 15.00, 15.02 | 2 |
| Propylene glycol | 180.00, 180.00 | 180.00, 180.05 | 150.00, 150.02 | 150.00, 150.00 | 3 |
| Glycerol | - | - | 20.00, 20.00 | 20.00, 20.02 | 4 |
| Nicotinamide | 25.00, 25.00 | 25.00, 25.02 | 12.50, 12.50 | 12.50, 12.50 | 5 |
| Weight check (1-5) | (580.57 v 580.59) | (581.54 v 581.60) | (578.85 v 578.89) | (559.38 v 559.38) | IPC |
| pH (1-5) | - | - | - | - | IPC |
| Lactobionic acid (PHA) | 25.00, 25.02 | 25.00, 25.01 | 25.00, 25.02 | 25.00, 25.00 | 6a |
| Gluconolactone (PHA) | - | - | - | - | 6b |
| Weight check (1-6) | (605.59 v 605.61) | (606.56 v 605.61) | (603.88 v 603.91) | (584.38 v 584.38) | IPC |
| pH (1-6) | 3.976 | 3.997 | 3.779 | 3.773 | IPC |
| 80% Lactic acid | 5.00, 5.02 | 5.00, 5.02 | 3.50, 3.51 | 3.50, 3.51 | 7a |
| 25% liquid NH3 | 1.75, 1.75 | 1.75, 1.74 | 1.50, 1.49 | 1.50, 1.50 | 7b |
| 25% NaOH | - | - | - | - | 7c |
| pH (1-7a) (1-7b) | (3.813) (4.676) | (3.815) (4.695) | (3.650) (4.446) | (3.653) (4.547) | IPC |
| Phenoxyethanol | 2.50, 2.51 | 2.50, 2,51 | 2.50, 2.51 | 2.50, 2.51 | 8 |
| Myristyl alcohol | 7.50, 7.50 | 7.50, 7.50 | 7.50, 7.51 | 7.50, 7.50 | 9 |
| EL-7040 hydro elastomer | 99.33, 99.46 | 99.33, 99.38 | 114.00, 114.16 | 114.00, 114.33 | 10 |
| DC-9040 elastomer | 49.67, 49.81 | 49.67, 49.80 | 57.00, 57.32 | 57.00, 57.21 | 11 |
| Carbomer | 2.00, 2.01 | 2.00, 2.01 | 2.0, 2.02 | 2.0, 2.02 | 12 |
| Trisiloxane | ~85.25 (to 100%) | ~85.25 (to 100%) | ~87.50 (to 100%) | ~87.50 (to 100%) | 13 |
| pH cream (4.4-4.8) | 4.574 | 4.581 | 4.435 | 4.454 | IPC |
| Total | 500.00g duplicates samples | | 500.00g duplicates samples | | |

**Examples: Table 11** Process control of Nicotinamide-PHA compositions (target wt, actual wt)

| Materials | **Example #52** | **Example #53** | **Example #54** | **Example #55** | **Example #56** | Order of addition In Process Control |
|---|---|---|---|---|---|---|
| Tare weight | **379.17** (Σ v act) | **359.77** (Σ v act) | **357.98** (Σ v act) | **358.82** (Σ v act) | **357.98** (Σ v act) | |
| Sodium chloride | 2.00, 2.01 | 2.00, 2.00 | 2.00, 2.00 | 2.00, 2.00 | 2.00, 2.00 | 1 |
| DI water | 15.00, 15.00 | 15.00, 15.01 | 15.00, 15.02 | 12.50, 12.52 | 20.00, 20.01 | 2 |
| Propylene glycol | 150.00, 150.03 | 150.00, 150.02 | 150.00, 150.00 | 150.00, 150.02 | 175.00, 175.01 | 3 |
| Glycerol | 20.00, 20.02 | 10.00, 10.00 | 0.00, 0.00 | 25.0, 25.00 | - | 4 |
| Nicotinamide | 12.50, 12.50 | 12.50, 12.50 | 12.50, 12.50 | 50.00, 50.02 | 50.00, 50.00 | 5 |
| Weight check (1-5) | (578.76 v 578.73) | - | - | - | 630.00 v 630.06 | IPC |
| pH (1-5) | | - | - | | 3.865 | 1PC |
| Lactobionic acid (PHA) | 25.00, 25.02 | 25.00, 25.11 | 25.00, 25.02 | 25.00, 25.00 | 25.00, 25.00 | 6a |
| Gluconolactone (PHA) | - | - | - | - | - | 6b |
| Weight check (1-6) | - | - | - | - | - | IPC |
| pH (1-6) | 3.747 | - | - | - | - | IPC |
| 80% Lactic acid | 3.50, 3.52 | 3.50, 3.50 | 3.50, 3.55 | - | - | 7a |
| 25% liquid NH3 | 1.50, 1.52 | 1.50, 1.50 | 1.50, 1.50 | 1.10, 1.10 | 1.10, 1.10 | 7b |
| 25% NaOH | - | - | - | | | 7c |
| pH (1-7) | 4.460 | 4.476 | 4.454 | - | - | IPC |
| Phenoxyethanol | 2.50, 2.51 | 2.50, 2.51 | 2.50, 2.53 | 2.50, 2.51 | 2.50, 2.51 | 8 |
| Myristyl alcohol | 7.50, 7.50 | 7.50, 7.51 | 7.50, 7.50 | 7.50, 7.63 | 7.50, 7.50 | 9 |
| EL-7040 hydro elastomer | 114.00, 114.23 | 114.00, 114.00 | 114.00, 114.08 | 102.00, 102.23 | 90.00, 90.15 | 10 |
| DC-9040 elastomer | 57.00, 57.13 | 57.00, 57.00 | 57.00, 57.27 | 54.90, 54.95 | 25.00, 25.12 | 11 |
| Carbomer | 2.00, 2.00 | 2.00, 2.00 | 2.00, 2.00 | 2.00, 2.00 | 2.00, 2.02 | 12 (+1-9) |
| SOLAVEIL CTP-7-PW-(LK) | - | - | - | 12.50, 12.51 | 25.00, 25.00 | 13 |
| Trisiloxane | ~87.46 (to 100%) | ~97.50 (to 100%) | ~107.50 (to 100%) | ~53.00 | 74.70 | 14 |
| pH cream (4.4-4.8) | 4.515 | 4.463 | 4.482 | 4.750 | 4.580 | IPC |
| IKA Helipath SP11 10rpm mPas | 19,000 | 14,400 | 10,500 | 36,300 | - | IPC |
| Total | 500.00g | 500.00g | 500.00g | 500.00g | 500.00g | |

The above described embodiments have been given by way of example only, and the skilled reader will naturally

appreciate that many variations could be made thereto without departing from the scope of the invention.

## Claims

1. A composition for topical application comprising:

a primary active agent for topical treatment of the skin, and
a user adherence-improving skin barrier restoring combination of:

1.0 to 5%w/w of nicotinamide;
1.0 to 5%w/w of polyhydroxy acid;
10 to 60%w/w of a partition coefficient enhancer (PC enhancer), having a structure of the general formula: $C_nH_{2n+2}O_2$ where n represents an integer from 3 to 5 inclusive;
a diffusion coefficient enhancer (DC enhancer) selected from the group consisting of a $C_{12}$ to $C_{14}$ straight chain fatty acid and a $C_{14}$ straight chain primary alcohol;
a first dimethicone macromer mixture including a dimethicone macromer and a hydrocarbyl methyl siloxane emollient selected from the group consisting of an alkyl methyl siloxane, an aryl methyl siloxane and an alkyl aryl methyl siloxane, and
a second dimethicone macromer mixture including a methyl siloxane compound and a cross-linked dimethicone macromer;
wherein the composition comprises less than 15 % water by weight.

2. The composition according to claim 1, wherein the first dimethicone macromer mixture includes a polyglycol dimethicone macromer, generally comprising a compound of the following structure:

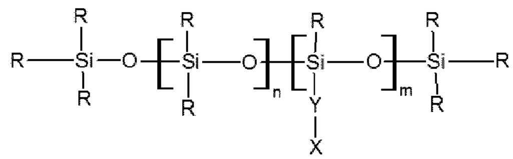

Where R represents H or hydrocarbyl group, in particular C1 to C6 alkyl group;
Y represents a hydrocarbyl group in particular C1 to C6 alkyl group;
X represents an amine, a quaternary amino group or acid functionality.
M and n independently represent an integer from 1 to 50.

3. The composition according to any one of the preceding claims, wherein the composition comprises 5 to 45%w/w first dimethicone macromer mixture, typically 10 to 40%w/w, generally 20 to 30%w/w, and/or comprising 5 to 45%w/w second dimethicone macromer mixture, typically 10 to 40%w/w, generally 20 to 30w%w/w, suitably wherein the first dimethicone macromer mixture includes a dimethicone macromer having a number average molecular weight of more than 1000 (typically more than 2000) and a hydrocarbyl methyl siloxane emollient (generally an alkyl methyl siloxane) having a number average molecular weight of less than 500.

4. The composition according to any one of the preceding claims, wherein the first dimethicone macromer mixture includes:

(a) a polyglycol dimethicone macromer cross-linked with a polyalkylene oxide compound (generally a polyethylene glycol compound, a polypropylene glycol compound or a copolymer of ethylene oxide and propylene oxide) or cross-linked with a diene, generally wherein the first dimethicone macromer mixture includes a polyglycol dimethicone macromer selected from the group consisting of PEG dimethicone PPG crosspolymer and PEG dimethicone bis-isoalkyl PPG crosspolymer; or
(b) a polyglycol dimethicone macromer comprising one or more pendant groups from the dimethicone backbone, said pendant group(s) being a polyalkylene oxide group (generally a polyethylene glycol compound, a polypropylene glycol compound or a copolymer of ethylene oxide and propylene oxide), generally wherein the polyglycol dimethicone macromer includes a polyethylene glycol pendant group and a polypropylene glycol pendant group from the dimethicone backbone.

**5.** The composition according to any one of the preceding claims, comprising a pyrrolidone carboxylic acid functionalized dimethicone macromer.

**6.** The composition according to any one of the preceding claims, wherein

(a) the second dimethicone macromer mixture includes a methyl siloxane compound having a number average molecular weight of less than 1000 and a cross-linked polyalkylsiloxane diol dimethicone macromer having a number average molecular weight of more than 1000, generally of more than 2000; and/or
(b) the first dimethicone macromer mixture comprises 5 to 30%w/w polyglycol dimethicone macromer, typically 10-20%w/w generally 12-19%w/w; and/or where the second dimethicone macromer mixture comprises 5 to 30%w/w cross-linked dimethicone macromer, typically 10-20%w/w generally 12-19%w/w.

**7.** The composition according to any one of the preceding claims, including less than 0.05% w/w water or substantially no water.

**8.** The composition according to any one of the preceding claims, wherein the polyhydroxy acid is selected from lactobionic acid, gluconolactone or galactose and any mixture of these.

**9.** The composition according to any one of the preceding claims, comprising a second mutually miscible PC enhancer/cosolvent selected from the group consisting of an alcohol, ether-alcohol, diol, triol or alkyl pyrrolidone, suitably selected from the group consisting of a diol of the general formula $C_nH_{2n+2}O_2$ where n represents an integer greater than 6; an alcohol of the general formula $C_nH_{2n+2}O$, where n represents an integer 2 or 3; an ether-alcohol of the general formula $C_nH_{2n+2}O_3$ or $C_nH_{2n+2}O_2$ where n represents an integer from 1 to 10 or an alkyl pyrrolidone; generally wherein the second mutually miscible PC enhancer/cosolvent is glycerol or N-methyl pyrrolidone.

**10.** The composition according to any one of the preceding claims, including:

(a) 25 to 45%w/w PC enhancer; and/or including less than 10%w/w diffusion coefficient enhancer, generally less than 5%w/w, optimally 0.5-2%w/w; and/or
(b) 25 to 45%w/w PC enhancer; and/or including less than 0.5-2%w/w diffusion coefficient enhancer, generally less than 0.25%%w/w, optimally 0.01-0.25%, for example; where retention of the primary active in the stratum corneum is required; and/or
(c) a highly volatile solvent selected from the group consisting of hexamethyldisiloxane, octamethyltrisiloxane, cyclopentacyloxane, ethanol, isopropyl alcohol and water.

**11.** The composition according to any one of the preceding claims, wherein the product pH is in the range 4.4-6.00 and /or the equilibrium pH is in the range pH 3-4.

**12.** The composition according to any one of the preceding claims, wherein the active agent is selected from

(a) the group consisting of retinoids, retinoic acid metabolic blocking agents (RAMBAs), cannabinoids including tetrahydrocannabinol and cannabidiol, alpha and beta hydroxy acids and polymers, lactobionic acid, gluconolactone, immune response modifier compounds, tranexamic acid, calcipotriol (aka calcipotriene) calcidiol, calcitriol, calcipotriene, paricalcitol, 22-oxacolcitriol, dihydrotachysterol, calciferol, nicotinamide, corticosteroids, anabolic steroids, estrogens, anti-rosacea, agents, antihistamines, antibacterial agents, antiacne agents, antifungal agents, antiviral agents, cytotoxic agents for use in actinic keratoses, basal cell and squamous cell cancers and melanoma, psoralens, anti-alopecia agents, anti-androgens, anti-pruritic agents, keratolytic agents, skin lightening and depigmenting agents, dithranol, antiseptics, anaesthetics, analgesics, neuropathics, non-steroidal antiinflammatory agents, vasoactive agents and agents to combat dry and ageing skin, or;
(b) nicotinamide at 5-10% by weight.

**13.** The composition according to any one of the preceding claims, wherein the primary active agent is not tranexamic acid in combination with nicotinamide 1-5% by weight and PHA 1-5% by weight.

**14.** The composition as claimed in any one of Claims 1 to 13 for use in therapy.

**15.** The composition as claimed in any one of Claims 1 to 13 for use in:

a) the prevention, alleviation or treatment of a medical condition of the human or animal body wherein the medical condition is caused by or associated with one or more of pain and/or inflammation, pigmentation, pruritus, acne, eczema, psoriasis, rosacea, skin blistering diseases such as bullous pemphigoid, nappy rash, dry skin, microbial conditions including fungal and/or bacterial conditions such as skin infections including yeast infections and dermatophyte infections, viral infections of the skin or mucosa, warts, dry or ageing skin, hypoandrogenism, immunological conditions, sun spots, actinic keratosis, basal cell and squamous cell skin cancers and melanoma, alopecia and dermatitis due to radiation therapy; or
(b) the treatment of inflammatory skin diseases such as eczema, psoriasis, acne, bullous pemphigoid and rosacea, when the active agent is a PARP-1 inhibitor, preferably nicotinamide; or
(c) the chemoprevention of actinic keratoses, basal cell and squamous cell skin cancers and melanoma when the active agent is a PARP-1 inhibitor, preferably nicotinamide; or
(d) the chemoprevention of actinic keratoses, basal cell and squamous cell skin cancers and melanoma when the active agent is a PARP-1 inhibitor, preferably nicotinamide in combination with a broad-spectrum inorganic or hydrophilic UV-block; or
(e) the treatment of actinic keratoses and melanoma when the active agent is a PARP-1 inhibitor, preferably nicotinamide; or
(f) the treatment of actinic keratoses and melanoma when the active agent is a PARP-1 inhibitor, preferably nicotinamide in combination with a broad-spectrum inorganic or hydrophilic UV-block; or
(g) the chemoprevention of radiation dermatitis when the active agent is a PARP-1 inhibitor, preferably nicotinamide; or
(h) the treatment of skin pigmentation when the active agent is a melanosome transfer inhibitor, preferably nicotinamide.

## Patentansprüche

**1.** Zusammensetzung zur topischen Anwendung, welche Folgendes umfasst:

einen Hauptwirkstoff für die topische Behandlung der Haut, und
eine die Anwenderhaftung verbessernde, die Hautbarriere wiederherstellende Kombination aus:

1,0 bis 5 Gew.-% Nicotinamid;
1,0 bis 5 Gew.-% Polyhydroxysäure;
10 bis 60 Gew.-% eines Verteilungskoeffizienten-Verstärkers (PC-Verstärker), der eine Struktur der allgemeinen Formel aufweist: $CnH_{2n+2}O_2$, wobei n eine ganze Zahl von 3 bis einschließlich 5 darstellt;
einen Diffusionskoeffizienten-Verstärker (DC-Enhancer), ausgewählt aus der Gruppe bestehend aus einer geradkettigen $C_{12}$ bis $C_{14}$-Fettsäure und einem geradkettigen primären $C_{14}$-Alkohol;
eine erste Dimethiconmakromermischung, die ein Dimethiconmakromer und ein Kohlenwasserstoffmethylsiloxan-Weichmacher enthält, der aus der Gruppe ausgewählt ist, die aus einem Alkylmethylsiloxan, einem Arylmethylsiloxan und einem Alkylarylmethylsiloxan besteht, und
einer zweiten Dimethiconmakromermischung, die eine Methylsiloxanverbindung und ein vernetztes Dimethiconmakromer enthält;
wobei die Zusammensetzung weniger als 15 Gewichtsprozent Wasser enthält.

**2.** Die Zusammensetzung nach Anspruch 1, wobei die erste Dimethiconmakromermischung ein Polyglycoldimethiconmakromer einschließt, das im Allgemeinen eine Verbindung der folgenden Struktur umfasst:

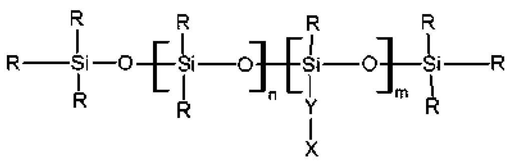

worin R H oder eine Hydrocarbylgruppe, insbesondere eine C1- bis C6-Alkylgruppe, darstellt;
Y eine Hydrocarbylgruppe, insbesondere eine C1- bis C6-Alkylgruppe, darstellt;
X stellt ein Amin, eine quaternäre Aminogruppe oder eine Säurefunktionalität dar.
M und n stellen unabhängig voneinander eine ganze Zahl von 1 bis 50 dar.

3. Die Zusammensetzung nach einem der vorangehenden Ansprüche, wobei die Zusammensetzung 5 bis 45% Gew./Gew. erste Dimethiconmakromermischung umfasst,
typischerweise 10 bis 40% Gew./Gew., im Allgemeinen 20 bis 30% Gew./Gew., und/oder 5 bis 45% Gew./Gew. zweite Dimethiconmakromermischung, typischerweise 10 bis 40% Gew./Gew., im Allgemeinen 20 bis 30% Gew./Gew., umfasst, wobei die erste Dimethiconmakromermischung ein Dimethiconmakromer mit einem zahlenmittleren Molekulargewicht von mehr als 1000 (typischerweise mehr als 2000) und ein Kohlenwasserstoffmethylsiloxan-Weichmacher (im Allgemeinen ein Alkylmethylsiloxan) mit einem zahlenmittleren Molekulargewicht von weniger als 500 einschließt.

4. Die Zusammensetzung nach einem der vorhergehenden Ansprüche, wobei die erste Dimethiconmakromermischung Folgendes enthält:

(a)ein Polyglykoldimethiconmakromer, das mit einer Polyalkylenoxidverbindung (im Allgemeinen eine Polyethylenglykolverbindung, eine Polypropylenglykolverbindung oder ein Copolymer aus Ethylenoxid und Propylenoxid) vernetzt ist oder mit einem Dien vernetzt ist, wobei das erste Dimethiconmakromergemisch im Allgemeinen ein Polyglykoldimethiconmakromer einschließt, das aus der Gruppe ausgewählt ist, die aus PEG-Dimethicon-PPG-Crosspolymer und PEG-Dimethicon-bis-isoalkyl-PPG-Crosspolymer besteht; oder
(b)einem Polyglykoldimethiconmakromer, das eine oder mehrere an der Dimethiconhauptkette hängende Gruppen umfasst, wobei die hängende(n) Gruppe(n) eine Polyalkylenoxidgruppe (im Allgemeinen eine Polyethylenglykolverbindung, eine Polypropylenglykolverbindung oder ein Copolymer aus Ethylenoxid und Propylenoxid) ist/sind, wobei das Polyglykoldimethiconmakromer im Allgemeinen eine an der Dimethiconhauptkette hängende Polyethylenglykolgruppe und eine an der Dimethiconhauptkette hängende Polypropylenglykolgruppe umfasst.

5. Die Zusammensetzung nach einem der vorhergehenden Ansprüche, umfassend ein mit Pyrrolidoncarbonsäure funktionalisiertes Dimethiconmakromer.

6. Die Zusammensetzung nach einem der vorangehenden Ansprüche, wobei

(a)die zweite Dimethiconmakromermischung eine Methylsiloxanverbindung mit einem zahlenmittleren Molekulargewicht von weniger als 1000 und ein vernetztes Polyalkylsiloxandiol-Dimethiconmakromer mit einem zahlenmittleren Molekulargewicht von mehr als 1000, im Allgemeinen von mehr als 2000, enthält; und/oder
(b)die erste Dimethiconmakromermischung 5 bis 30 % Gew./Gew. Polyglycoldimethiconmakromer, typischerweise 10-20 % Gew./Gew., im Allgemeinen 12-19 % Gew./Gew. umfasst; und/oder wobei die zweite Dimethiconmakromermischung 5 bis 30 % Gew./Gew. vernetztes Dimethiconmakromer, typischerweise 10-20 % Gew./Gew., im Allgemeinen 12-19 % Gew./Gew. umfasst.

7. Die Zusammensetzung nach einem der vorhergehenden Ansprüche, die weniger als 0,05% Gew./Gew. Wasser oder im Wesentlichen kein Wasser enthält.

8. Die Zusammensetzung nach einem der vorangehenden Ansprüche, wobei die Polyhydroxysäure ausgewählt ist aus Lactobionsäure, Gluconolacton oder Galactose und jeder Mischung davon.

9. Zusammensetzung nach einem der vorhergehenden Ansprüche, umfassend einen zweiten mit einander mischbaren PC-Verstärker/Kosolvens, ausgewählt aus der Gruppe, bestehend aus einem Alkohol, Etheralkohol, Diol, Triol oder Alkylpyrrolidon, geeignet ausgewählt aus der Gruppe, bestehend aus einem Diol der allgemeinen Formel $C_nH_{2n}+_2O_2$, worin n eine ganze Zahl größer als 6 darstellt; einem Alkohol der allgemeinen Formel $C_nH_{2n+2}O$, in der n eine ganze Zahl von 2 oder 3 darstellt; einem EtherAlkohol der allgemeinen Formel $CnH_{2n+2}O_3$ oder $C_nH_{2n}+_2O_2$, in der n eine ganze Zahl von 1 bis 10 darstellt, oder einem Alkylpyrrolidon; wobei es sich bei dem zweiten mit einander mischbaren PC-Verstärker/Kosolvens im Allgemeinen um Glycerin oder N-Methylpyrrolidon handelt.

10. Die Zusammensetzung nach einem der vorangehenden Ansprüche, einschließlich:

(a)25 bis 45% Gew./Gew. PC-Verstärker; und/oder weniger als 10% Gew./Gew. Diffusionskoeffizient-Verstärker, im Allgemeinen weniger als 5% Gew./Gew., optimal 0,5-2% Gew./Gew.; und/oder
(b)25 bis 45% Gew./Gew. PC-Verstärker; und/oder mit weniger als 0,5-2% Gew./Gew. Diffusionskoeffizient-Verstärker, im Allgemeinen weniger als 0,25 % Gew./Gew., optimal z.B. 0,01-0,25 %, wenn die Retention des primären Wirkstoffs im Stratum corneum erforderlich ist; und/oder
(c)ein leicht flüchtiges Lösungsmittel, ausgewählt aus der Gruppe bestehend aus Hexamethyldisiloxan, Octamethyltrisiloxan, Cyclopentacyloxan, Ethanol, Isopropylalkohol und Wasser.

**11.** Die Zusammensetzung nach einem der vorhergehenden Ansprüche, wobei der Produkt-pH-Wert im Bereich von 4,4-6,00 liegt und/oder der Gleichgewichts-pH-Wert im Bereich von pH 3-4 liegt.

**12.** Die Zusammensetzung nach einem der vorangehenden Ansprüche, wobei der Wirkstoff ausgewählt ist aus

(a) der Gruppe bestehend aus Retinoiden, Retinsäure-Stoffwechselblockern (RAMBAs), Cannabinoiden einschließlich Tetrahydrocannabinol und Cannabidiol, Alpha- und Beta-Hydroxysäuren und -polymeren, Lactobionsäure, Gluconolacton, Immunreaktionsmodifizierungsverbindungen, Tranexamsäure, Calcipotriol (auch bekannt als Calcipotrien), Calcidiol, Calcitriol, Calcipotrien, Paricalcitol, 22-Oxacolcitriol, Dihydrotachysterol, Calciferol, Nicotinamid, Kortikosteroide, anabole Steroide, Östrogene, Mittel gegen Rosazea, Mittel, Antihistaminika, antibakterielle Mittel, Mittel gegen Akne, antimykotische Mittel, antivirale Mittel, zytotoxische Mittel zur Verwendung bei aktinischen Keratosen, Basalzell- und Plattenepithelkarzinomen und Melanomen, Psoralene, Mittel gegen Alopezie, Antiandrogene, Juckreizstillende Mittel, keratolytische Mittel, hautaufhellende und depigmentierende Mittel, Dithranol, Antiseptika, Anästhetika, Analgetika, Neuropathien, nicht-steroidale entzündungshemmende Mittel, vasoaktive Mittel und Mittel gegen trockene und alternde Haut, oder;
(b) Nicotinamid in einer Menge von 5-10 Gew.-%.

**13.** Die Zusammensetzung nach einem der vorhergehenden Ansprüche, wobei der primäre Wirkstoff nicht Tranexamsäure in Kombination mit 1-5 Gew.-% Nikotinamid und 1-5 Gew.-% PHA ist.

**14.** Die Zusammensetzung nach einem der Ansprüche 1 bis 13 zur Verwendung in der Therapie.

**15.** Die Zusammensetzung nach einem der Ansprüche 1 bis 13 zur Verwendung bei:

a) die Vorbeugung, Linderung oder Behandlung eines medizinischen Zustands des menschlichen oder tierischen Körpers, wobei der medizinische Zustand durch ein oder mehrere der Folgenden verursacht wird oder damit verbunden ist: Schmerzen und/oder Entzündungen, Pigmentierung, Juckreiz, Akne, Ekzem, Psoriasis, Rosazea, Hautblasenbildung wie bullöses Pemphigoid, Windelausschlag, trockene Haut, mikrobielle Erkrankungen, einschließlich Pilz- und/oder Bakterienerkrankungen wie Hautinfektionen, einschließlich Hefepilzinfektionen und Dermatophyteninfektionen, Virusinfektionen der Haut oder der Schleimhäute, Warzen, trockene oder alternde Haut, Hypoandrogenismus, immunologische Erkrankungen, Sonnenflecken, aktinische Keratose, Basalzell- und Plattenepithelkarzinome der Haut und Melanome, Alopezie und Dermatitis infolge einer Strahlentherapie; oder
(b) die Behandlung von entzündlichen Hauterkrankungen wie Ekzemen, Psoriasis, Akne, bullösem Pemphigoid und Rosazea, wenn der Wirkstoff ein PARP-1-Inhibitor, vorzugsweise Nicotinamid, ist; oder
(c) die Chemoprävention von aktinischen Keratosen, Basalzell- und Plattenepithelkarzinomen der Haut und Melanomen, wenn der Wirkstoff ein PARP-1-Inhibitor, vorzugsweise Nikotinamid, ist; oder
(d) die Chemoprävention von aktinischen Keratosen, Basalzell- und Plattenepithelkarzinomen der Haut und Melanomen, wenn der Wirkstoff ein PARP-1-Inhibitor, vorzugsweise Nikotinamid, in Kombination mit einem anorganischen oder hydrophilen UV-Blocker mit breitem Spektrum ist; oder
(e) die Behandlung von aktinischen Keratosen und Melanomen, wenn der Wirkstoff ein PARP-1-Inhibitor, vorzugsweise Nicotinamid, ist; oder
(f) die Behandlung von aktinischen Keratosen und Melanomen, wenn der Wirkstoff ein PARP-1-Inhibitor, vorzugsweise Nikotinamid, in Kombination mit einem anorganischen oder hydrophilen UV-Blocker mit breitem Spektrum ist; oder
(g) die Chemoprävention von Strahlendermatitis, wenn der Wirkstoff ein PARP-1-Inhibitor, vorzugsweise Nikotinamid, ist; oder
(h) die Behandlung von Hautpigmentierung, wenn der Wirkstoff ein Melanosomentransfer-Inhibitor, vorzugsweise Nikotinamid, ist.

## Revendications

**1.** Composition pour application topique comprenant :

un agent actif principal pour le traitement topique de la peau, et

une combinaison de restauration de la barrière cutanée améliorant l'adhérence de l'utilisateur :

de 1,0 à 5 % p/p de nicotinamide ;
de 1,0 à 5 % p/p d'acide polyhydroxylé ;
de 10 à 60 % p/p d'un amplificateur de coefficient de partage (amplificateur PC), ayant une structure de formule générale : $C_nH_{2n+2}O_2$ où n représente un nombre entier de 3 à 5, bornes comprises ;
un amplificateur de coefficient de diffusion (amplificateur DC) choisi dans le groupe constitué d'un acide gras à chaîne droite en $C_{12}$ à $C_{14}$ et d'un alcool primaire à chaîne droite en $C_{14}$ ;
un premier mélange de macromères de diméthicone comprenant un macromère de diméthicone et un émollient hydrocarbylméthylsiloxane choisi dans le groupe constitué d'un alkylméthylsiloxane, d'un arylméthylsiloxane et d'un alkylarylméthylsiloxane, et
un second mélange de macromères de diméthicone comportant un composé méthylsiloxane et un macromère de diméthicone réticulé ;
dans laquelle la composition comprend moins de 15 % en poids d'eau.

**2.** Composition selon la revendication 1, dans laquelle le premier mélange de macromères de diméthicone comporte un macromère de polyglycol diméthicone, comprenant généralement un composé de structure suivante :

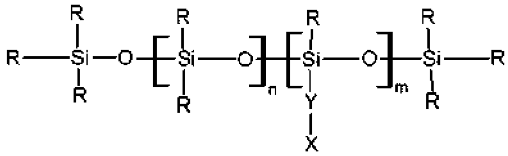

où R représente H ou un groupe hydrocarbyle, en particulier un groupe alkyle en $C_1$ à $C_6$ ;
Y représente un groupe hydrocarbyle en particulier un groupe alkyle en $C_1$ à $C_6$ ;
X représente une amine, un groupe amino quaternaire ou une fonctionnalité acide,
M et n représentent indépendamment un nombre entier de 1 à 50.

**3.** Composition selon l'une quelconque des revendications précédentes, dans laquelle la composition comprend de 5 à 45 % p/p d'un premier mélange de macromères de diméthicone, typiquement de 10 à 40 % p/p, généralement de 20 à 30 % p/p, et/ou comprend de 5 à 45 % p/p d'un second mélange de macromères de diméthicone, typiquement de 10 à 40 % p/p, généralement de 20 à 30 % p/p, dans laquelle avantageusement le premier mélange de macromères de diméthicone comprend un macromère de diméthicone ayant un poids moléculaire moyen en nombre supérieur à 1 000 (typiquement supérieur à 2 000) et un émollient hydrocarbylméthylsiloxane (généralement un alkylméthylsiloxane) ayant un poids moléculaire moyen en nombre inférieur à 500.

**4.** Composition selon l'une quelconque des revendications précédentes, dans laquelle le premier mélange de macromères de diméthicone comporte :

(a) un macromère de polyglycol diméthicone réticulé avec un composé de poly(oxyde d'alkylène) (généralement un composé de polyéthylène glycol, un composé de polypropylène glycol ou un copolymère d'oxyde d'éthylène et d'oxyde de propylène) ou réticulé avec un diène, généralement dans laquelle le premier mélange de macromère de diméthicone comprend un macromère de polyglycol diméthicone choisi dans le groupe constitué du polymère croisé PEG diméthicone PPG et du polymère croisé PEG diméthicone bis-isoalkyl PPG ; ou
(b) un macromère de polyglycol diméthicone comprenant un ou plusieurs groupes pendants du squelette diméthicone, le ou lesdits groupes pendants étant un groupe poly(oxyde d'alkylène) (généralement un composé de polyéthylène glycol, un composé de polypropylène glycol ou un copolymère d'oxyde d'éthylène et d'oxyde de propylène), généralement dans laquelle le macromère de polyglycol diméthicone comporte un groupe pendant polyéthylène glycol et un groupe pendant polypropylène glycol du squelette diméthicone.

**5.** Composition selon l'une quelconque des revendications précédentes, comprenant un macromère de diméthicone fonctionnalisé par un acide pyrrolidone carboxylique.

**6.** Composition selon l'une quelconque des revendications précédentes, dans laquelle

(a) le second mélange de macromères de diméthicone comprend un composé de méthylsiloxane ayant un poids moléculaire moyen en nombre inférieur à 1 000 et un macromère de polyalkylsiloxane diol diméthicone

réticulé ayant un poids moléculaire moyen en nombre supérieur à 1 000, généralement supérieur à 2 000 ; et/ou
(b) le premier mélange de macromères de diméthicone comprend de 5 à 30 % p/p de macromère de polyglycol diméthicone, typiquement de 10 à 20 % p/p, généralement de 12 à 19 % p/p ; et/ou où le second mélange de macromères de diméthicone comprend de 5 à 30 % p/p de macromère de diméthicone réticulé, typiquement de 10 à 20 % p/p, généralement de 12 à 19 % p/p.

**7.** Composition selon l'une quelconque des revendications précédentes, comprenant moins de 0,05 % p/p d'eau ou pratiquement pas d'eau.

**8.** Composition selon l'une quelconque des revendications précédentes, dans laquelle l'acide polyhydroxylé est choisi parmi l'acide lactobionique, la gluconolactone ou le galactose et tout mélange de ceux-ci .

**9.** Composition selon l'une quelconque des revendications précédentes, comprenant un second activateur de PC/cosolvant mutuellement miscible choisi dans le groupe constitué d'un alcool, d'un éther-alcool, d'un diol, d'un triol ou d'une alkylpyrrolidone, avantageusement choisi dans le groupe constitué d'un diol de la formule générale $C_nH_{2n+2}O_2$ où n représente un nombre entier supérieur à 6 ; un alcool de formule générale $C_nH_{2n+2}O$, où n représente un nombre entier 2 ou 3 ; un éther-alcool de formule générale $C_nH_{2n+2}O_3$ ou $C_nH_{2n+2}O_2$ où n représente un nombre entier de 1 à 10 ou une alkylpyrrolidone ; généralement dans laquelle le second activateur de PC/cosolvant mutuellement miscible est le glycérol ou la N-méthylpyrrolidone.

**10.** Composition selon l'une quelconque des revendications précédentes, comportant :

(a) de 25 à 45 % p/p d'amplificateur de PC ; et/ou comportant moins de 10 % p/p d'amplificateur de coefficient de diffusion, généralement moins de 5 % p/p, de manière optimale 0,5 à 2 % p/p ; et/ou
(b) de 25 à 45 % p/p d'amplificateur de PC ; et/ou comprenant moins de 0,5 à 2 % p/p d'amplificateur de coefficient de diffusion, généralement moins de 0,25 % p/p, de manière optimale de 0,01 à 0,25 %, par exemple ; où une rétention de l'actif primaire dans la couche cornée est nécessaire ; et/ou
(c) un solvant hautement volatil choisi dans le groupe constitué de l'hexaméthyldisiloxane, de l'octaméthyltrisiloxane, du cyclopentacyloxane, de l'éthanol, de l'alcool isopropylique et de l'eau.

**11.** Composition selon l'une quelconque des revendications précédentes, dans laquelle le pH du produit est dans la plage de 4,4 à 6,00 et/ou le pH à l'équilibre est dans la plage de 3 à 4.

**12.** Composition selon l'une quelconque des revendications précédentes, dans laquelle l'agent actif est choisi parmi

(a) le groupe constitué des rétinoïdes, des agents bloquants métaboliques de l'acide rétinoïque (RAMBA), des cannabinoïdes, dont le tétrahydrocannabinol et le cannabidiol, des acides et polymères alpha et bêta-hydroxylés, de l'acide lactobionique, de la gluconolactone, des composés modificateurs de la réponse immunitaire, de l'acide tranexamique, du calcipotriol (aka calcipotriene), du calcidiol, du calcitriol, du calcipotriène, du paricalcitol, du 22-oxacolcitriol, du dihydrotachystérol, du calciférol, du nicotinamide, des corticostéroïdes, des stéroïdes anabolisants, des œstrogènes, des agents anti-rosacée, des antihistaminiques, des agents antibactériens, des agents antiacnéiques, des agents antifongiques, des agents antiviraux, des agents cytotoxiques destinés à être utilisés dans les kératoses actiniques, les cancers basocellulaires et les épidermoïdes et le mélanome, des psoralènes, des agents anti-alopécie, des anti-androgènes, des agents antiprurigineux, des agents kératolytiques, des agents éclaircissants et des dépigmentants pour la peau, du dithranol, des antiseptiques, anesthésiques, des analgésiques, des agents neuropathiques, des agents anti-inflammatoires non stéroïdiens, des agents vasoactifs et des agents de lutte contre la peau sèche et vieillissante, ou ;
(b) le nicotinamide à raison de 5 à 10 % en poids.

**13.** Composition selon l'une quelconque des revendications précédentes, dans laquelle l'agent actif principal n'est pas l'acide tranexamique en combinaison avec du nicotinamide à raison de 1 à 5 % en poids et du PHA à raison de 1 à 5 % en poids.

**14.** Composition selon l'une quelconque des revendications 1 à 13, destinée à être utilisée en thérapie.

**15.** Composition selon l'une quelconque des revendications 1 à 13, destinée à être utilisée dans :

a) la prévention, le soulagement ou le traitement d'une pathologie médicale du corps humain ou animal, dans

laquelle la pathologie médicale est causée par ou associée à un ou plusieurs éléments parmi la douleur et/ou l'inflammation, la pigmentation, le prurit, l'acné, l'eczéma, le psoriasis, la rosacée, maladies vésiculeuses cutanées telles que la pemphigoïde bulleuse, l'érythème fessier, la peau sèche, les affections microbiennes, notamment les affections fongiques et/ou bactériennes, telles que les infections cutanées, notamment les infections à levures et les infections à dermatophytes, les infections virales de la peau ou des muqueuses, les verrues, la peau sèche ou vieillissante, l'hypoandrogénie, les affections immunologiques, les taches solaires, la kératose actinique, les cancers basocellulaires et épidermoïdes de la peau, le mélanome, l'alopécie et la dermatite dus à une radiothérapie ; ou

(b) le traitement des maladies inflammatoires de la peau telles que l'eczéma, le psoriasis, l'acné, la pemphigoïde bulleuse et la rosacée, lorsque l'agent actif est un inhibiteur de PARP-1, de préférence le nicotinamide ; ou

(c) la chimioprévention des kératoses actiniques, des cancers basocellulaires et épidermoïdes de la peau et du mélanome lorsque l'agent actif est un inhibiteur de PARP-1, de préférence le nicotinamide ; ou

(d) la chimioprévention des kératoses actiniques, des cancers basocellulaires et épidermoïdes de la peau et du mélanome lorsque l'agent actif est un inhibiteur de PARP-1, de préférence le nicotinamide en combinaison avec un bloqueur d'UV inorganique ou hydrophile à large spectre ; ou

(e) le traitement des kératoses actiniques et des mélanomes lorsque l'agent actif est un inhibiteur de PARP-1, de préférence le nicotinamide ; ou

(f) le traitement des kératoses actiniques et du mélanome lorsque l'agent actif est un inhibiteur de PARP-1, de préférence le nicotinamide en combinaison avec un bloqueur d'UV inorganique ou hydrophile à large spectre ; ou

(g) la chimioprévention de la dermatite radiologique lorsque l'agent actif est un inhibiteur de PARP-1, de préférence le nicotinamide ; ou

(h) le traitement de la pigmentation cutanée lorsque l'agent actif est un inhibiteur du transfert des mélanosomes, de préférence le nicotinamide.

Figure 1

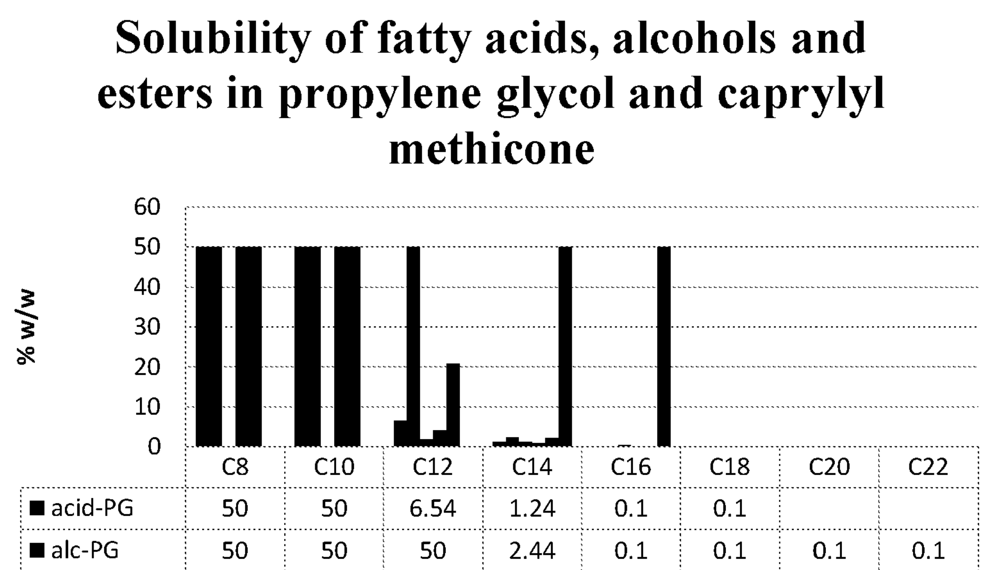


| | C8 | C10 | C12 | C14 | C16 | C18 | C20 | C22 |
|---|---|---|---|---|---|---|---|---|
| ■ acid-PG | 50 | 50 | 6.54 | 1.24 | 0.1 | 0.1 | | |
| ■ alc-PG | 50 | 50 | 50 | 2.44 | 0.1 | 0.1 | 0.1 | 0.1 |
| ■ IP ester-PG | | | 1.98 | 1.35 | 0.47 | | | |
| ■ acid-CM | 50 | 50 | 4.16 | 0.93 | 0.21 | 0.1 | 0.1 | 0.1 |
| ■ alc-CM | 50 | 50 | 20.85 | 2.3 | 0.22 | 0.1 | 0.1 | 0.1 |
| ■ IP ester-CM | | | | 50 | 50 | | | |

Figure 2

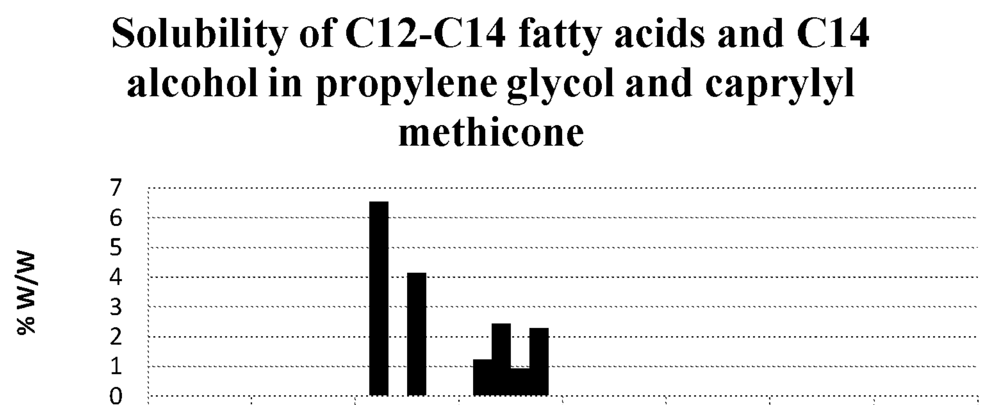


| | C8 | C10 | C12 | C14 | C16 | C18 | C20 | C22 |
|---|---|---|---|---|---|---|---|---|
| ■ acid-PG | | | 6.54 | 1.24 | | | | |
| ■ alc-PG | | | | 2.44 | | | | |
| ■ acid-CM | | | 4.16 | 0.93 | | | | |
| ■ alc-CM | | | | 2.3 | | | | |

Figure 3

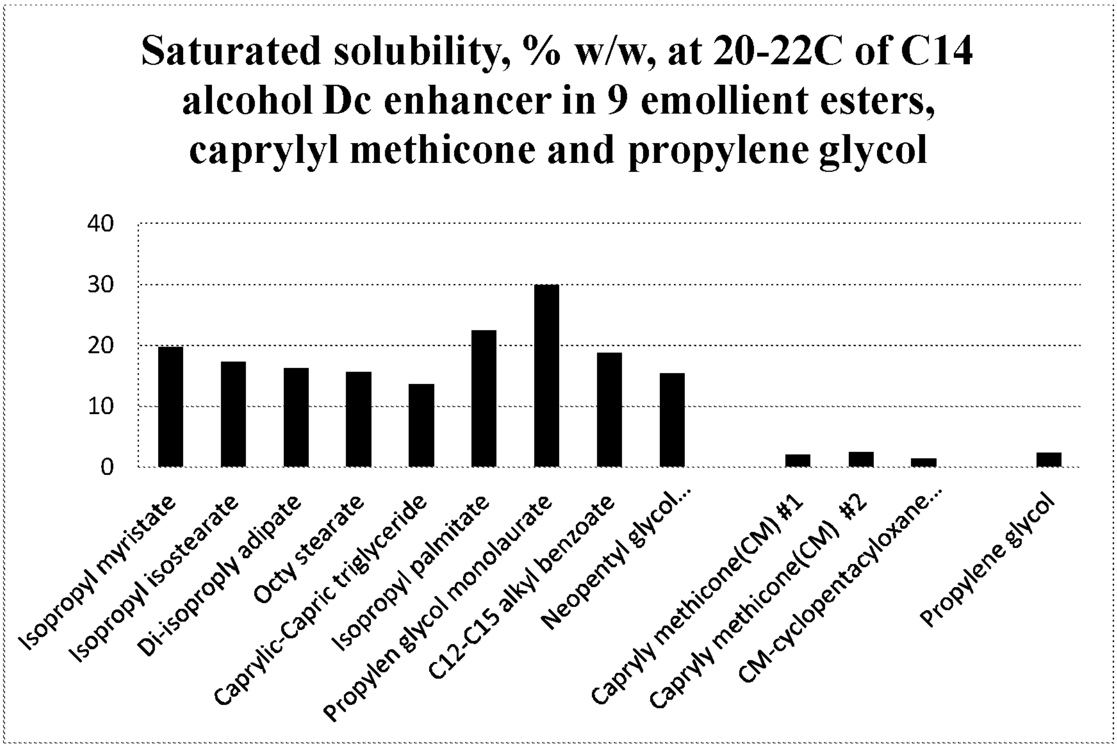
Saturated solubility, % w/w, at 20-22C of C14 alcohol Dc enhancer in 9 emollient esters, caprylyl methicone and propylene glycol
40
30
20
10
0
Isopropyl myristate
Isopropyl isostearate
Di-isoproply adipate
Octy stearate
Caprylic-Capric triglyceride
Isopropyl palmitate
Propylen glycol monolaurate
C12-C15 alkyl benzoate
Neopentyl glycol...
Capryly methicone(CM) #1
Capryly methicone(CM) #2
CM-cyclopentacyloxane...
Propylene glycol

Figure 4

Figure 4a

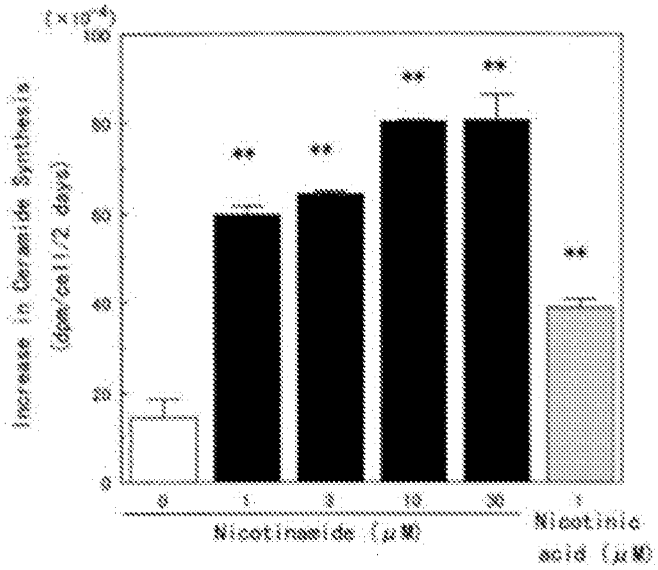


Nicotinamide increases ceramide synthesis dose-dependently. Nicotinamide (1~30 μmol $L^{-1}$) or nicotinic acid (1 μmol $L^{-1}$) was added to the culture for 6 days. Ceramide synthesis was measured by incorporation of [$^{14}$C]-serine into ceramide for 2 days (see Materials and methods). Mean ± SEM; $n = 3$; $^{**}P < 0{\cdot}01$, Dunnett's multiple comparison; dpm, disintegrations per min.

Figure 4b

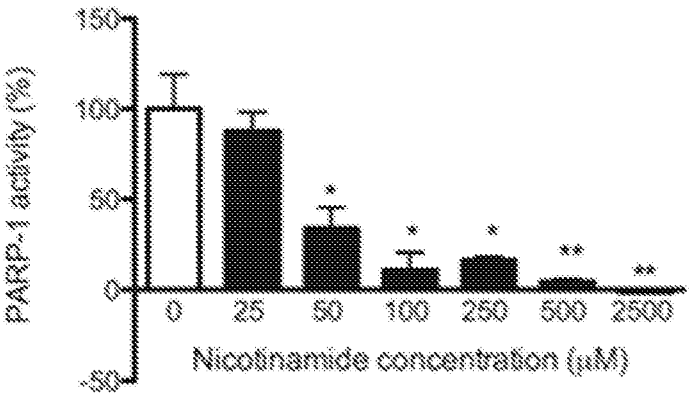


Nicotinamide dose-responsively inhibits poly-ADP-ribose polymerase (PARP)-1 activity. Nicotinamide at concentrations ≥50 μM inhibited PARP-1, assessed with a chemiluminescent activity assay ($n = 8$ for positive control; $n = 12$, 12, 4, 6, 5 and 6 for nicotinamide at concentrations from 25 to 2500 μM, respectively; $^{*}P < 0.01$; $^{**}P < 0.001$; one-way ANOVA compared with control).

Figure 5

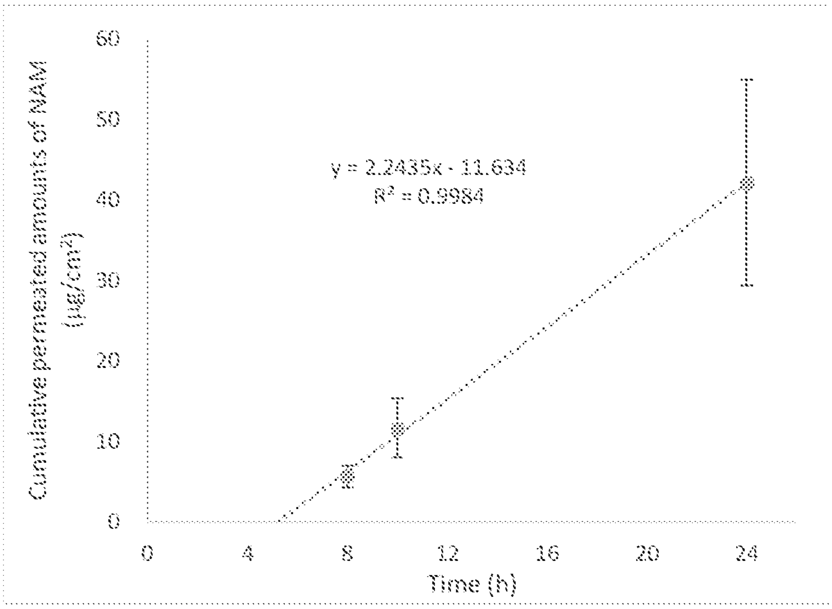

Cumulative permeated amounts of NAM (μg/cm²)
60
50
40
30
20
10
0
0
4
8
12
16
20
24
Time (h)
y = 2.2435x - 11.634
R² = 0.9984

Figure 6

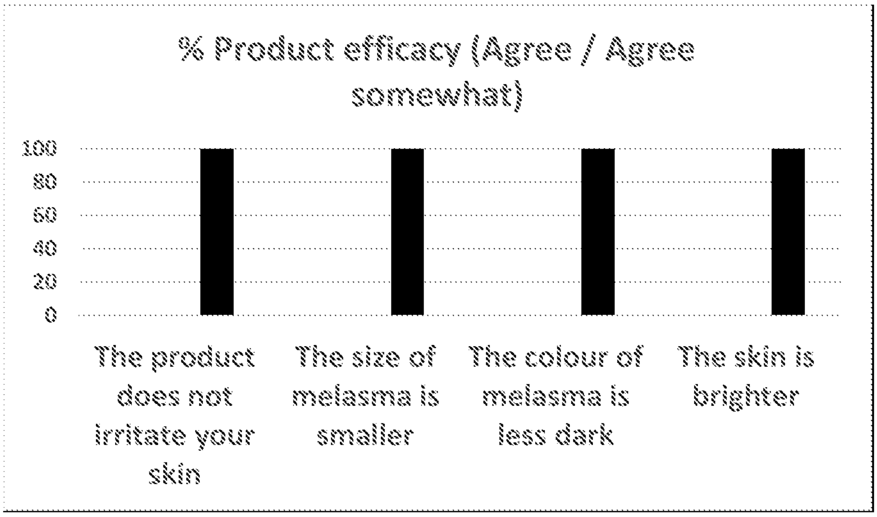
% Product efficacy (Agree / Agree somewhat)
100
80
60
40
20
0
The product does not irritate your skin
The size of melasma is smaller
The colour of melasma is less dark
The skin is brighter

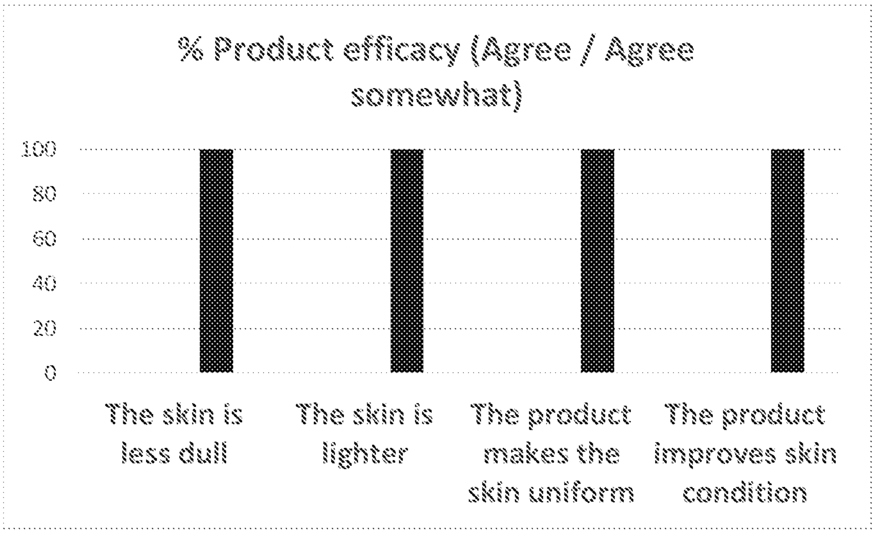
% Product efficacy (Agree / Agree somewhat)
100
80
60
40
20
0
The skin is less dull
The skin is lighter
The product makes the skin uniform
The product improves skin condition

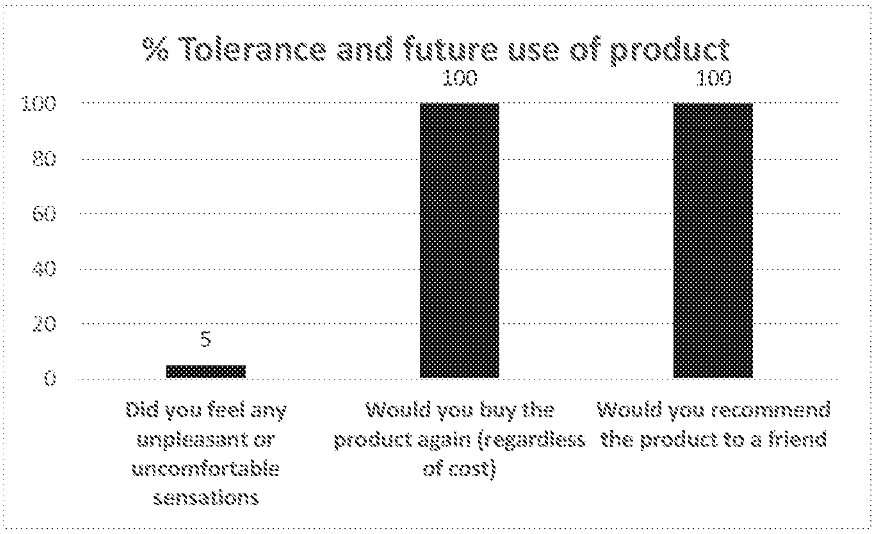
% Tolerance and future use of product
100
100
100
80
60
40
20
0
5
Did you feel any unpleasant or uncomfortable sensations
Would you buy the product again (regardless of cost)
Would you recommend the product to a friend

Figure 7

*Figures 7.1a and 7.1b:*

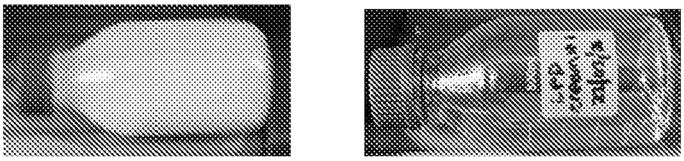

*Figures 7.2a, 7.2b and 7.2c:*

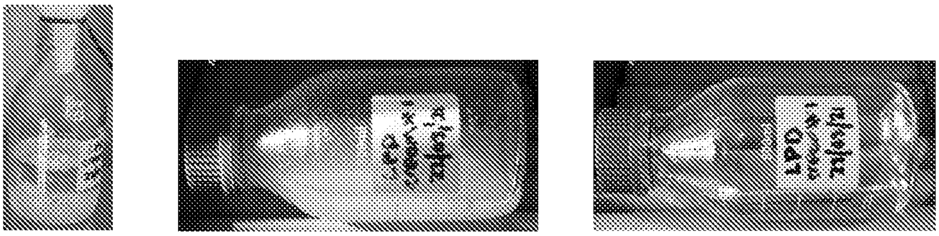

*Figures 7.3a, 7.3b and 7.3c:*

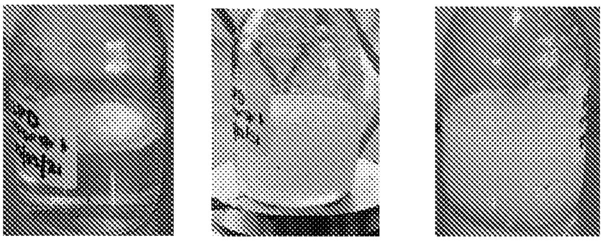

*Figures 7.4a, 7.4b, 7.4c, 7.4d and 7.4e:*

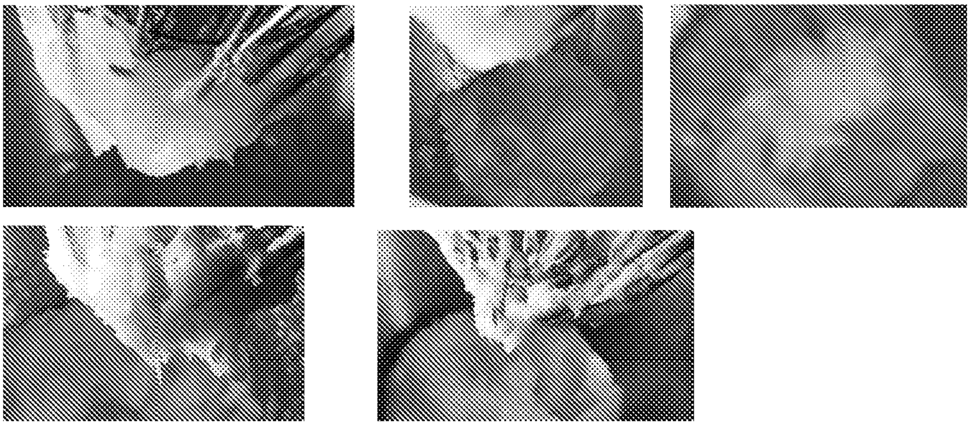

*Figures 7.5a and 7.5b:*

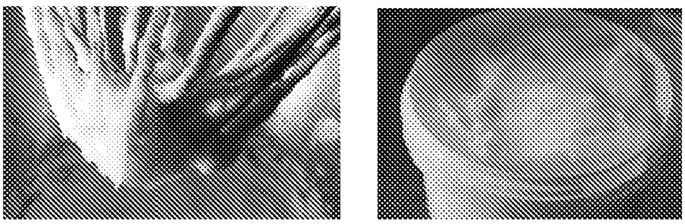

Figure 8

Figure 8a:

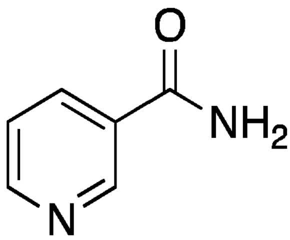

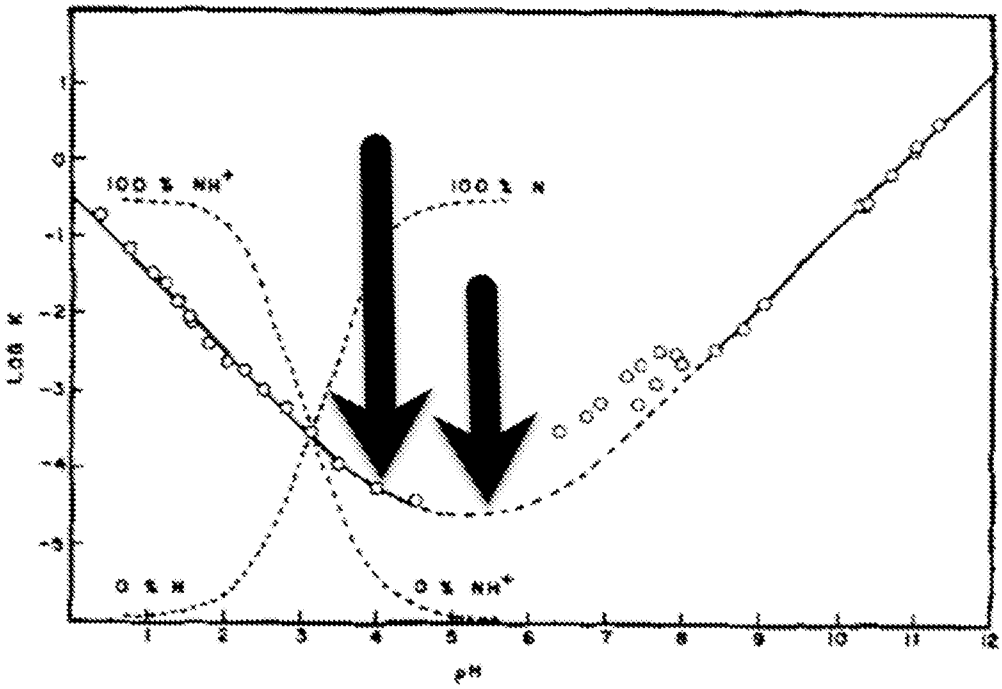


Figure 8b:

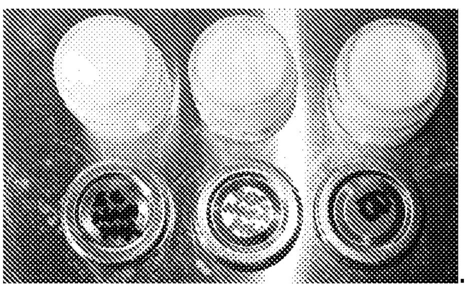.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 8541470 B
- GB 2549418 B
- US 10028927 B
- WO 2016139471 A1
- GB 2562270 A


### Non-patent literature cited in the description


- **ZSCHOCKE et al.** *Venereol,* May 2014, vol. 28 (2), 4-9
- **TAN et al.** *Expert Opin Drug Deliv.,* October 2012, vol. 9 (10), 1263-71
- **DEVAUX et al.** *Acad Dermatol Venereol,* May 2012, vol. 26 (3), 61-7
- **TANNO O et al.** *British Journal of Dermatology,* 2000, vol. 143, 524-531
- **SOMA Y et al.** *Int J Dermatol.,* March 2005, vol. 44 (3), 197-202
- **DRAELOS ZD et al.** *J Cosmet Laser Ther,* June 2006, vol. 8 (2), 96-101
- **KAWADA A et al.** *J Dermatol.,* October 2008, vol. 35 (10), 637-42
- **WOHLRAB ; KREFT.** *Skin Pharmacol Physiol.,* 2014, vol. 27 (6), 311-5
- **GRIMES et al.** *Cutis,* February 2004, vol. 73 (2), 3-13
- **HACHEM JP et al.** Acute acidification of stratum corneum membrane domains using polyhydroxyl acids improves lipid processing and inhibits degradation of corneodesmosomes. *J Invest Dermatol.,* February 2010, vol. 130 (2), 500-10
- **PARK J.** *Photochem Photobiol.,* 2010, vol. 86 (4), 942-948
- **TING L et al.** *Signal Transduct Target Therap.,* 2017, vol. 2, 17023
- **UNGERSTEDT JS. et al.** *Clin Exp Immunol.,* 2003, vol. 131 (1), 48-52
- **HWANG ; SONG.** *Cell Mol Life Sci.,* 2017, vol. 74 (18), 3347-3362
- **HAKOSAKI et al.** *Br J Dermatol.,* 2002, vol. 147 (1), 20-3
- *Exp Dermatol.,* 2005, vol. 14 (7), 498-508
- **HAKOSAKI et al.** *Br J Dermatol.,* 2002
- **HAKOSAKI et al.** *Exp Dermatol.,* 2005
- **TANNO et al.** *British Journal of Dermatology.,* 2000, vol. 143, 524-531
- **DRAELOS ZD et al.** *J Cosmet Laser Ther.,* June 2006, vol. 8 (2), 96-101
- **ZHANG Y et al.** *Pharmaceutics.,* 2019, vol. 11, 668-681
- **DAMIAN et al.** *J Invest Dermatol.,* 2008, vol. 128 (2), 447-54
- **MOLONEY-DAMIAN et al.** *Br J Dermatol,* 2010, vol. 162 (5), 1138-9
- *J. Invest. Dermatol.,* 2012, vol. 132, 1497-1500
- **CHEN-DAMIAN et al.** A Phase 3 Randomised Trial of Nicotinamide for Skin-Cancer Chemoprevention. *N. Engl J Med,* 2015, vol. 373 (17), 161-26
- **MALESU A et al.** *Photochem Photobiol Sci,* February 2020, vol. 19 (2), 171-179
- **MALESU et al.** *Photochem Photobiol Sci,* February 2020, vol. 19 (2), 171-179
- *Nicotinamide Chemoprevention for Keratinocyte Carcinoma in Solid Organ Transplant Recipients: A Pilot, Placebo-controlled, Randomized Trial,* December 2020
- **DRAGO et al.** Eur J Dermatol. Dept. Dermatology, 2017, vol. 27, 382-385
- **NIKAS, IP et al.** The Role of Nicotinamide in Cancer Chemoprevention and Therapy. *Biomolecules,* March 2020, vol. 10 (3), 477-497
- **CHEN A.** *Chin J Cancer.,* July 2011, vol. 30 (7), 463-471
- **DRAGO et al.** Eur J Dermatol. Dept Dermatology, 2017, vol. 27, 382-385
- Nicotinamide: a potential addition to the anti-psoriatic weaponry. *FASEB J,* 2003, vol. 17 (11), 1377-9
- **LEVINE D et al.** *J Am Acad Dermatol.,* 2010, vol. 63 (5), 775-81
- **SIADAT, AH et al.** *Adv Biomed R.,* 2013, vol. 2, 90
- **KOLBACH DN. et al.** *BJD.,* 1995, vol. 13 (1), 88-90
- **GRANGE et al.** Nicotinamide inhibits Propionibacterium acnes-induced IL-8 production in keratinocytes through the NF-kB and MAPK pathways. *Journal of Dermatological Science,* 2009, vol. 56, 106-112
- **BOWSTROM A. et al.** *Radiotherapy and Oncology.,* 2001, vol. 59, 257-265

- **HAKOSAKI et al.** *Exp Dermatol.,* 2005, vol. 14 (7), 498-508
- **BONGIOVANNI T et al.** *Review Proc. Med. Def. Bioscience.,* 1993, vol. 1
- **NG SP. et al.** *J Cosmet Dermatol.,* 2020, vol. 19 (10), 2656-2662
- **BALA et al.** *Dermatol Surg.,* 2018, vol. 44 (6), 814-825
- **PERTERSON RV ; HAMILL RD.** *J Soc. Cos. Chem.,* 1968, vol. 19, 627-640
- **FLORENCE AT et al.** *Novel anhydrous emulsions*
- **FINHOLT ; HIGUCHI.** Rate Studies on the Hydrolysis of Niacinamide. *J. Pharm Sci,* 1962, vol. 51 (7), 655-661